# EUROPEAN PATENT APPLICATION

(11) **EP 4 678 764 A2**
(43) Date of publication of application: **14.01.2026**
(21) Application number: 25211221.4
(22) Date of filing: 22.12.2020
(51) Int. Cl.: C12Q 1/6869

(54) **NANOPARTICLE WITH SINGLE SITE FOR TEMPLATE POLYNUCLEOTIDE ATTACHMENT**

(30) Priority: 23.12.2019 US 201962952799 P; 23.12.2019 US 201962952866 P
(62) Divisional of application: 20842860.7
(71) Applicant: ILLUMINA, INC., San Diego, CA 92122 (US); Illumina Cambridge Limited, Cambridge CB21 6DF (GB); Illumina Singapore Pte. Ltd., Singapore 757716 (SG)
(72) Inventor: YANG, Xiangyuan, 757716 Singapore (SG); GEORGE, Wayne N., Cambridge, CB21 6DF (GB); GATTI LAFRANCONI, Pietro, Cambridge, CB21 6DF (GB); TEO, Yin Nah, 757716 Singapore (SG); BROWN, Andrew A., Cambridge, CB21 6DF (GB); BACIGALUPO, Maria Rogert, San Diego, 92122 (US); BRUSTAD, Eric, San Diego, 92122 (US); BOHRA, Hassan, San Diego, 92122 (US); WANG, Clifford, San Diego, 92122 (US)
(74) Representative: Murgitroyd & Company

(57) **Abstract**

Provided is a nanoparticle including a scaffold, a single template site for bonding a template polynucleotide to the scaffold, and a plurality of accessory sites for bonding accessory oligonucleotides to the scaffold, wherein the scaffold is selected from an asymmetrical acrylamide polymer one or a dendrimer including lysyl constitutional repeating units, the single template site for bonding a template polynucleotide to the scaffold is selected from a covalent template bonding site and a noncovalent template bonding site and the plurality of accessory sites for bonding accessory oligonucleotides to the scaffold are selected from covalent accessory oligonucleotide bonding sites and noncovalent accessory oligonucleotide bonding sites. Also provided are methods of using the nanoparticle.

## Description

### CROSS-REFERENCE TO RELATED APPLICATIONS

This application is an International Application filed pursuant to the Patent Cooperation Treaty, which claims benefit of priority from U.S. Provisional Patent Application No. 62/952,799, filed on December 23, 2019, and U.S. Provisional Patent Application No. 62/952,866, filed on December 23, 2019, the entire contents of which are incorporated herein by reference.

### SEQUENCE LISTING

The instant application contains a Sequence Listing, created on December 16, 2020; the file, in ASCII format, is designated 1912732.txt and is 3.2 KB in size. The file is hereby incorporated by reference in its entirety into the instant application.

### BACKGROUND

Many current sequencing platforms use "sequencing by synthesis" (SBS) technology and fluorescence-based methods for detection. In some examples, numerous target polynucleotides isolated from a library to be sequences, or template polynucleotides, are attached to a surface of a substrate in a process known as seeding. Multiple copies of the template polynucleotides may then be synthesized in attachment to the surface in proximity to where a template polynucleotide of which it is a copy was seeded, in a process called clustering. Subsequently, nascent copies of the clustered polynucleotides are synthesized under conditions in which they emit a signal identifying each nucleotide as it is attached to the nascent strand. Clustering of a plurality of copies of the seeded template polynucleotide in proximity to where it was initially seeded results in amplification of signal generated during the visualizable polymerization, improving detection.

Seeding and clustering for SBS work well when as much of an available substrate surface as possible is seeded by template polynucleotides, which may maximize an amount of sequencing information obtainable during a sequencing run. By contrast, generally speaking the less available surface area of a substrate used for seeding and clustering, the less efficient an SBS process may be, resulting in increased time, reactants, expense, and complicated data processing for obtaining a given amount of sequencing information of a given library.

Seeding and clustering also work well when template polynucleotides from a library with sequences that differ from each other seed on, or attach to, positions of the surface sufficiently distal from each other such that clustering results in spatially distinct clusters of copied polynucleotides each resulting from the seeding of a single template polynucleotide, a condition generally referred to as monoclonality. That is, a library of template polynucleotides may generally include a high number of template polynucleotide molecules whose nucleotide sequences differ from each other's. If two such template polynucleotides seed too closely together on a surface of a substrate, clustering may result in spatially comingled populations of copied polynucleotides, some of which having a sequence of one of the template polynucleotides that seeded nearby and others having a sequence of another template polynucleotide that also seeded nearby on the surface. Or, two clusters formed from two different template polynucleotides that seeded in too close proximity to each other may be too adjacent to each other or adjoin each other such that an imaging system used in an SBS process may be unable to distinguish them as separate clusters even though there may be no or minimal spatial comingling of substrate-attached sequences between the clusters. Such a disadvantageous condition may generally be referred to as polyclonality. It may be more difficult, time consuming, expensive, and less efficient, and require more complicated data analytics to obtain unambiguous sequence information from a polyclonal cluster if present.

### SUMMARY

It is therefore desirable to perform SBS under conditions under which as much available surface area as possible of a substrate surface is used for seeding and clustering, while also promoting separation of seeded template polynucleotides so as to maximize monoclonality of clusters as possible and minimize polyclonal clusters as much as possible. Disclosed herein are compositions and methods that may be used for advantageously increasing seeding density and monoclonal clustering in SBS.

In one aspect, provided is a nanoparticle, including a scaffold, a single template site for bonding a template polynucleotide to the scaffold selected from a covalent template bonding site and a noncovalent template bonding site, and a plurality of accessory sites for bonding accessory oligonucleotides to the scaffold selected from covalent accessory oligonucleotide bonding sites and noncovalent accessory oligonucleotide bonding sites, wherein the scaffold is a compound of Formula I:
each X is a compound of formula II: 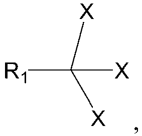 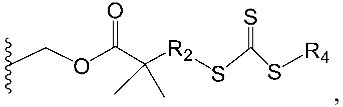
wherein R₂ is selected from Formula IIIa: 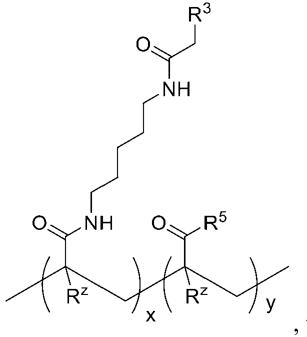 wherein R⁵ is selected from 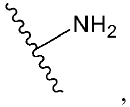 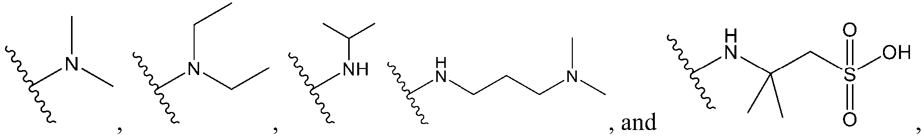 X is an integer in the range of from 1-2,000 and y is an integer in the range of from 1-10,000 and a ratio of x:y may be from approximately 10:90 to approximately 1:99, and wherein each R^{z} is independently H or C₁₋₄ alkyl, and Formula IIIb: 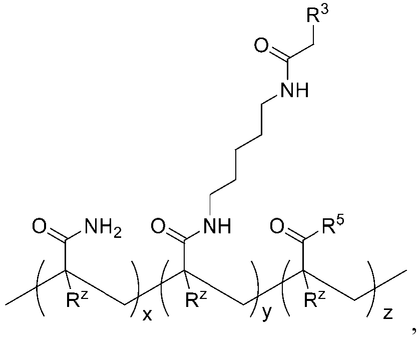 wherein R⁵ is selected from 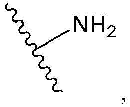 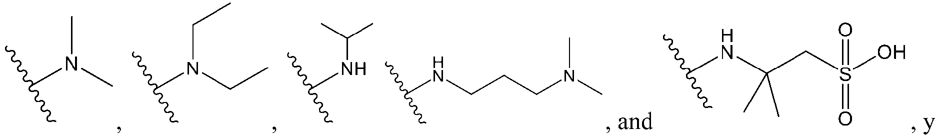 is an integer in the range of from 1-2,000 and x and z are integers whose sum is in a range of from 1-10,000 and a ratio of (x:y):z may be from approximately (85):15 to approximately (95):5, and wherein each R^{z} is independently H or C₁₋₄ alkyl, R₁ includes the single template site for bonding a template polynucleotide to the scaffold, R⁴ is selected from an optionally substituted C₁-C₂₀ alkyl, an optionally substituted C₁-C₂₀ alkenyl, an optionally substituted C₁-C₂₀ alkynyl, an optionally substituted C₁-C₂₀ oxaalkyl, an optionally substituted C₁-C₂₀ thiaalkyl, and an optionally substituted C₁-C₂₀ azaalkyl, wherein substituted includes substitution with one or more of a C₁-C₂₀ alkyl, a double-bonded oxygen, and a hydroxyl group, and R³ includes the accessory site for bonding accessory oligonucleotides.

In an example the single template site includes a covalent template bonding site. In another example, the covalent template bonding site is selected from amine-NHS ester bonding site, an amine-imidoester bonding site, an amine-pentofluorophenyl ester bonding site, an amine-hydroxymethyl phosphine bonding site, a carboxyl-carbodiimide bonding site, a thiol-maleimide bonding site, a thiol-haloacetyl bonding site, a thiol-pyridyl disulfide bonding site, a thiol-thiosulfonate bonding site, a thiol-vinyl sulfone bonding site, an aldehyde-hydrazide bonding site, an aldehyde-alkoxyamine bonding site, an aldehyde-NHS ester bonding site, a hydroxy-isocyanate bonding site, an azide-alkyne bonding site, an azide-phosphine bonding site, a transcyclooctene-tetrazine bonding site, a norbornene-tetrazine bonding site, an azide-cyclooctyne bonding site, an azide-norbornene bonding site, an oxime bonding site, a SpyTag-SpyCatcher bonding site, a Snap-tag-O⁶-Benzylguanine bonding site, a CLIP-tag-O²-benzylcytosine bonding site, and a sortase-coupling bonding site.

In another example, the single template site includes a noncovalent template bonding site. In yet another example, the noncovalent template bonding site includes a polynucleotide hybridization site. In still a further example, the noncovalent template bonding site includes a noncovalent peptide binding site and the noncovalent peptide binding site is selected from a coiled-coil bonding site and an avidin-biotin bonding site.

In another example, the plurality of accessory sites for bonding accessory oligonucleotides to the scaffold include covalent accessory oligonucleotide bonding sites. In still another example, the covalent accessory oligonucleotide bonding sites are selected from amine-NHS ester bonding sites, amine-imidoester bonding sites, amine-pentofluorophenyl ester bonding sites, amine-hydroxymethyl phosphine bonding sites, carboxyl-carbodiimide bonding sites, thiol-maleimide bonding sites, thiol-haloacetyl bonding sites, thiol-pyridyl disulfide bonding sites, thiol-thiosulfonate bonding sites, thiol-vinyl sulfone bonding sites, aldehyde-hydrazide bonding sites, aldehyde-alkoxyamine bonding sites, hydroxy-isocyanate bonding sites, azide-alkyne bonding sites, azide-phosphine bonding sites, transcyclooctene-tetrazine bonding sites, norbornene-tetrazine bonding sites, an azide-cyclooctyne bonding sites, azide-norbornene bonding sites, oxime bonding sites, SpyTag-SpyCatcher bonding sites, Snap-tag-O⁶-Benzylguanine bonding sites, CLIP-tag-O²-benzylcytosine bonding sites, sortase-coupling bonding sites, and any combination of two or more of the foregoing.

In another example, the accessory oligonucleotide bonding sites include noncovalent accessory oligonucleotide bonding sites. In yet another example, the noncovalent accessory oligonucleotide bonding sites include polynucleotide hybridization sites. In still another example, the noncovalent accessory oligonucleotide bonding sites include noncovalent peptide binding sites and the noncovalent peptide binding sites are selected from one or both of coiled-coil bonding sites and avidin-biotin bonding sites.

In another example, the nanoparticle further includes a single template polynucleotide bonded to the single template site. In yet another example, the scaffold further includes a plurality of accessory oligonucleotides bonded to the plurality of accessory sites.

In another example, the nanoparticle is at least 10 nm in diameter, at least 20 nm in diameter, at least 30 nm in diameter, at least about 40 nm in diameter, at least about 50 nm in diameter, at least about 60 nm in diameter, at least about 70 nm in diameter, at least about 80 nm in diameter, at least about 90 nm in diameter, at least about 100 nm in diameter, at least about 125 nm in diameter, at least about 150 nm in diameter, at least about 175 nm in diameter, at least about 200 nm in diameter, at least about 225 nm in diameter, at least about 250 nm in diameter, at least about 275 nm in diameter, at least about 300 nm in diameter, at least about 325 nm in diameter, at least about 350 nm in diameter, at least about 375 nm in diameter, at least about 400 nm in diameter, at least about 425 nm in diameter, at least about 450 nm in diameter, at least about 475 nm in diameter, at least about 500 nm in diameter, at least about 550 nm in diameter, at least about 600 nm in diameter, at least about 650 nm in diameter, at least about 700 nm in diameter, at least about 750 nm in diameter, at least about 800 nm in diameter, at least about 850 nm in diameter, at least about 900 nm in diameter, or at least about 950 nm in diameter.

In another aspect, provided is a method, including bonding a single template polynucleotide to the single template site of the nanoparticle. In yet another aspect, provided is a method including bonding a plurality of accessory oligonucleotides to the plurality of accessory sites of the nanoparticle. In still another example, the method further includes, synthesizing one or more scaffold-attached copies selected from copies of the template polynucleotide, copies of the polynucleotides complementary to the template polynucleotide, and copies of both, wherein the scaffold-attached copies extend from the accessory oligonucleotides. In yet another example, the method further includes attaching the scaffold to a substrate, wherein attaching comprises hybridizing accessory oligonucleotides with oligonucleotides attached to the substrate.

In an example, the substrate includes a plurality of nanowells and the oligonucleotides attached to the substrate are attached within the plurality of nanowells. In another example, no more than one scaffold binds within any one of the nanowells. In still another example, the method further includes synthesizing one or more substrate-attached copies selected from copies of the template polynucleotide, copies of the polynucleotides complementary to the template polynucleotide, and copies of both, wherein the substrate-attached copies extend from oligonucleotides attached to a substrate. In yet another example, the method further includes sequencing at least one of scaffold-attached copies and substrate-attached copies, wherein sequencing comprises sequencing by synthesis.

In another aspect, provided is a nanoparticle, including a scaffold, a single template site for bonding a template polynucleotide to the scaffold selected from a covalent template bonding site and a noncovalent template bonding site, and a plurality of accessory sites for bonding accessory oligonucleotides to the scaffold selected from covalent accessory oligonucleotide bonding sites and noncovalent accessory oligonucleotide bonding sites, wherein the scaffold comprises a dendrimer wherein the dendrimer includes from 2 to 10 generations of constitutional repeating units, the constitutional repeating units include lysine wherein a lysine of an upstream generation forms a peptide bond with a first lysine of an immediately downstream generation and an isopeptide bond with a second lysine of the immediately downstream generation, the single template site extends from the C-terminal end of the lysine of the first generation of the dendrimer and the plurality of accessory sites extend from NH₂ groups of lysines of the last generation of the dendrimer.

In an example the single template site includes a covalent template bonding site. In another example, the covalent template bonding site is selected from amine-NHS ester bonding site, an amine-imidoester bonding site, an amine-pentofluorophenyl ester bonding site, an amine-hydroxymethyl phosphine bonding site, a carboxyl-carbodiimide bonding site, a thiol-maleimide bonding site, a thiol-haloacetyl bonding site, a thiol-pyridyl disulfide bonding site, a thiol-thiosulfonate bonding site, a thiol-vinyl sulfone bonding site, an aldehyde-hydrazide bonding site, an aldehyde-alkoxyamine bonding site, an aldehyde-NHS ester bonding site, a hydroxy-isocyanate bonding site, an azide-alkyne bonding site, an azide-phosphine bonding site, a transcyclooctene-tetrazine bonding site, a norbornene-tetrazine bonding site, an azide-cyclooctyne bonding site, an azide-norbornene bonding site, an oxime bonding site, a SpyTag-SpyCatcher bonding site, a Snap-tag-O⁶-Benzylguanine bonding site, a CLIP-tag-O²-benzylcytosine bonding site, and a sortase-coupling bonding site.

In another example, the single template site includes a noncovalent template bonding site. In yet another example, the noncovalent template bonding site includes a polynucleotide hybridization site. In still a further example, the noncovalent template bonding site includes a noncovalent peptide binding site and the noncovalent peptide binding site is selected from a coiled-coil bonding site and an avidin-biotin bonding site.

In another example, the plurality of accessory sites for bonding accessory oligonucleotides to the scaffold include covalent accessory oligonucleotide bonding sites. In still another example, the covalent accessory oligonucleotide bonding sites are selected from amine-NHS ester bonding sites, amine-imidoester bonding sites, amine-pentofluorophenyl ester bonding sites, amine-hydroxymethyl phosphine bonding sites, carboxyl-carbodiimide bonding sites, thiol-maleimide bonding sites, thiol-haloacetyl bonding sites, thiol-pyridyl disulfide bonding sites, thiol-thiosulfonate bonding sites, thiol-vinyl sulfone bonding sites, aldehyde-hydrazide bonding sites, aldehyde-alkoxyamine bonding sites, hydroxy-isocyanate bonding sites, azide-alkyne bonding sites, azide-phosphine bonding sites, transcyclooctene-tetrazine bonding sites, norbornene-tetrazine bonding sites, an azide-cyclooctyne bonding sites, azide-norbornene bonding sites, oxime bonding sites, SpyTag-SpyCatcher bonding sites, Snap-tag-O⁶-Benzylguanine bonding sites, CLIP-tag-O²-benzylcytosine bonding sites, sortase-coupling bonding sites, and any combination of two or more of the foregoing.

In another example, the accessory oligonucleotide bonding sites include noncovalent accessory oligonucleotide bonding sites. In yet another example, the noncovalent accessory oligonucleotide bonding sites include polynucleotide hybridization sites. In still another example, the noncovalent accessory oligonucleotide bonding sites include noncovalent peptide binding sites and the noncovalent peptide binding sites are selected from one or both of coiled-coil bonding sites and avidin-biotin bonding sites.

In another example, the nanoparticle further includes a single template polynucleotide bonded to the single template site. In yet another example, the scaffold further includes a plurality of accessory oligonucleotides bonded to the plurality of accessory sites.

In another example, the nanoparticle is at least 10 nm in diameter, at least 20 nm in diameter, at least 30 nm in diameter, at least about 40 nm in diameter, at least about 50 nm in diameter, at least about 60 nm in diameter, at least about 70 nm in diameter, at least about 80 nm in diameter, at least about 90 nm in diameter, at least about 100 nm in diameter, at least about 125 nm in diameter, at least about 150 nm in diameter, at least about 175 nm in diameter, at least about 200 nm in diameter, at least about 225 nm in diameter, at least about 250 nm in diameter, at least about 275 nm in diameter, at least about 300 nm in diameter, at least about 325 nm in diameter, at least about 350 nm in diameter, at least about 375 nm in diameter, at least about 400 nm in diameter, at least about 425 nm in diameter, at least about 450 nm in diameter, at least about 475 nm in diameter, at least about 500 nm in diameter, at least about 550 nm in diameter, at least about 600 nm in diameter, at least about 650 nm in diameter, at least about 700 nm in diameter, at least about 750 nm in diameter, at least about 800 nm in diameter, at least about 850 nm in diameter, at least about 900 nm in diameter, or at least about 950 nm in diameter.

In another aspect, provided is a method, including bonding a single template polynucleotide to the single template site of the nanoparticle. In yet another aspect, provided is a method including bonding a plurality of accessory oligonucleotides to the plurality of accessory sites of the nanoparticle. In still another example, the method further includes, synthesizing one or more scaffold-attached copies selected from copies of the template polynucleotide, copies of the polynucleotides complementary to the template polynucleotide, and copies of both, wherein the scaffold-attached copies extend from the accessory oligonucleotides. In yet another example, the method further includes attaching the scaffold to a substrate, wherein attaching comprises hybridizing accessory oligonucleotides with oligonucleotides attached to the substrate.

In an example, the substrate includes a plurality of nanowells and the oligonucleotides attached to the substrate are attached within the plurality of nanowells. In another example, no more than one scaffold binds within any one of the nanowells. In still another example, the method further includes synthesizing one or more substrate-attached copies selected from copies of the template polynucleotide, copies of the polynucleotides complementary to the template polynucleotide, and copies of both, wherein the substrate-attached copies extend from oligonucleotides attached to a substrate. In yet another example, the method further includes sequencing at least one of scaffold-attached copies and substrate-attached copies, wherein sequencing comprises sequencing by synthesis.

In another aspect, provided is a nanoparticle, including a scaffold, a single template site for bonding a template polynucleotide to the scaffold selected from a covalent template bonding site and a noncovalent template bonding site, and a plurality of accessory sites for bonding accessory oligonucleotides to the scaffold selected from covalent accessory oligonucleotide bonding sites and noncovalent accessory oligonucleotide bonding sites, wherein the scaffold is a compound of Formula IV:
each X is a compound of formula V: 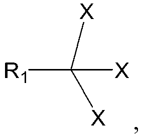 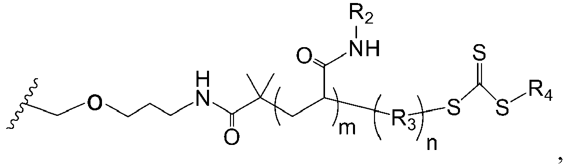
wherein R₂ is selected from Formula VIa: 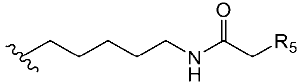 and Formula VIb: 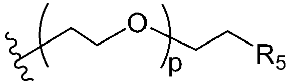 wherein p is an integer selected from 1 to 20, and R₅ includes the accessory site for bonding accessory oligonucleotides, R₃ is selected from a direct bond, 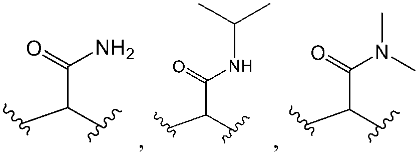 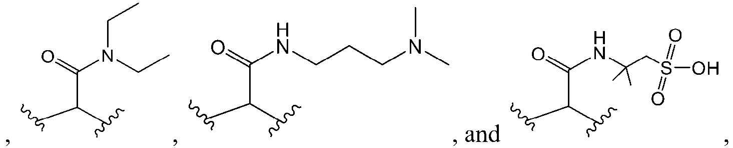 m is an integer from 1 to 2,000 and n is an integer from 1 to 10,000, R¹ includes the single template site for bonding a template polynucleotide to the scaffold, R⁴ is selected from an optionally substituted C₁-C₂₀ alkyl, an optionally substituted C₁-C₂₀ alkenyl, an optionally substituted C₁-C₂₀ alkynyl, an optionally substituted C₁-C₂₀ oxaalkyl, an optionally substituted C₁-C₂₀ thiaalkyl, and an optionally substituted C₁-C₂₀ azaalkyl, wherein substituted includes substitution with one or more of a C₁-C₂₀ alkyl, a double-bonded oxygen, and a hydroxyl group, and R³ includes the accessory site for bonding accessory oligonucleotides. In any of the foregoing examples, the trithiocarbonate group 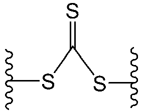 may optionally be replaced by a direct bond, a -CH₂- linkage, an -S- linkage, a -N- linkage, or an -O- linkage.

In an example the single template site includes a covalent template bonding site. In another example, the covalent template bonding site is selected from amine-NHS ester bonding site, an amine-imidoester bonding site, an amine-pentofluorophenyl ester bonding site, an amine-hydroxymethyl phosphine bonding site, a carboxyl-carbodiimide bonding site, a thiol-maleimide bonding site, a thiol-haloacetyl bonding site, a thiol-pyridyl disulfide bonding site, a thiol-thiosulfonate bonding site, a thiol-vinyl sulfone bonding site, an aldehyde-hydrazide bonding site, an aldehyde-alkoxyamine bonding site, an aldehyde-NHS ester bonding site, a hydroxy-isocyanate bonding site, an azide-alkyne bonding site, an azide-phosphine bonding site, a transcyclooctene-tetrazine bonding site, a norbornene-tetrazine bonding site, an azide-cyclooctyne bonding site, an azide-norbornene bonding site, an oxime bonding site, a SpyTag-SpyCatcher bonding site, a Snap-tag-O⁶-Benzylguanine bonding site, a CLIP-tag-O²-benzylcytosine bonding site, and a sortase-coupling bonding site.

In another example, the single template site includes a noncovalent template bonding site. In yet another example, the noncovalent template bonding site includes a polynucleotide hybridization site. In still a further example, the noncovalent template bonding site includes a noncovalent peptide binding site and the noncovalent peptide binding site is selected from a coiled-coil bonding site and an avidin-biotin bonding site.

In another example, the plurality of accessory sites for bonding accessory oligonucleotides to the scaffold include covalent accessory oligonucleotide bonding sites. In still another example, the covalent accessory oligonucleotide bonding sites are selected from amine-NHS ester bonding sites, amine-imidoester bonding sites, amine-pentofluorophenyl ester bonding sites, amine-hydroxymethyl phosphine bonding sites, carboxyl-carbodiimide bonding sites, thiol-maleimide bonding sites, thiol-haloacetyl bonding sites, thiol-pyridyl disulfide bonding sites, thiol-thiosulfonate bonding sites, thiol-vinyl sulfone bonding sites, aldehyde-hydrazide bonding sites, aldehyde-alkoxyamine bonding sites, hydroxy-isocyanate bonding sites, azide-alkyne bonding sites, azide-phosphine bonding sites, transcyclooctene-tetrazine bonding sites, norbornene-tetrazine bonding sites, an azide-cyclooctyne bonding sites, azide-norbornene bonding sites, oxime bonding sites, SpyTag-SpyCatcher bonding sites, Snap-tag-O⁶-Benzylguanine bonding sites, CLIP-tag-O²-benzylcytosine bonding sites, sortase-coupling bonding sites, and any combination of two or more of the foregoing.

In another example, the accessory oligonucleotide bonding sites include noncovalent accessory oligonucleotide bonding sites. In yet another example, the noncovalent accessory oligonucleotide bonding sites include polynucleotide hybridization sites. In still another example, the noncovalent accessory oligonucleotide bonding sites include noncovalent peptide binding sites and the noncovalent peptide binding sites are selected from one or both of coiled-coil bonding sites and avidin-biotin bonding sites.

In another example, the nanoparticle further includes a single template polynucleotide bonded to the single template site. In yet another example, the scaffold further includes a plurality of accessory oligonucleotides bonded to the plurality of accessory sites.

In another example, the nanoparticle is at least 10 nm in diameter, at least 20 nm in diameter, at least 30 nm in diameter, at least about 40 nm in diameter, at least about 50 nm in diameter, at least about 60 nm in diameter, at least about 70 nm in diameter, at least about 80 nm in diameter, at least about 90 nm in diameter, at least about 100 nm in diameter, at least about 125 nm in diameter, at least about 150 nm in diameter, at least about 175 nm in diameter, at least about 200 nm in diameter, at least about 225 nm in diameter, at least about 250 nm in diameter, at least about 275 nm in diameter, at least about 300 nm in diameter, at least about 325 nm in diameter, at least about 350 nm in diameter, at least about 375 nm in diameter, at least about 400 nm in diameter, at least about 425 nm in diameter, at least about 450 nm in diameter, at least about 475 nm in diameter, at least about 500 nm in diameter, at least about 550 nm in diameter, at least about 600 nm in diameter, at least about 650 nm in diameter, at least about 700 nm in diameter, at least about 750 nm in diameter, at least about 800 nm in diameter, at least about 850 nm in diameter, at least about 900 nm in diameter, or at least about 950 nm in diameter.

In another aspect, provided is a method, including bonding a single template polynucleotide to the single template site of the nanoparticle. In yet another aspect, provided is a method including bonding a plurality of accessory oligonucleotides to the plurality of accessory sites of the nanoparticle. In still another example, the method further includes, synthesizing one or more scaffold-attached copies selected from copies of the template polynucleotide, copies of the polynucleotides complementary to the template polynucleotide, and copies of both, wherein the scaffold-attached copies extend from the accessory oligonucleotides. In yet another example, the method further includes attaching the scaffold to a substrate, wherein attaching comprises hybridizing accessory oligonucleotides with oligonucleotides attached to the substrate.

In an example, the substrate includes a plurality of nanowells and the oligonucleotides attached to the substrate are attached within the plurality of nanowells. In another example, no more than one scaffold binds within any one of the nanowells. In still another example, the method further includes synthesizing one or more substrate-attached copies selected from copies of the template polynucleotide, copies of the polynucleotides complementary to the template polynucleotide, and copies of both, wherein the substrate-attached copies extend from oligonucleotides attached to a substrate. In yet another example, the method further includes sequencing at least one of scaffold-attached copies and substrate-attached copies, wherein sequencing comprises sequencing by synthesis.

In another aspect, provided is a nanoparticle, including a scaffold, a single template site for bonding a template polynucleotide to the scaffold selected from a covalent template bonding site and a noncovalent template bonding site, and a plurality of accessory sites for bonding accessory oligonucleotides to the scaffold selected from covalent accessory oligonucleotide bonding sites and noncovalent accessory oligonucleotide bonding sites, wherein the scaffold is a compound of Formula VII: 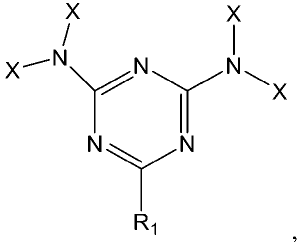
each X is a compound of formula VIII: 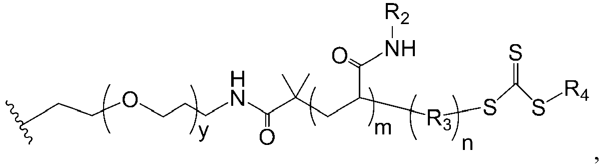
wherein y is an integer from 1 to 20, R₂ is selected from Formula IXa: 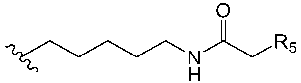 and Formula IXb: 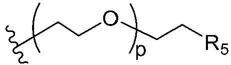 wherein p is an integer selected from 1 to 20, and R₅ includes the accessory site for bonding accessory oligonucleotides, R₃ is selected from a direct bond, 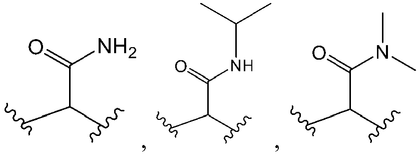 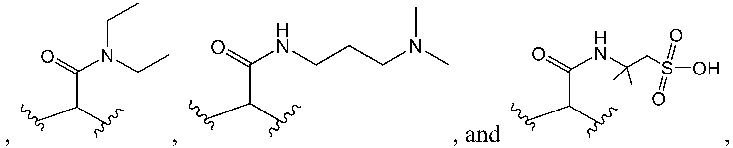 m is an integer from 1 to 2,000 and n is an integer from 1 to 10,000, R¹ includes the single template site for bonding a template polynucleotide to the scaffold, R⁴ is selected from an optionally substituted C₁-C₂₀ alkyl, an optionally substituted C₁-C₂₀ alkenyl, an optionally substituted C₁-C₂₀ alkynyl, an optionally substituted C₁-C₂₀ oxaalkyl, an optionally substituted C₁-C₂₀ thiaalkyl, and an optionally substituted C₁-C₂₀ azaalkyl, wherein substituted includes substitution with one or more of a C₁-C₂₀ alkyl, a double-bonded oxygen, and a hydroxyl group, and R³ includes the accessory site for bonding accessory oligonucleotides. In any of the foregoing examples, the trithiocarbonate group 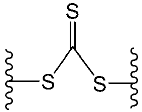 may optionally be replaced by a direct bond, a -CH₂- linkage, an -S- linkage, a -N- linkage, or an -O- linkage.

In an example the single template site includes a covalent template bonding site. In another example, the covalent template bonding site is selected from amine-NHS ester bonding site, an amine-imidoester bonding site, an amine-pentofluorophenyl ester bonding site, an amine-hydroxymethyl phosphine bonding site, a carboxyl-carbodiimide bonding site, a thiol-maleimide bonding site, a thiol-haloacetyl bonding site, a thiol-pyridyl disulfide bonding site, a thiol-thiosulfonate bonding site, a thiol-vinyl sulfone bonding site, an aldehyde-hydrazide bonding site, an aldehyde-alkoxyamine bonding site, an aldehyde-NHS ester bonding site, a hydroxy-isocyanate bonding site, an azide-alkyne bonding site, an azide-phosphine bonding site, a transcyclooctene-tetrazine bonding site, a norbornene-tetrazine bonding site, an azide-cyclooctyne bonding site, an azide-norbornene bonding site, an oxime bonding site, a SpyTag-SpyCatcher bonding site, a Snap-tag-O⁶-Benzylguanine bonding site, a CLIP-tag-O²-benzylcytosine bonding site, and a sortase-coupling bonding site.

In another example, the single template site includes a noncovalent template bonding site. In yet another example, the noncovalent template bonding site includes a polynucleotide hybridization site. In still a further example, the noncovalent template bonding site includes a noncovalent peptide binding site and the noncovalent peptide binding site is selected from a coiled-coil bonding site and an avidin-biotin bonding site.

In another example, the plurality of accessory sites for bonding accessory oligonucleotides to the scaffold include covalent accessory oligonucleotide bonding sites. In still another example, the covalent accessory oligonucleotide bonding sites are selected from amine-NHS ester bonding sites, amine-imidoester bonding sites, amine-pentofluorophenyl ester bonding sites, amine-hydroxymethyl phosphine bonding sites, carboxyl-carbodiimide bonding sites, thiol-maleimide bonding sites, thiol-haloacetyl bonding sites, thiol-pyridyl disulfide bonding sites, thiol-thiosulfonate bonding sites, thiol-vinyl sulfone bonding sites, aldehyde-hydrazide bonding sites, aldehyde-alkoxyamine bonding sites, hydroxy-isocyanate bonding sites, azide-alkyne bonding sites, azide-phosphine bonding sites, transcyclooctene-tetrazine bonding sites, norbornene-tetrazine bonding sites, an azide-cyclooctyne bonding sites, azide-norbornene bonding sites, oxime bonding sites, SpyTag-SpyCatcher bonding sites, Snap-tag-O⁶-Benzylguanine bonding sites, CLIP-tag-O²-benzylcytosine bonding sites, sortase-coupling bonding sites, and any combination of two or more of the foregoing.

In another example, the accessory oligonucleotide bonding sites include noncovalent accessory oligonucleotide bonding sites. In yet another example, the noncovalent accessory oligonucleotide bonding sites include polynucleotide hybridization sites. In still another example, the noncovalent accessory oligonucleotide bonding sites include noncovalent peptide binding sites and the noncovalent peptide binding sites are selected from one or both of coiled-coil bonding sites and avidin-biotin bonding sites.

In another example, the nanoparticle further includes a single template polynucleotide bonded to the single template site. In yet another example, the scaffold further includes a plurality of accessory oligonucleotides bonded to the plurality of accessory sites.

In another example, the nanoparticle is at least 10 nm in diameter, at least 20 nm in diameter, at least 30 nm in diameter, at least about 40 nm in diameter, at least about 50 nm in diameter, at least about 60 nm in diameter, at least about 70 nm in diameter, at least about 80 nm in diameter, at least about 90 nm in diameter, at least about 100 nm in diameter, at least about 125 nm in diameter, at least about 150 nm in diameter, at least about 175 nm in diameter, at least about 200 nm in diameter, at least about 225 nm in diameter, at least about 250 nm in diameter, at least about 275 nm in diameter, at least about 300 nm in diameter, at least about 325 nm in diameter, at least about 350 nm in diameter, at least about 375 nm in diameter, at least about 400 nm in diameter, at least about 425 nm in diameter, at least about 450 nm in diameter, at least about 475 nm in diameter, at least about 500 nm in diameter, at least about 550 nm in diameter, at least about 600 nm in diameter, at least about 650 nm in diameter, at least about 700 nm in diameter, at least about 750 nm in diameter, at least about 800 nm in diameter, at least about 850 nm in diameter, at least about 900 nm in diameter, or at least about 950 nm in diameter.

In another aspect, provided is a method, including bonding a single template polynucleotide to the single template site of the nanoparticle. In yet another aspect, provided is a method including bonding a plurality of accessory oligonucleotides to the plurality of accessory sites of the nanoparticle. In still another example, the method further includes, synthesizing one or more scaffold-attached copies selected from copies of the template polynucleotide, copies of the polynucleotides complementary to the template polynucleotide, and copies of both, wherein the scaffold-attached copies extend from the accessory oligonucleotides. In yet another example, the method further includes attaching the scaffold to a substrate, wherein attaching comprises hybridizing accessory oligonucleotides with oligonucleotides attached to the substrate.

In an example, the substrate includes a plurality of nanowells and the oligonucleotides attached to the substrate are attached within the plurality of nanowells. In another example, no more than one scaffold binds within any one of the nanowells. In still another example, the method further includes synthesizing one or more substrate-attached copies selected from copies of the template polynucleotide, copies of the polynucleotides complementary to the template polynucleotide, and copies of both, wherein the substrate-attached copies extend from oligonucleotides attached to a substrate. In yet another example, the method further includes sequencing at least one of scaffold-attached copies and substrate-attached copies, wherein sequencing comprises sequencing by synthesis.

### BRIEF DESCRIPTION OF THE DRAWINGS

These and other features, aspects, and advantages of the present disclosure will become better understood when the following detailed description is read with reference to the accompanying drawings, wherein:
FIG. 1 shows an example of a nanoparticle in accordance with aspects of the present disclosure.
FIG. 2 shows an example of portions of a scaffold of a nanoparticle in accordance with aspects of the present disclosure.
FIG. 3 shows an example of a single template polynucleotide site and accessory attachment sites of a nanoparticle in accordance with aspects of the present disclosure.
FIG. 4 shows an example a method of synthesizing a nanoparticle in accordance with the present disclosure.
FIG. 5 shows example of a nanoparticle having a dendrimer structure with constitutional repeating units including lysine.
FIG. 6 shows a synthesis method for synthesizing examples of a nanoparticle in accordance with aspects of the present disclosure.
FIG. 7 shows examples of attachment of a template to a nanoparticle in accordance with aspects of the present disclosure.
FIG. 8 shows an example of noncovalently attaching a template polynucleotide to a nanoparticle by hybridization, in accordance with aspects of the present disclosure.
FIG. 9 shows an example of noncovalently attaching a template polynucleotide to a nanoparticle by a coiled-coil peptide binding site, in accordance with aspects of the present disclosure.
FIG. 10 is a graph showing a number of nanoparticles per nanowell according to nanowell surface area, in accordance with aspects of the present invention.
FIGs. 11A-11C show an example of seeding a substrate with template polynucleotides using a scaffold in accordance with aspects of the present disclosure.

### DETAILED DESCRIPTION

Reference throughout the specification to "one example", "another example", "an example", and so forth, means that a particular element (e.g., feature, structure, and/or characteristic) described in connection with the example is included in at least one example described herein, and may or may not be present in other examples. In addition, it is to be understood that the described elements for any example may be combined in any suitable manner in the various examples unless the context clearly dictates otherwise.

This disclosure relates to compositions and methods for increasing monoclonal clustering during SBS. In an example, principles of size exclusion are used to prevent individual template polynucleotides from seeding and therefore promoting clustering too close to each other. By associating each individual template polynucleotide with a nanoparticle of a given, sufficient spatial dimension, the template polynucleotides may be induced to attach to a substrate's surface sufficiently distal from each other to reduce formation of polyclonal clusters and increase formation of monoclonal clusters. A nanoparticle may include a bonding site for a template polynucleotide. The nanoparticle may have only one, single site for attachment of a template polynucleotide. One and only one template polynucleotide may therefore be capable of attaching to a nanoparticle, such that attachment of a template polynucleotide to the scaffold prevents attachment of a second template polynucleotide to the same nanoparticle, the attached template polynucleotide having occupied the single template polynucleotide bonding site thereof. Attachment of only a single template polynucleotide per nanoparticle, and resulting spatial distribution of template polynucleotides attached to such nanoparticles from each other due, directly or indirectly, to the sizes of the attached nanoparticles, reduces formation of polyclonal clusters.

The nanoparticle may also include other types of one or more bonding sites for attachment of the nanoparticle to compositions or surfaces in addition to a template polynucleotide, referred to herein as accessory bonding sites. For example, in addition to a single template polynucleotide bonding site, a nanoparticle may include accessory bonding sites that permit attachment of the nanoparticle to the surface of a substrate for us in an SBS process. In another example, a nanoparticle may possess one or more accessory bonding sites for attachment of one or more surface polymers to the nanoparticle. In another example, a nanoparticle may include one or more accessory bonding sites for attachment of an accessory oligonucleotide to the nanoparticle, wherein the oligonucleotide may bind to an end of a template polynucleotide or copy thereof as part of a clustering process, as described more fully below. In another example, such accessory oligonucleotides may be hybridizable to oligonucleotides attached to a surface of a substrate for use in an SBS process such that the nanoparticle with single template polynucleotide attached thereto may attach to such substrate surface.

Whereas a scaffold may include a single bonding site for a template polynucleotide and one or more accessory sites for attachment of, for example, an accessory oligonucleotide, the single template polynucleotide bonding site may be of a chemistry or structure different from that of accessory bonding sites. Of all of the bonding sites, the single template polynucleotide bonding site may be the only one having a chemistry or structure designed for attaching to a template polynucleotide with a corresponding chemistry or structure for attachment thereto. By comparison, the one or more accessory bonding sites may possess a different chemistry or structure, which is not compatible with binding or attaching to a template polynucleotide. Rather, the one or more accessory bonding sites may have a chemistry or structure compatible for binding or attaching to other compositions or structures to which the accessory bonding sites are intended to bind, such as accessory oligonucleotides, polymers, etc., and incompatible with binding or attaching to a template polynucleotide. Thus, a template polynucleotide would be incapable of binding or attaching to the one or more accessory bonding sites, resulting in attachment of only one template polynucleotide per nanoparticle, at the single template polynucleotide bonding site of the nanoparticle.

A template polynucleotide may be a polynucleotide obtained from a sample, such as a polydeoxyribonucleic acid isolated from a sample, or a cDNA molecule copied from a mRNA molecule that was obtained from a sample. An SBS process may be performed, for example, to determine a nucleotide sequence of a template polynucleotide, or to identify one or more polymorphisms or alterations in genetic sequence of a template polynucleotide in comparison to a reference sequence. A library may be prepared from one or more samples, the library including a plurality of template polynucleotides obtained from the one or more samples. Template polynucleotides may be obtained by obtaining polynucleotide sequences that are portions of sequences that were present in the sample or copied from the sample. By sequencing a plurality of template polynucleotides in an SBS process, sequence, genotype, or other sequence-related information may be determined as to the template polynucleotides and, when sequence information about a plurality of template polynucleotides in a library is collected an analyzed, about the sample from which the library was obtained.

A template polynucleotide may be processed as part of a process of obtaining a template polynucleotide from sample. Part of processing may include adding polynucleotide sequences, such as to the 5-prime, 3-prime, or both ends of the template to assist in subsequence SBS processing. As further disclosed herein, a template polynucleotide may further be modified by adding features that promote or permit forming a bond with a site on a nanoparticle.

A template polynucleotide may be of any given length suitable for obtaining sequencing information in an SBS process. For example, a template polynucleotide may be about 50 nucleotides in length, about 75 nucleotides in length, about 100 nucleotides in length, about 125 nucleotides in length, about 150 nucleotides in length, about 175 nucleotides in length, about 200 nucleotides in length nucleotides in length, about 225 nucleotides in length, about 250 nucleotides in length, about 275 nucleotides in length, about 300 nucleotides in length, about 325 nucleotides in length, about 350 nucleotides in length, about 375 nucleotides in length, about 400 nucleotides in length, about 425 nucleotides in length, about 450 nucleotides in length, about 475 nucleotides in length, about 500 nucleotides in length, about 525 nucleotides in length, about 550 nucleotides in length, about 575 nucleotides in length, about 600 nucleotides in length, about 625 nucleotides in length, about 650 nucleotides in length, about 675 nucleotides in length, about 700 nucleotides in length, about 725 nucleotides in length, about 750 nucleotides in length, about 775 nucleotides in length, about 800 nucleotides in length, about 825 nucleotides in length, about 850 nucleotides in length, about 875 nucleotides in length, about 900 nucleotides in length, about 925 nucleotides in length, about 950 nucleotides in length, about 975 nucleotides in length, about 1,000 nucleotides in length, about 1,025 nucleotides in length, about 1,050 nucleotides in length, about 1,075 nucleotides in length, about 1,100 nucleotides in length, about, 1,125 nucleotides in length, about 1,150 nucleotides in length, about 1,175 nucleotides in length, about 1,200 nucleotides in length, about 1,225 nucleotides in length, about 1,250 nucleotides in length, about 1,275 nucleotides in length, about 1,300 nucleotides in length, about 1,325 nucleotides in length, about 1,350 nucleotides in length, about 1,375 nucleotides in length, about 1,400 nucleotides in length, about 1,425 nucleotides in length, about 1,450 nucleotides in length, about 1,475 nucleotides in length, about 1,500 nucleotides in length, or longer.

In some examples, there may be two or more different populations of accessory bonding sites on a nanoparticle, some with one type of chemistry or structure compatible with binding or attaching to one population of compositions or structures, and others with a second type of chemistry or structure compatible with binding or attaching to another population of compositions or structures. For example, one population of accessory sites may have a chemistry or structure compatible with binding to accessory oligonucleotides which accessory oligonucleotides may bind to copies of template polynucleotides that participate in, for example, clustering of a template polynucleotide on a nanoparticle, as described more fully below, while other accessory sites may have a different chemistry or structure compatible with binding or attaching to a surface of a substrate for performing SBS.

A nanoparticle may include a scaffold. A scaffold is a structural component of a nanoparticle occupying volume according to a minimum amount of distance desired between template nanoparticles or a maximum density of template nanoparticles attached to nanoparticles as may be desirable for a given application. A scaffold may include the aforementioned bonding sites, as in single template polynucleotide binding site and one or more accessory bonding site. Together, the scaffold with bonding sites, may constitute a nanoparticle. A scaffold may be synthesized so as to include, and may include once synthesized, more than one type of chemistry or structure for attachment. That is, it may be synthesized to include or be modified to include a single site of attachment to a template polynucleotide, plus one or more additional bonding sites with a different chemistry or structure from the single template polynucleotide bonding site corresponding to accessory bonding sites.

A scaffold may include an asymmetrical polymer, wherein several polymer chains extend from a scaffold core, which also includes a different binding site for a template polynucleotide. Polymer chains, linear or branched, may extend from the scaffold core, with accessory bonding sites on the polymers, and another bonding site with an orthogonal bonding chemistry relative to that of the accessory bonding sites is present on the scaffold core for bonding to a single template polynucleotide. In an example, a scaffold may include a core from which acrylamide monomer-containing heteropolymers or homopolymers extend including accessory bonding sites, and another bonding site with a different bonding chemistry for bonding a template polynucleotide. In an example, a scaffold core may include attachment points for two or three linear or branched polymers.

In an example, a scaffold may include two or three linear or branched polymers individually liked to a scaffold core. A non-limiting example of a scaffold is a compound of Formula I: 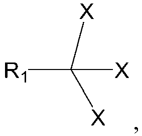
each X is a compound of formula II: 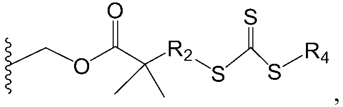
wherein R₂ is selected from Formula IIIa: 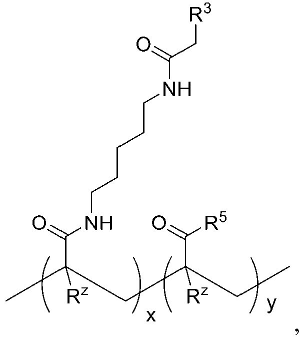 wherein R⁵ is selected from 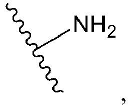 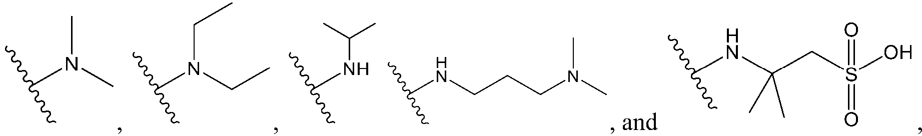 X is an integer in the range of from 1-2,000 and y is an integer in the range of from 1-10,000 and a ratio of x:y may be from approximately 10:90 to approximately 1:99, and wherein each R^{z} is independently H or C₁₋₄ alkyl, and Formula IIIb: [0067] 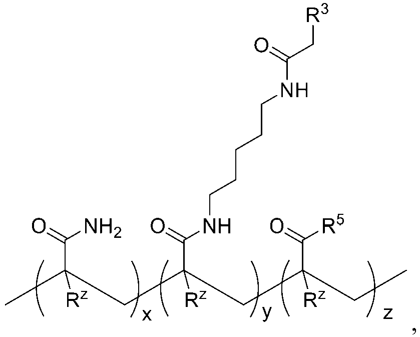 wherein R⁵ is selected from 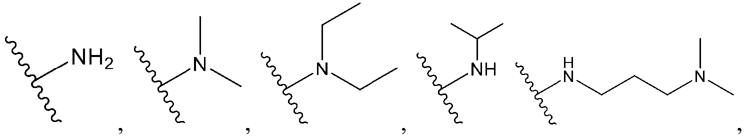 and 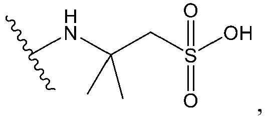 y is an integer in the range of from 1-2,000 and x and z are integers whose sum is in a range of from 1-10,000 and a ratio of (x:y):z may be from approximately (85):15 to approximately (95):5, and wherein each R^{z} is independently H or C₁₋₄ alkyl, R₁ includes the single template site for bonding a template polynucleotide to the scaffold, R⁴ is selected from an optionally substituted C₁-C₂₀ alkyl, an optionally substituted C₁-C₂₀ alkenyl, an optionally substituted C₁-C₂₀ alkynyl, an optionally substituted C₁-C₂₀ oxaalkyl, an optionally substituted C₁-C₂₀ thiaalkyl, and an optionally substituted C₁-C₂₀ azaalkyl, wherein substituted includes substitution with one or more of a C₁-C₂₀ alkyl, a double-bonded oxygen, and a hydroxyl group, and R³ includes the accessory site for bonding accessory oligonucleotides. In any of the foregoing examples, the trithiocarbonate group 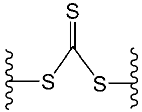 may optionally be replaced by a direct bond, a -CH₂- linkage, an -S- linkage, a -N- linkage, or an -O- linkage.

In another example, where R₂ is a compound of Formula IIIa 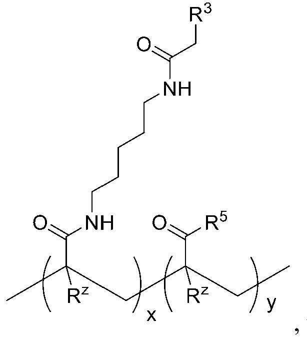 y may be 0 and x may be an integer of from 1 to 2,000.

In an example, R₁ includes an amine-NHS ester bonding site, an amine-imidoester bonding site, an amine-pentofluorophenyl ester bonding site, an amine-hydroxymethyl phosphine bonding site, a carboxyl-carbodiimide bonding site, a thiol-maleimide bonding site, a thiol-haloacetyl bonding site, a thiol-pyridyl disulfide bonding site, a thiol-thiosulfonate bonding site, a thiol-vinyl sulfone bonding site, an aldehyde-hydrazide bonding site, an aldehyde-alkoxyamine bonding site, an aldehyde-NHS ester bonding site, a hydroxy-isocyanate bonding site, an azide-alkyne bonding site, an azide-phosphine bonding site, a transcyclooctene-tetrazine bonding site, a norbornene-tetrazine bonding site, an azide-cyclooctyne bonding site, an azide-norbornene bonding site, an oxime bonding site, a SpyTag-SpyCatcher bonding site, a Snap-tag-O⁶-Benzylguanine bonding site, a CLIP-tag-O²-benzylcytosine bonding site, or a sortase-coupling bonding site.. In a specific non-limiting example, R₁ includes an amine group, a tetrazine group, or a dibenzocyclooctene group.

In an example, each R³ may include an amine-NHS ester bonding site, an amine-imidoester bonding site, an amine-pentofluorophenyl ester bonding site, an amine-hydroxymethyl phosphine bonding site, a carboxyl-carbodiimide bonding site, a thiol-maleimide bonding site, a thiol-haloacetyl bonding site, a thiol-pyridyl disulfide bonding site, a thiol-thiosulfonate bonding site, a thiol-vinyl sulfone bonding site, an aldehyde-hydrazide bonding site, an aldehyde-alkoxyamine bonding site, an aldehyde-NHS ester bonding site, a hydroxy-isocyanate bonding site, an azide-alkyne bonding site, an azide-phosphine bonding site, a transcyclooctene-tetrazine bonding site, a norbornene-tetrazine bonding site, an azide-cyclooctyne bonding site, an azide-norbornene bonding site, an oxime bonding site, a SpyTag-SpyCatcher bonding site, a Snap-tag-O⁶-Benzylguanine bonding site, a CLIP-tag-O²-benzylcytosine bonding site, or a sortase-coupling bonding site. In a specific non-limiting example, each R³ includes an azide group.

In a specific, non-limiting example, the scaffold includes the following structure (wherein only one X is drawn in full, for simplicity of depiction, but the other two Xs have the same structure as that of the fully drawn X):

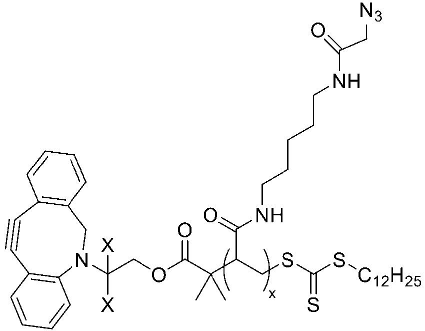

In another example, X may include 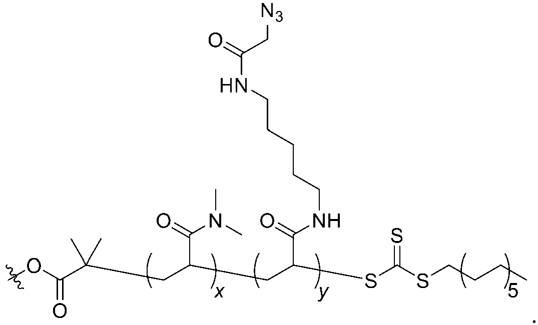 In another example, X may include 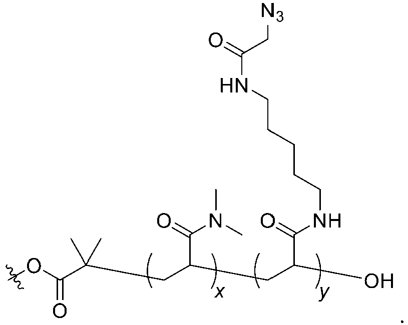 In another example, X may include

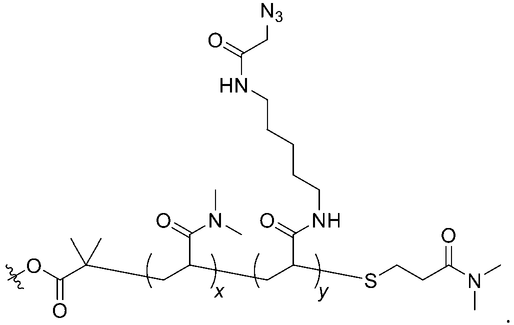

In another example, R¹ is bonded to a template polynucleotide, and one or more R³ is bonded to an accessory, such as an accessory oligonucleotide. In another example, the accessory oligonucleotide is attached to a nucleotide sequence that is a copy of a template polynucleotide or is complementary to the template polynucleotide.

Another non-limiting example of a scaffold is a compound of Formula IV 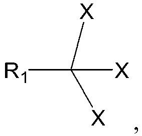
each X is a compound of formula V: 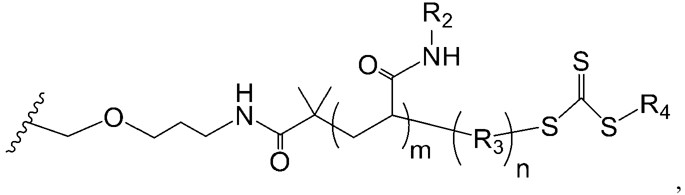
wherein R₂ is selected from Formula VIa: 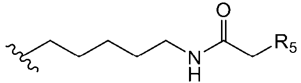 and Formula VIb: 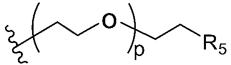 wherein p is an integer selected from 1 to 20, and R₅ includes the accessory site for bonding accessory oligonucleotides, R₃ is selected from a direct bond, 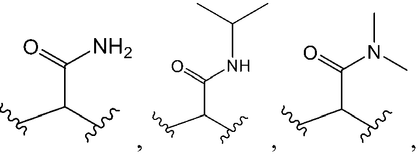 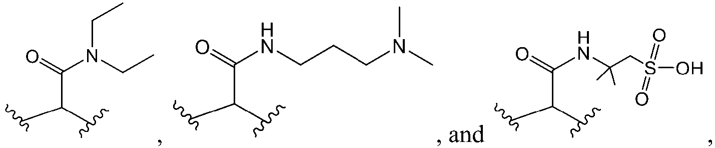 m is an integer from 1 to 2,000 and n is an integer from 1 to 10,000, R¹ includes the single template site for bonding a template polynucleotide to the scaffold, R⁴ is selected from an optionally substituted C₁-C₂₀ alkyl, an optionally substituted C₁-C₂₀ alkenyl, an optionally substituted C₁-C₂₀ alkynyl, an optionally substituted C₁-C₂₀ oxaalkyl, an optionally substituted C₁-C₂₀ thiaalkyl, and an optionally substituted C₁-C₂₀ azaalkyl, wherein substituted includes substitution with one or more of a C₁-C₂₀ alkyl, a double-bonded oxygen, and a hydroxyl group, and R³ includes the accessory site for bonding accessory oligonucleotides. In any of the foregoing examples, the trithiocarbonate group 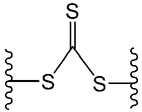 may optionally be replaced by a direct bond, a -CH₂-linkage, an -S- linkage, a -N- linkage, or an -O- linkage.

In an example, R₁ includes an amine-NHS ester bonding site, an amine-imidoester bonding site, an amine-pentofluorophenyl ester bonding site, an amine-hydroxymethyl phosphine bonding site, a carboxyl-carbodiimide bonding site, a thiol-maleimide bonding site, a thiol-haloacetyl bonding site, a thiol-pyridyl disulfide bonding site, a thiol-thiosulfonate bonding site, a thiol-vinyl sulfone bonding site, an aldehyde-hydrazide bonding site, an aldehyde-alkoxyamine bonding site, an aldehyde-NHS ester bonding site, a hydroxy-isocyanate bonding site, an azide-alkyne bonding site, an azide-phosphine bonding site, a transcyclooctene-tetrazine bonding site, a norbornene-tetrazine bonding site, an azide-cyclooctyne bonding site, an azide-norbornene bonding site, an oxime bonding site, a SpyTag-SpyCatcher bonding site, a Snap-tag-O⁶-Benzylguanine bonding site, a CLIP-tag-O²-benzylcytosine bonding site, or a sortase-coupling bonding site. In another example, R₁ includes a coiled-coil bonding site or an avidin-biotin bonding site. . In a specific non-limiting example, R₁ includes an amine group, a tetrazine group, or a dibenzocyclooctene group.

In an example, each R₅ may include an amine-NHS ester bonding site, an amine-imidoester bonding site, an amine-pentofluorophenyl ester bonding site, an amine-hydroxymethyl phosphine bonding site, a carboxyl-carbodiimide bonding site, a thiol-maleimide bonding site, a thiol-haloacetyl bonding site, a thiol-pyridyl disulfide bonding site, a thiol-thiosulfonate bonding site, a thiol-vinyl sulfone bonding site, an aldehyde-hydrazide bonding site, an aldehyde-alkoxyamine bonding site, an aldehyde-NHS ester bonding site, a hydroxy-isocyanate bonding site, an azide-alkyne bonding site, an azide-phosphine bonding site, a transcyclooctene-tetrazine bonding site, a norbornene-tetrazine bonding site, an azide-cyclooctyne bonding site, an azide-norbornene bonding site, an oxime bonding site, a SpyTag-SpyCatcher bonding site, a Snap-tag-O⁶-Benzylguanine bonding site, a CLIP-tag-O²-benzylcytosine bonding site, or a sortase-coupling bonding site. In another example, each R₅ includes a coiled-coil bonding site or an avidin-biotin bonding site. In a specific non-limiting example, each R₅ includes an azide group.

In a specific, non-limiting example, the scaffold includes the following structure (wherein only one X is drawn in full, for simplicity of depiction, but the other two Xs have the same structure as that of the fully drawn X):

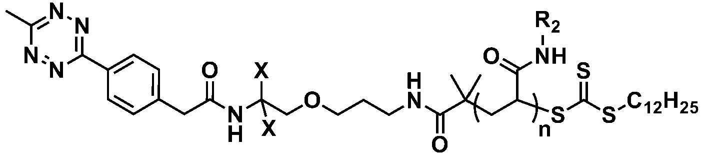

In a specific, non-limiting example, the scaffold includes the following structure (wherein only one X is drawn in full, for simplicity of depiction, but the other two Xs have the same structure as that of the fully drawn X): 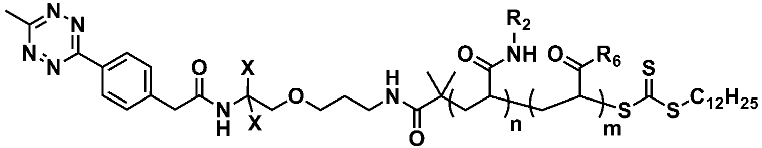 wherein R₆ is selected from

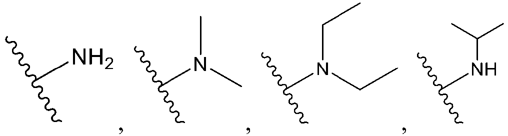

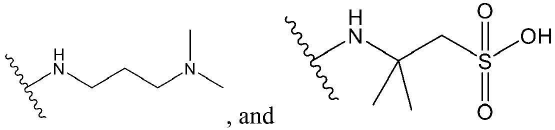

In another specific, non-limiting example, the scaffold includes the following structure (wherein only one X is drawn in full, for simplicity of depiction, but the other two Xs have the same structure as that of the fully drawn X):

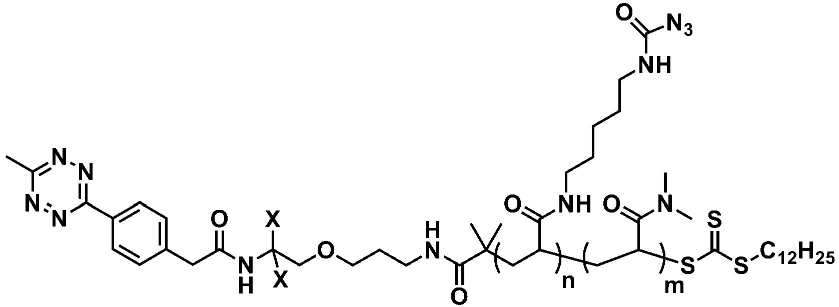

In another specific, non-limiting example, the scaffold includes the following structure (wherein only one X is drawn in full, for simplicity of depiction, but the other two Xs have the same structure as that of the fully drawn X):

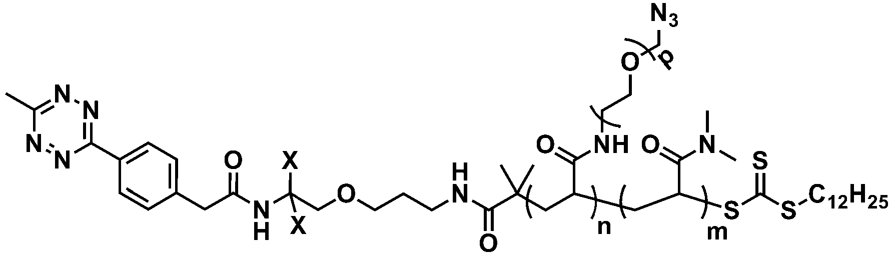

In another example, R¹ is bonded to a template polynucleotide, and one or more R⁵ is bonded to an accessory, such as an accessory oligonucleotide. In another example, the accessory oligonucleotide is attached to a nucleotide sequence that is a copy of a template polynucleotide or is complementary to the template polynucleotide.

In another example, the scaffold core may be or may have originated from a precursor triazine molecule. A non-limiting example of a scaffold is a compound of Formula IV: 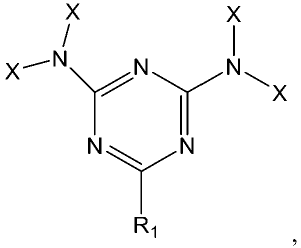
each X is a compound of formula VIII: 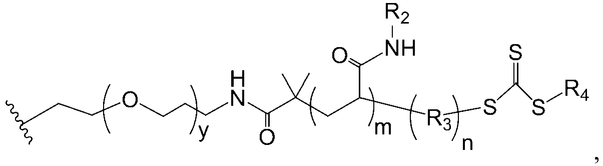
wherein y is an integer from 1 to 20, R₂ is selected from Formula IXa: 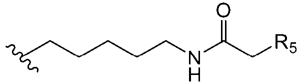 and Formula IXb: 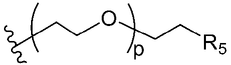 wherein p is an integer selected from 1 to 20, and R₅ includes the accessory site for bonding accessory oligonucleotides, R₃ is selected from a direct bond, 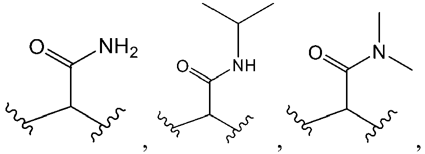 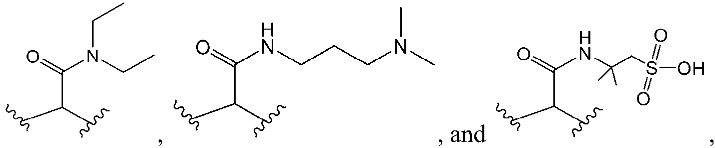 m is an integer from 1 to 2,000 and n is an integer from 1 to 10,000, R¹ includes the single template site for bonding a template polynucleotide to the scaffold, R⁴ is selected from an optionally substituted C₁-C₂₀ alkyl, an optionally substituted C₁-C₂₀ alkenyl, an optionally substituted C₁-C₂₀ alkynyl, an optionally substituted C₁-C₂₀ oxaalkyl, an optionally substituted C₁-C₂₀ thiaalkyl, and an optionally substituted C₁-C₂₀ azaalkyl, wherein substituted includes substitution with one or more of a C₁-C₂₀ alkyl, a double-bonded oxygen, and a hydroxyl group, and R³ includes the accessory site for bonding accessory oligonucleotides. In any of the foregoing examples, the trithiocarbonate group 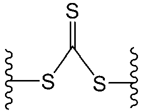 may optionally be replaced by a direct bond, a -CH₂-linkage, an -S- linkage, a -N- linkage, or an -O- linkage.

In an example, R₁ includes an amine-NHS ester bonding site, an amine-imidoester bonding site, an amine-pentofluorophenyl ester bonding site, an amine-hydroxymethyl phosphine bonding site, a carboxyl-carbodiimide bonding site, a thiol-maleimide bonding site, a thiol-haloacetyl bonding site, a thiol-pyridyl disulfide bonding site, a thiol-thiosulfonate bonding site, a thiol-vinyl sulfone bonding site, an aldehyde-hydrazide bonding site, an aldehyde-alkoxyamine bonding site, an aldehyde-NHS ester bonding site, a hydroxy-isocyanate bonding site, an azide-alkyne bonding site, an azide-phosphine bonding site, a transcyclooctene-tetrazine bonding site, a norbornene-tetrazine bonding site, an azide-cyclooctyne bonding site, an azide-norbornene bonding site, an oxime bonding site, a SpyTag-SpyCatcher bonding site, a Snap-tag-O⁶-Benzylguanine bonding site, a CLIP-tag-O²-benzylcytosine bonding site, or a sortase-coupling bonding site. In another example, R₁ includes a coiled-coil bonding site or an avidin-biotin bonding site. In a specific non-limiting example, R₁ includes an amine group, a tetrazine group, or a dibenzocyclooctene group.

In an example, each R₅ may include an amine-NHS ester bonding site, an amine-imidoester bonding site, an amine-pentofluorophenyl ester bonding site, an amine-hydroxymethyl phosphine bonding site, a carboxyl-carbodiimide bonding site, a thiol-maleimide bonding site, a thiol-haloacetyl bonding site, a thiol-pyridyl disulfide bonding site, a thiol-thiosulfonate bonding site, a thiol-vinyl sulfone bonding site, an aldehyde-hydrazide bonding site, an aldehyde-alkoxyamine bonding site, an aldehyde-NHS ester bonding site, a hydroxy-isocyanate bonding site, an azide-alkyne bonding site, an azide-phosphine bonding site, a transcyclooctene-tetrazine bonding site, a norbornene-tetrazine bonding site, an azide-cyclooctyne bonding site, an azide-norbornene bonding site, an oxime bonding site, a SpyTag-SpyCatcher bonding site, a Snap-tag-O⁶-Benzylguanine bonding site, a CLIP-tag-O²-benzylcytosine bonding site, or a sortase-coupling bonding site. In another example, each R₅ includes a coiled-coil bonding site or an avidin-biotin bonding site. In a specific non-limiting example, each R₅ includes an azide group.

In a specific, non-limiting example, the scaffold includes the following structure (wherein only one X is drawn in full, for simplicity of depiction, but the other two Xs have the same structure as that of the fully drawn X):

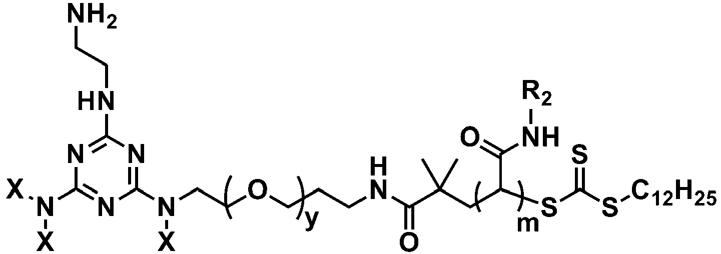

In another specific, non-limiting example, the scaffold includes the following structure (wherein only one X is drawn in full, for simplicity of depiction, but the other two Xs have the same structure as that of the fully drawn X): 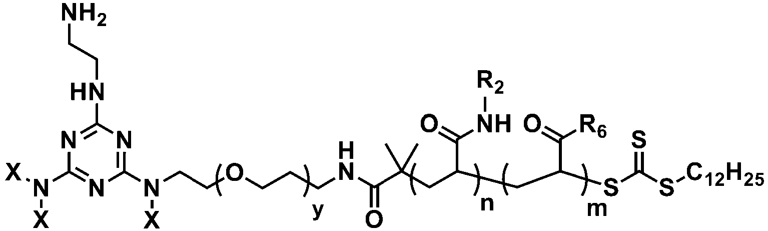 wherein R₆ is selected from

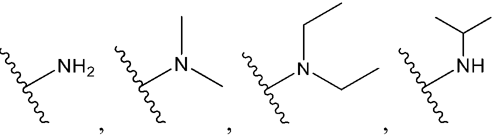

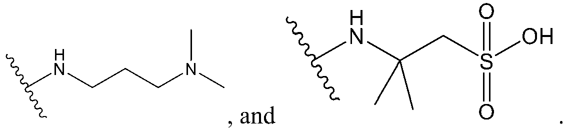

In another specific, non-limiting example, the scaffold includes the following structure (wherein only one X is drawn in full, for simplicity of depiction, but the other two Xs have the same structure as that of the fully drawn X):

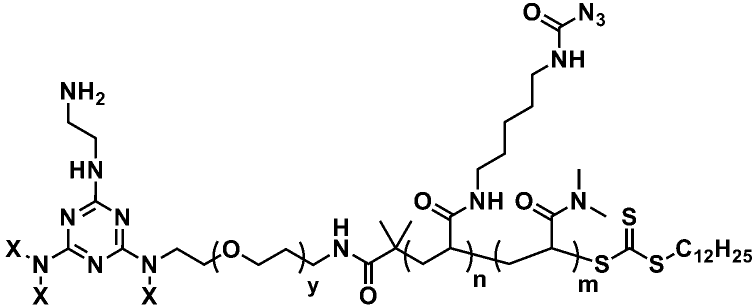

In another specific, non-limiting example, the scaffold includes the following structure (wherein only one X is drawn in full, for simplicity of depiction, but the other two Xs have the same structure as that of the fully drawn X):

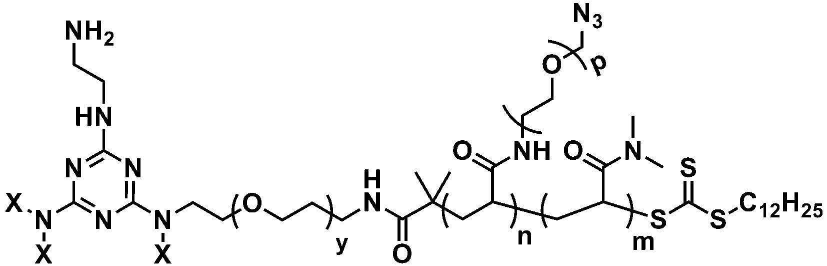

In another example, R¹ is bonded to a template polynucleotide, and one or more R⁵ is bonded to an accessory, such as an accessory oligonucleotide. In another example, the accessory oligonucleotide is attached to a nucleotide sequence that is a copy of a template polynucleotide or is complementary to the template polynucleotide.

Polymer chains may be grown from a scaffold core using controlled radical polymerization (CRP) methods. a polymer may be grown from a scaffold core by a Reversible Addition-Fragmentation Chain Transfer (RAFT) polymerization method, an ATRP (Atom Transfer Radical Polymerization) method or an NMP (nitroxide-mediated radical polymerization) method. In another example, a polymer may be synthesized then bonded to a scaffold core. A CRP method may include tight control over the degree of polymerization (DP) of polymers attached to a scaffold core, and thus controlling polymer molecular weight and nanoparticle size. For example, a DP of about 100 per chain, about 150 per chain, about 200 per chain, about 250 per chain about 300 per chain, about 350 per chain, about 400 per chain, about 450 per chain, about 500 per chain, about 550 per chain, about 600 per chain, about 650 per chain, about 700 per chain, about 750 per chain may be used, about 800 per chain, about 850 per chain, about 900 per chain, about 950 per chain, about 1,000 per chain, about 1,050 per chain, about 1,100 per chain, about 1,150 per chain, about 1,200 per chain, about 1,250 per chain, about 1,300 per chain, about 1,350 per chain, about 1,400 per chain, about 1,450 per chain, about 1,500 per chain, about 1,550 per chain, about 1,600 per chain, about 1,650 per chain, about 1,700 per chain, about 1,750 per chain, about 1,800 per chain, about 1,850 per chain, about 1,900 per chain, about 1,950, or about 2,000 per chain. In some examples, after growing a first such polymer from a scaffold core, a second such polymer may further be extended from the fist polymer by a RAFT process, as a "living" RAFT polymerization process.

In another example, a scaffold may include a dendron wherein a constitutional repeating until includes a lysine amino acid, and wherein the lysine is a branch point, polymerization occurring by formation of a peptide bond between a carboxylic acid of an amino acid the alpha-amino group of the lysine of the immediately upstream generation, and an isopeptide bond between an epsilon-amino terminal of the lysine of the immediately upstream generation. A lysine thereby functions as a branch point in the dendrimer structure. In an example, a core unit of the dendrimer may include a lysine. For example, the carboxylic acid of a core unit lysine may attach to a single template polynucleotide bonding site, whereas the alpha- and epsilon- amino groups may branch and attach to downstream amino acids. For example, a cysteine may be attached to the core lysine, providing a single thiol template polynucleotide bonding site. In other examples, other amino acids, modified amino acids, or other structures may extend from the core lysine to provide a single template polynucleotide bonding site. End groups of the dendrons most downstream branches may include accessory bonding sites. For example, accessory bonding sites may include lysyl alpha- and epsilon-amino groups of the terminal generations of the dendron. As used herein, the terms downstream and upstream refer to directions along a chain of a branch in relation to a core unit of the dendron, with upstream meaning in the direction of the core unit and downstream meaning in the direction of the end units of the branch chains. A dendron may have 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, or more generations.

In an example, an upstream lysine attaches to two downstream lysines via its alpha- and epsilon- amino groups. Each such downstream lysine may likewise attach to two further lysines via formation of peptide and isopeptide bonds with their alpha- and epsilon-amino groups, resulting in a dendron of constitutional repeating units of lysines, each attached via their upstream carboxylic acid to an amino group of an upstream lysine and two downstream lysines via its amino groups.

In another example, a scaffold may include a first polypeptide sequence including one or more lysine residues in the polypeptide. One or more such lysine may form an isopeptide bond to a next polypeptide via its epsilon amino group as well as a peptide bond to a neighboring amino acid in the polypeptide via its alpha-amino group. Such next polypeptide may also be or include one or more lysine, and one or more such lysine of the next polypeptide may form an isopeptide bond to an additional polypeptide via its epsilon amino group as well as a peptide bond to a neighboring amino acid in the next polypeptide via its alpha-amino group. Such additional polypeptide may likewise include one or more lysines forming one or more isopeptide bonds to one or more further polypeptide, and so forth. Successive polynucleotides with successive lysine branch points may further be included. In this example, a scaffold includes polypeptides attached to each other in a branched formation, connected at lysine residues. The first polypeptide sequence may include or be attached to a single template polynucleotide bonding site, and last polypeptides (e.g., the next, additional, further, or subsequent polypeptide which includes no lysine forming an isopeptide bond to a successive polypeptide) may include accessory bonding sites (such as, for example, on the terminal amino group of the N-terminal amino acid, an epsilon amino acid of a lysine of the last polypeptides, or both) of the last polypeptides.

A template polynucleotide for attachment to a scaffold may be of any suitable length, including for sequencing in an SBS process. For example, a template polynucleotide may be about 50 nucleotides in length, about 75 nucleotides in length, about 100 nucleotides in length, about 125 nucleotides in length, about 150 nucleotides in length, about 175 nucleotides in length, about 200 nucleotides in length, about 225 nucleotides in length, about 250 nucleotides in length, about 275 nucleotides in length, about 300 nucleotides in length, about 325 nucleotides in length, about 350 nucleotides in length, about 375 nucleotides in length, about 400 nucleotides in length, about 425 nucleotides in length, about 450 nucleotides in length, about 475 nucleotides in length, about 500 nucleotides in length, about 525 nucleotides in length, about 550 nucleotides in length, about 575 nucleotides in length, about 600 nucleotides in length, about 625 nucleotides in length, about 650 nucleotides in length, about 675 nucleotides in length, about 700 nucleotides in length, about 725 nucleotides in length, about 750 nucleotides in length, about 775 nucleotides in length, about 800 nucleotides in length, about 825 nucleotides in length, about 850 nucleotides in length, about 875 nucleotides in length, about 900 nucleotides in length, about 925 nucleotides in length, about 950 nucleotides in length, about 975 nucleotides in length, about 1,000 nucleotides in length, about 1,100 nucleotides in length, about 1,200 nucleotides in length, about 1,300 nucleotides in length, about 1,40 nucleotides in length, about 1,500 nucleotides in length, about 1,600 nucleotides in length, about 1,700 nucleotides in length, about, 1,800 nucleotides in length, about 1,900 nucleotides in length, about 2,000 nucleotides in length, or longer.

Attachment of a single template polynucleotide or accessory (e.g., accessory oligonucleotide, accessory composition, or accessory structure) to a scaffold may be accomplished by inclusion of moieties or structures on the scaffold and template polynucleotide or accessory that are complementary to each other, meaning they are configured to bind to one another, covalently or non-covalently, to form an attachment therebetween. They may be complementary for covalent binding or complementary for non-covalent binding. A scaffold may include a single template site with a moiety or structure that is complementary to or with a moiety or structure (a single template site) that is attached to a template polynucleotide. The scaffold may also include or be attached to other moieties or structures that are complementary to or with a moiety or structure (accessory sites) attached to an accessory. Cross-reactivity between a moiety or structure attached to a template polynucleotide and a moiety or structure of an accessory site should be avoided, to prevent attachment of more than one template polynucleotide to a scaffold. Cross-reactivity between a moiety or structure attached to an accessory and a moiety or structure of the single template site should also be avoided, to prevent occupation of the single template site by accessories that prevents attachment of a single template polynucleotide thereto. In some examples, such cross-reactivity may be avoided by blocking the single template site or accessory sites chemically while accessories bind to the accessory sites or single template polynucleotides attach to the single template site, respectively, then unblocking the unoccupied site to permit attachment of the single template polynucleotide or accessory thereto.

A non-exclusive list of complementary binding partners is presented in Table 1:

| Bonding site | Example moiety/structure on (a) scaffold-attached bonding site or (b) template polynucleotide or accessory | Example moiety/structure on (a) template polynucleotide or accessory or (b) scaffold-attached bonding site |
|---|---|---|
| amine-NHS | amine group, -NH₂ | N-Hydroxysuccinimide ester |
| | | 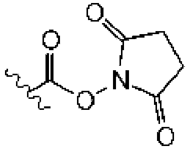 |
| amine-imidoester | amine group, -NH₂ | imidoester |
| | | 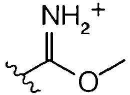 |
| amine-pentofluorophenyl ester | amine group, -NH₂ | pentofluorophenyl ester, |
| | | 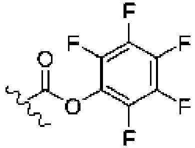 |
| amine-hydroxymethyl phosphine | amine group, -NH₂ | hydroxymethyl phosphine |
| | | 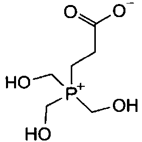 |
| amine-carboxylic acid | amine group, -NH₂ | carboxylic acid group, - C(=O)OH (e.g., following activation of the carboxylic acid by a carbodiimide such as EDC (1-ethyl-3-(-3-dimethylaminopropyl) carbodiimide hydrochloride) or DCC (N', N'-dicyclohexyl carbodiimide) to allow for formation of an amide bond of the activated carboxylic acid with an amine group) |
| thiol-maleimide | thiol, -SH | maleimide |
| | | 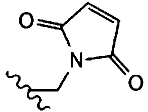 |
| thiol-haloacetyl | thiol, -SH | haloacetyl (e.g., iodoacetyl or other haloacetyl) |
| | | 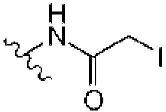 |
| thiol-pyridyl disulfide | thiol, -SH | pyridyl disulfide |
| | | 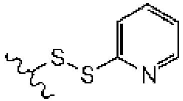 |
| thiol-thiosulfonate | thiol, -SH | thiosulfonate |
| | | 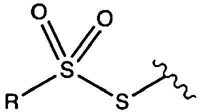 |
| thiol-vinyl sulfone | thiol, -SH | vinyl sulfone |
| | | 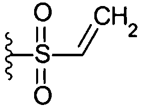 |
| aldehyde-hydrazide | aldehyde, -C(=O)H | hydrazide |
| | | 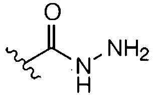 |
| aldehyde-alkoxyamine | aldehyde, -C(=O)H | alkoxyamine |
| | | 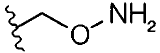 |
| hydroxy-isocyanate | hydroxyl, -OH | isocyanate |
| | | 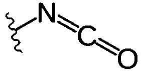 |
| azide-alkyne | azide, -N₃ | alkyne |
| | | 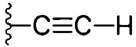 |
| azide-phosphine | azide, -N₃ | phosphine, e.g.: |
| | | 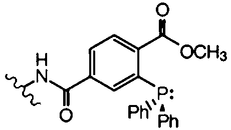 |
| azide-cyclooctyne | azide, -N₃ | cyclooctyne, e.g. dibenzocyclooctyne |
| | | 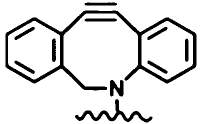 |
| | | or BCN (bicyclo[6.1.0]nonyne) |
| | | 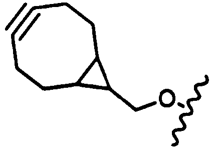 |
| azide-norbornene | azine, -N₃ | norbornene |
| | | 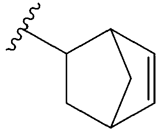 |
| transcyclooctene-tetrazine | transcyclooctene | tetrazine, e.g., benzyl-methyltetrazine: |
| | 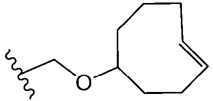 | 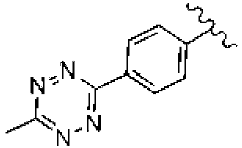 |
| norbornene-tetrazine | norbornene | tetrazine, e.g. benzyl-tetrazine |
| | 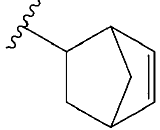 | 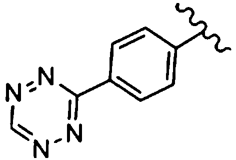 |
| oxime | aldehyde or ketone (e.g., amine group or N-terminus of polypeptide converted to an aldehyde or ketone by pyroxidal phosphate) | alkoxyamine |
| SpyTag-SpyCatcher | SpyTag: amino acid sequence AHIVMVDAYKPTK (SEQ ID NO:1) | SpyCatcher amino acid sequence: |
| | | MKGSSHHHHHHVDIPTT ENLYFQGAMVDTLSGLS SEQGQSGDMTIEEDSATH IKFSKRDEDGKELAGAT MELRDSSGKTISTWISDG QVKDFYLYPGKYTFVET AAPDGYEVATAITFTVNE QGQVTVNGKATK (SEQ ID NO:2) |
| SNAP-tag-O⁶-Benzylguanine | SNAP-tag (O-6-methylguanine-DNA methyltransferase) | O⁶-Benzylguanine |
| | | 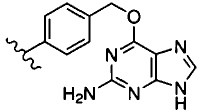 |
| CLIP-tag-O²-benzylcytosine | CLIP-tag (modified O-6-methylguanine-DNA methyltransferase) | O²-benzylcytosine |
| | | 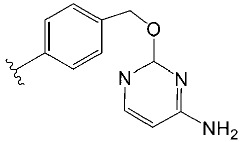 |
| Sortase-coupling | -Leu-Pro-X-Thr-Gly | -Gly₍₃₋₅₎ |

Any of the foregoing can be added to or included in a scaffold as disclosed herein for attachment to a template polynucleotide or accessories such as accessory oligonucleotides, which template polynucleotide or accessory may include or be modified to include a complementary moiety or structure of the foregoing pairs for bonding to the scaffold.

Any suitable bioconjugation methods for adding or forming bonds between such pairs of complementary moieties or structures may be used. Modified nucleotides may be commercially available possessing examples of one or the other of examples of such pairs of complementary moieties or structures, and methods for including one or more of such examples of moieties or structures in or attaching or including them to polymer, a nucleotide, or polynucleotide are also known. Also commercially available may be bifunctional linker molecules with a moiety or structure from one complementary pair of bonding partners listed in Table 1 at one end and a moiety or structure from another complementary pair of bonding partners listed in Table 1. A moiety or structure of a scaffold, template polynucleotide, or of an accessory, or an oligo or polypeptide being attached to any of the foregoing features to as to provide a moiety or structure for bonding between any of such foregoing features, may be bound to one end of such a linker, resulting in the initial moiety or structure being effectively replaced with another, i.e., the moiety or structure present on the other end of the linker.

Modified amino acids may be commercially available possessing examples of one or the other of examples of such pairs of complementary moieties or structures, and methods for including one or more of such examples of moieties or structures in or attaching them to an amino acid or polypeptide are also known. Methods for forming bonds between members of such pairs of complementary moieties or structures are known. Thus, such complementary moieties or structures can be added to or included in a scaffold and a template polynucleotide or a scaffold and an accessory to form bonding sites and permit attachment therebetween.

As used herein, the term "polypeptide" is intended to mean a chain of amino acids bound together by peptide bonds. The terms "protein" and "polypeptide" may be used interchangeably. A polypeptide may include a sequence of a number of amino acids bound to each other by peptide bonds and the number of amino acids may be about 2 or more, about 5 or more, about 10 or more, about 15 or more, about 20 or more, about 25 or more, about 30 or more, about 35 or more, about 40 or more, about 45 or more, about 50 or more, about 55 or more, about 60 or more, about 65 or more, about 70 or more, about 75 or more, about 80 or more, about 85 or more, about 90 or more, about 95 or more, about 100 or more, about 110 or more, about 120 or more, about 130 or more, about 140 or more, about 150 or more, about 160 or more, about 170 or more, about 180 or more, about 190 or more, about 200 or more, about 225 or more, about 250 or more, about 275 or more, about 300 or more, about 325 or more, about 350 or more, about 375 or more, about 400 or more, about 425 or more, about 450 or more, about 475 or more, about 500 or more, about 550 or more, about 600 or more, about 650 or more, about 700 or more, about 750 or more, about 800 or more, about 850 or more, about 900 or more, about 950 or more, about 1000, or higher.

In some cases, a polypeptide, or protein, may adopt a structure or three-dimensional conformation to promote or permit bonding to another bonding partner such as another polypeptide that also adopts a three-dimensional structure conducive to such bonding, or other, non-protein bonding partners. A polypeptide may also adopt a three-dimensional conformation conducive to performing enzymatic reactions on other substrate polypeptides or other molecules, or so as to serve as a substrate for another enzymatic or other reaction. A polypeptide may also adopt a three-dimensional conformation such that a site or sites, such as an amino terminal, a carboxyl terminal, a side group of an amino acid, or a modification to an amino acid, may be accessible for bonding with another molecule.

Various bioconjugation chemistries can be used for attaching a template polynucleotide to a scaffold. A chemical moiety may be included in or added to such a site having an ability to form a covalent conjugation to a complementary chemical moiety, which complementary moiety may be attached to or included in a template polynucleotide. A template polynucleotide may then be conjugated to the scaffold, such as through covalent attachment between the complementary chemical moieties.

In another example, a scaffold may include or be attached to, as a single template site, a polypeptide sequence capable of forming a covalent attachment to another polypeptide sequence or other chemical moiety. Such other polypeptide or other chemical moiety may then be included in or attached to a template polynucleotide, such that the single template site of the scaffold and the template polynucleotide may covalently bond to each other. Alternatively, the template polynucleotide may have the first such polypeptide sequence, and the single template site of the scaffold may have such other polypeptide sequence or other chemical moiety capable of covalently bonding to the polypeptide sequence of the template polynucleotide. Non-limiting examples of such pairs include the SpyTag/SpyCatcher system, the Snap-tag/ O⁶-Benzylguanine system, and the CLIP-tag/O²-benzylcytosine system.

Amino acid sequences for the complementary pairs of the SpyTag/SpyCatcher system and polynucleotides encoding them may be available. Examples of sequences are provided in Table 1. Several amino acid site mutations for a SpyTag sequence and for a SpyCatcher sequence may be available for inclusion in recombinant polypeptides. A Snap-tag is a functional O-6-methylguanine-DNA methyltransferase, and a CLIP-tag is a modified version of Snap-tag. Nucleotide sequences encoding Snap-tag, CLIP-tag, SpyCatcher, may be commercially available for subcloning and inclusion in engineered polypeptide sequences.

Alternatively, complementary pairs for covalent attachment on a single template site of a scaffold and a template polynucleotide may be covalently attached to each other via an enzymatically catalyzed formation of a covalent bond. For example, a single template site of a scaffold and a template polynucleotide may include motifs capable of covalent attachment to each other by sortase-mediated coupling, e.g. a LPXTG amino acid sequence on one and an oligoglycine nucleophilic sequence on the other (with a repeat of, e.g., from 3 to 5 glycines). Sortase-mediated transpeptidation may then be carried out to result in covalent attachment of the scaffold and template polynucleotide at the single template site.

In another example, a scaffold may include a region for non-covalent attachment of a single template polynucleotide at a single template site. For example, a scaffold may include an oligonucleotide for hybridizing to an end of a template polynucleotide by Watson-Crick base pairing. In another example, a scaffold and template polynucleotide may include or be attached to complementary peptide binding sites. For example, the scaffold and template polynucleotide may include or be attached to peptide sequences that may bind to each other as complementary pairs of a coiled coil motif. A coiled coil motif is a structural feature of some polypeptides where two or more polypeptide strands each form an alpha-helix secondary structure and the alpha-helices coil together to form a tight non-covalent bond. A coiled coil sequence may include a heptad repeat, a repeating pattern of the seven amino acids HPPHCPC (where H indicates a hydrophobic amino acid, C typically represents a charged amino acid and P represents a polar, hydrophilic amino acid). An example of a heptad repeat is found in a leucine zipper coiled coil, in which the fourth amino acid of the heptad is frequently leucine.

A scaffold may include or be attached to one amino acid sequence that forms part of a coiled coil bonding pair and a template polynucleotide may be attached to another amino acid sequence that forms another part of a coiled coil bonding pair, complementary to that which is or is attached to the scaffold, such that the two attach to each other. For example, a scaffold may be covalently attached to one amino acid sequence that forms part of a coiled coil bonding pair and a template polynucleotide may be attached to another amino acid sequence that forms another part of a coiled coil bonding pair, complementary to that which is or is attached to the scaffold, such that the two attach to each other.

In another example, the scaffold and the template polynucleotide may each include or be attached to other complementary partners of peptide pairs that bind together non-covalently. An example includes a biotin-avidin binding pair. Biotin and avidin peptides (such as avidin, streptavidin, and neutravidin, all of which are referred to collectively as "avidin" herein unless specifically stated otherwise) form strong noncovalent bonds to each other. One part of such pair, whether binding portion of biotin or of avidin, may be part of or attached to either the scaffold or template polynucleotide, with the complementary part correspondingly part of or attached to the scaffold or template polynucleotide, permitting non-covalent attachment therebetween.

Numerous methods are available for including one or more biotin moiety in or adding one or more biotin moiety to a DNA molecule, template polynucleotide, scaffold, oligo-DNA, other polypeptide, or other composition for bonding molecules together as disclosed herein (such as template polynucleotides to a scaffold, or accessories to a scaffold). For example, biotinylated nucleotides are commercially available for incorporation into a DNA molecule by a polymerase, and kits are commercially available for adding a biotin moiety to a polynucleotide or a polypeptide. Biotin residues can also be added to amino acids or modified amino acids or nucleotides or modified nucleotides. Linking chemistries shown in Table 1 can also be used for adding a biotin group to proteins such as on carboxylic acid groups, amine groups, or thiol groups. Several biotin ligase enzymes are also available for enzymatically targeted biotinylation such as of polypeptides (e.g., of the lysine reside of the AviTag amino acid sequence GLNDIFEAQKIEWHE (SEQ ID NO:3) included in a polypeptide). A genetically engineered ascorbate peroxidase (APEX) is also available for modifying biotin to permit biotinylation of electron-rich amino acids such as tyrosine, and possibly tryptophan, cysteine, or histidine.

In another example, a polypeptide including the amino acid sequence DSLEFIASKLA (SEQ ID NO:4) may be biotinylated (at the more N-terminal of the two S residues present in the sequence), which is a substrate for Sfp phosphopantetheinyl transferase-catalyzed covalent attachment thereto with small molecules conjugated to coenzyme A (CoA). For example, a polypeptide including this sequence could be biotinylated through covalent attachment thereto by a CoA-biotin conjugate. This system may also be used for attaching many other types of bonding moieties or structures identified in Table 1 for use in creating bonding sites for a scaffold to bond to a DNA molecule or polypeptide or other molecule as disclosed herein. For example, a CoA conjugated to any of the reactive pair moieties identified in Table 1 could be covalently attached to a polypeptide containing the above-identified sequence by Sfp phosphopantetheinyl transferase, thereby permitting bonding of another composition thereto that includes the complementary bonding partner.

Other enzymes may be used for adding bonding moiety to a polypeptide. For example, a lipoic acid ligase enzyme can add a lipoic acid molecule, or a modified lipoic acid molecule including a bonding moiety identified in Table 1 such as an alkyne or azide group, can be covalently linked to the amine of a side group of a lysine reside in an amino acid sequence DEVLVEIETDKAVLEVPGGEEE (SEQ ID NO:5) or GFEIDKVWYDLDA (SEQ ID NO:6) included in a polypeptide. In another example, a scaffold, template polynucleotide, or other polypeptide or DNA molecule included therein or intended to be bonded thereto may include or be attached to an active serine hydrolase enzyme. Fluorophosphonate molecules become covalently linked to serine residues in the active site of serine hydrolase enzymes. Commercially available analogs of fluorophosphonate molecules including bonding moieties identified in Table 1, such as an azide group or a desthiobiotin group (an analog of biotin that can bind to avidin). Thus, such groups can be covalently attached to serine hydrolase enzyme included in or attached to a polypeptide or DNA molecule used in or attached to a scaffold as disclosed herein and such bonding moiety or structure can be covalently added thereto by use by attachment of a suitable modified fluorophosphonate molecule for creating a bonding site on such protein for a complementary bonding partner from Table 1 (such as for azide-alkyne, azide-phosphine, azide-cyclooctyne, azide-norbornene, or desthiobiotin-avidin bonding).

Any of the foregoing methods of biotinylating compositions to promote bonding to a polypeptide including an avidin sequence (such as an avidin polypeptide included in or attached to another composition), or otherwise adding functional groups to polypeptides, as part of a scaffold, attached to a scaffold, part of an accessory, or attached to an accessory or template polynucleotide, for bonding between a scaffold and a template polynucleotide or between a scaffold and an accessory, may be used for permitting or promoting bonding between such components as disclosed herein.

For attachment to a single template site of a scaffold, a template polynucleotide may have a complementary attachment moiety or structure added thereto. In an example, during preparation of a library sample, a plurality of template polynucleotides may be prepared for sequencing. Commonly during such sample preparation, template polynucleotides of the library sample are modified to include particular nucleotide sequences in addition to the sequences already included therein as part of the library to be sequenced. Such added nucleotide sequences may serve any of various functions, including for subsequent identification of the template polynucleotide or attachment to a surface of an SBS substrate as part of a seeding process. In accordance with the present disclosure, such preparation of template polynucleotides may also include a complementary attachment moiety or structure being attached thereto or included therein.

For example, preparation of a template polynucleotide may include attachment of a nucleotide sequence in the template polynucleotide, such as extending from one of its ends, and the sequence is complementary to another sequence which other sequence is included in or attached to the single template site of the scaffold. Hybridization due to Watson-Crick base pairing results in bonding between the two. In another example, an accessory, such as an accessory oligonucleotide, may be modified to permit covalent attachment to it of a moiety or structure that is complementary thereto. For example, modifications to a nucleotide included in an accessory such as an accessory oligonucleotide, such as on a phosphate group, the base, or the sugar, may be included to provide a site for covalent attachment to accessory sites of a scaffold. Accessory sites of the scaffold may in turn include a complementary moiety or structure permitting attachment to accessories such as oligo-DNA accessories. In an example, nucleotides modified to include an attachment moiety with which a complementary moiety of an accessory bonding site of a scaffold, included in a polynucleotide sequence added to a template polynucleotide during sample preparation. Numerous modified nucleotides bearing such chemical moieties are commercially available for covalent attachment of compositions to DNA molecules in which such modified nucleotides have been incorporated.

In another example, a template polynucleotide may be modified, such as during sample preparation, by attaching to it a polypeptide. Such polypeptide may possess an amino acid sequence and/or structure so as to be complementary to an amino acid structure of a single template site of a scaffold, such that the template polynucleotide may attach, via its attached polypeptide, to the single template site of the scaffold. Examples of pairs of polypeptides for covalent or noncovalent bonding between a single template site of a scaffold and a template polynucleotide were provided above and include, as non-limiting examples, alpha-helical amino acid sequences with heptad repeats for formation of coiled coil attachments to one another, biotin-avidin binding pairs, SpyTag/SpyCatcher system, LPXTG/oligoglycine nucleophilic pairs for sortase-mediated transpeptidation bonding. In another example, a template polynucleotide may be modified during sample preparation to include one of a Snap-tag sequence or O⁶-Benzylguanine, and a single template site of a scaffold may include the other of the two, to permit covalent bonding between the two in accordance with the Snap-tag/O⁶-Benzylguanine system. In another example, a template polynucleotide may be modified during sample preparation to include one of a CLIP-tag sequence or O²-benzylcytosine, and a single template site of a scaffold may include the other of the two, to permit covalent bonding between the two in accordance with the CLIP-tag/O²-benzylcytosine system. , and the CLIP-tag/O²-benzylcytosine system.

Any of the foregoing examples may likewise be used for attachment of one or more accessories to one or more accessory sites on a scaffold. For attachment to an accessory site of a DNA scaffold or of a polypeptide scaffold, an accessory (such as an accessory oligo-DNA) may have a complementary attachment moiety or structure added thereto. In an example, a nucleotide sequence may be included in or attached to an accessory and may include a complementary attachment moiety or structure being attached thereto or included therein.

In another example, an accessory (such as an accessory oligo-DNA) may include or be attached to a nucleotide sequence, such as extending from one of its ends in the case of an accessory oligo-DNA, and the sequence is complementary to another sequence which other sequence is included in or attached to accessory sites of the scaffold. Watson-Crick base-pairing between the complementary sequences results in hybridization and bonding between the two and, thus, attachment of accessories to accessory bonding sites. In another example, the accessory may include covalent modification thereof to permit covalent attachment to it of a moiety or structure that is complementary thereto. For example, modifications to a nucleotide included in a template polynucleotide, such as on a phosphate group, the base, or the sugar, may be included to provide a site for covalent attachment to an accessory site of a scaffold. Accessory sites of the scaffold may in turn include a complementary moiety or structure permitting attachment to accessories such as oligo-DNA accessories. In an example, nucleotides modified to include an attachment moiety with which a complementary moiety of an accessory bonding site of a scaffold may be included in a polynucleotide sequence added to or included in an accessory such as an accessory oligo-DNA to permit bonding between them. Numerous modified nucleotides bearing such chemical moieties are commercially available for covalent attachment of compositions to DNA molecules in which such modified nucleotides have been incorporated.

In another example, an accessory may be modified by attaching to it a polypeptide. Such polypeptide may possess an amino acid sequence and structure so as to be complementary to an amino acid structure of an accessory site of a scaffold, such that the accessories may attach, via their attached polypeptides, to the accessory sites of the scaffold. Examples of pairs of polypeptides for covalent or noncovalent bonding between accessory sites and accessories were provided above and include, as non-limiting examples, alpha-helical amino acid sequences with heptad repeats for formation of coiled coil attachments to one another, biotin-avidin binding pairs, SpyTag/SpyCatcher system, LPXTG/oligoglycine nucleophilic pairs for sortase-mediated transpeptidation bonding. In another example, an accessory, such as an accessory oligo-DNA, may be modified to include one of a Snap-tag sequence or O⁶-Benzylguanine, and accessory sites of a scaffold may include the other of the two, to permit covalent bonding between the two in accordance with the Snap-tag/O⁶-Benzylguanine system. In another example, an accessory may be include one of a CLIP-tag sequence or O²-benzylcytosine, and accessory sites of a scaffold may include the other of the two, to permit covalent bonding between the two in accordance with the CLIP-tag/O²-benzylcytosine system.

In an example, a single template polynucleotide may be bound to a single template site of a scaffold, and multiple accessory nucleotides, such as accessory oligo-DNA molecules, may be bound to accessory sites of a scaffold. Examples of such oligo-DNA molecules may be primers for performing clustering on the scaffold. As part of a conventional clustering process, copies of a template polynucleotide or its complement are made on a surface of a substrate. As explained above, in some instances such on-surface clustering may unfavorably result in formation of one or more polyclonal clusters. As disclosed herein, clustering may be performed on a scaffold, such as in solution, without prior attachment of the scaffold to a surface. In other examples, a scaffold with a single template polynucleotide attached may be attached to a surface of a substrate and clustering may then be performed on the surface of the substrate, on the scaffold, or on the scaffold and on the surface of the substrate.

For a clustering procedure, a modification may be made to a template polynucleotide such as during sample preparation to include one or more nucleotide sequences at one or both of its 3-prime and 5-prime ends. A copy or copies of the template nucleotide and nucleotide sequences complementary to the template nucleotide may then be synthesized on, as disclosed herein, a scaffold, forming a cluster. Such on-scaffold clustering may result in formation of a monoclonal cluster.

For example, a template polynucleotide may bond to a single template attachment site with its 5-prime end oriented towards the scaffold and its 3-prime end oriented away from the site of bonding to the scaffold. The 3-prime end may include a nucleotide sequence that is complementary to a nucleotide sequence included in a first primer. A "primer" is defined as a single stranded nucleic acid sequence (e.g., single strand DNA or single strand RNA) that serves as a starting point for DNA or RNA synthesis. A primer can be any number of bases long and can include a variety of non-natural nucleotides. In an example, the primer is a short strand, ranging from 20 to 40 bases, or 10 to 20 bases. Copies of primers complementary to the 3-prime end of the template polynucleotide may further be attached to accessory sites of the scaffold.

A polymerization reaction may then be performed, in which the 3-prime end of the template polynucleotide hybridizes via Watson-Crick base pairing to a scaffold-bound first primer complementary thereto. A polymerase in the polymerization reaction may create a nascent strand complement to the template polynucleotide as attached to the scaffold, initiated from the scaffold-attached primer to which the 3-prime end of the template polynucleotide is hybridized. The template polynucleotide and its complement may then be dehybridized.

The complement to the template polynucleotide, at the 3-prime end of the complement, may include a nucleotide sequence that is complementary to a second primer sequence. Copies of second primers complementary to the 3-prime end of the complement to the template polynucleotide may further be attached to accessory sites of the scaffold. A second polymerization reaction may then be performed, in which the 3-prime end of the template polynucleotide hybridizes via Watson-Crick base pairing to a scaffold-bound first primer complementary thereto and the 3-prime end of the complement to the template polynucleotide hybridizes via Watson-Crick base pairing to a scaffold-bound second primer complementary thereto. A polymerase in the second polymerization reaction may create another nascent strand complement to the template polynucleotide as attached to the scaffold, initiated from the scaffold-attached first primer to which the 3-prime end of the template polynucleotide is hybridized. And the polymerase in the second polymerization reaction may further create a nascent strand copy of the template polynucleotide as attached to the scaffold, initiated from the scaffold-attached second primer to which the 3-prime end of the complement to the template polymerized in the prior polymerization reaction is hybridized. The template polynucleotide and copy thereof and its complements may then be dehybridized.

Subsequent polymerization reactions may then be performed in an iterative process. 3-prime ends of scaffold-bound template polynucleotide and copies thereof hybridize to scaffold-bound first primers complementary thereto, and 3-prime ends of scaffold-bound complements to the template polynucleotide hybridize to scaffold-bound second primers complementary thereto. Nascent strands are polymerized by a polymerase, initiated at the scaffold-bound first and second primers to which the scaffold bound template polynucleotide and complements to and copies thereof are hybridized. Following dehybridization of the strands following polymerization, successive polymerization reactions are performed, thereby multiplying the number of copies of template polynucleotide and complements thereto attached to the scaffold. In this manner, copies of and complements to the template polynucleotide are amplified, with the amplified copies bound to the scaffold, forming a cluster. As disclosed herein, this clustering process may be performed on a scaffold, such as in solution, as opposed to conventional clustering which is performed on a surface of a substrate in a conventional SBS process. Because there are copies of and complements to only a single template polynucleotide clustered on the scaffold, a monoclonal cluster is present on the scaffold.

In such an example, where a sequence at or attached to the 5-prime end of a template polynucleotide bonds to a single template site, orienting the 3-prime end of the template polynucleotide away from the scaffold, the template polynucleotide may bond to the single template site of the scaffold by hybridization to a primer sequence attached to or part of the single template site, referred to as a template site primer. In an example, a template polynucleotide, as prepared by a sample preparation process, may have at or attached to its 5-prime end a nucleotide sequence complementary to the template site primer. 3-prime to such nucleotide sequence complementary to the template site primer, the template polynucleotide may include a nucleotide sequence that corresponds to the nucleotide sequence of the above-described second primer (the second primer being a scaffold-attached primer to which a 3-prime end of a complement to the template polynucleotide may hybridize by complementary Watson-Crick base pairing). Inclusion of such sequence in the template polynucleotide means that a complement to the template polynucleotide, synthesized during a polymerization step, would have, towards its 3-prime end, a polynucleotide sequence that is complementary to the sequence of such second primer. Having such sequence towards the 3-prime end of a complement to a template polynucleotide enables hybridization of the 3-prime end of the complement to such second primer during a subsequent polymerization reaction during clustering.

At the 3-prime end of the template polynucleotide, oriented away from the template polynucleotide's 5-prime end bound to the single template site, the template polynucleotide may include a sequence complementary to the first primer as described above. During a first polymerization step, as described above, such nucleotide sequence at the template polynucleotide's 3-prime end may hybridize to a first primer, followed by polymerization of a nascent complement to the template polynucleotide. It may be advantageous for there to be a discontinuation of polymerization of a complement to the template polynucleotide between the portion of the template polynucleotide hybridized to the template site primer and a nucleotide sequence located 3-prime thereto in the template polynucleotide that includes the sequence of the second primer. That is, it may be advantageous for the complement of the template polynucleotide to have at its 3-prime end a sequence complementary to the second primer. However, if there is no discontinuation of polymerization after adding to the nascent complement to the template polynucleotide a nucleotide sequence complementary to the sequence corresponding to the second primer, the 3-prime end of the complement to the template polynucleotide would not end there.

For example, if a nucleotide sequence complementary to the template site primer is 5-prime to and contiguous with the sequence complementary to the second primer, the 3-prime end of the synthesized complement to the template polynucleotide may include a nucleotide sequence included in the template site primer. For example, a DNA polymerase, in polymerizing the complement to the template polynucleotide, may displace the template site primer from hybridization to the 5-prime end of the template polynucleotide and polymerize the addition of a nucleotide sequence corresponding thereto to the 3-prime end of the complement to the template polynucleotide. Such an outcome may be unwanted if it impaired an ability of the 3-prime end of the complement to the template polynucleotide from hybridizing to an above-described second primer at an accessory site.

In an example it may therefore be desirable to incorporate a discontinuation of polymerization 3-prime to the 5-prime end of the template polynucleotide where such 5-prime end of the template polynucleotide bonds to the single template site by hybridizing to a template site primer. For example, a linker, such as a PEG linker, alkyl linker, or other chemical moiety may be included to connect the nucleotide sequence that hybridizes to the template site primer to the 5-prime end of the template polynucleotide. The presence of such a linker, rather than a contiguous nucleotide sequence connection, would prevent a polymerase from adding a nucleotide sequence corresponding to the template site primer to the 3-prime end of the complement of the template polynucleotide, which would instead end with a nucleotide sequence complementary to the nucleotide sequence of the second primer as may be desired.

In other examples, a template polynucleotide may have or be attached to a polynucleotide sequence at the template polynucleotide's 3-prime end that is complementary to a primer that is part of or attached to a single template site of a scaffold, referred to as a template site primer. Following hybridization of such sequence of or attached to the 3-prime end of the template polynucleotide to template site primer, a polymerization process may be performed wherein a DNA polymerase polymerizes formation of a nascent polynucleotide complementary to the template polynucleotide, initiated from the template site primer. Dehybridization of the template polynucleotide from the scaffold-attached complement to the template polynucleotide is then performed. The 3-prime end of the scaffold-attached complement to the template polynucleotide, oriented away from the site of attachment to the scaffold, may include a nucleotide sequence that is complementary to the above-described second primer sequence (the second primer being a scaffold-attached primer to which a 3-prime end of a complement to the template polynucleotide may hybridize by complementary Watson-Crick base pairing). Copies of second primers complementary to the 3-prime end of the complement to the template polynucleotide may further be attached to accessory sites of the scaffold. A second polymerization reaction may then be performed, in which the 3-prime end of the complement to the template polynucleotide hybridizes via Watson-Crick base pairing to a scaffold-bound second primer complementary thereto. A polymerase in the second polymerization reaction may create a nascent strand copy of the template polynucleotide (i.e., a complement to the scaffold-bound complement to the template polynucleotide), initiated from the scaffold-attached second primer to which the 3-prime end of the complement to the template polymerized in the prior polymerization reaction is hybridized. A dehybridization step may then be performed to dehybridize the scaffold bound complement to the template polynucleotide and copy of the template polynucleotide from each other.

The copy of the template polynucleotide, at the 3-prime end of the copy, may include a nucleotide sequence that is complementary to the above-described first primer sequence. Copies of first primers complementary to the 3-prime end of the copy of the template polynucleotide, described above, may further be attached to accessory sites of the scaffold. A third polymerization reaction may then be performed, in which the 3-prime end of the copy of the template polynucleotide hybridizes via Watson-Crick base pairing to a scaffold-bound first primer complementary thereto and the 3-prime end of the complement to the template polynucleotide hybridizes via Watson-Crick base pairing to a scaffold-bound second primer complementary thereto. A polymerase in the third polymerization reaction may create another nascent strand complement to the template polynucleotide as attached to the scaffold, initiated from the scaffold-attached first primer to which the 3-prime end of the copy of the template polynucleotide is hybridized. And the polymerase in the third polymerization reaction may further create a nascent strand copy of the template polynucleotide, initiated from the scaffold-attached second primer to which the 3-prime end of the complement to the template polymerized in the prior polymerization reaction is hybridized. A dehybridization step dehybridizing the copies of and complements to the template polynucleotide from each other may then be performed.

Subsequent polymerization reactions may then be performed in an iterative process. 3-prime ends of scaffold-bound copies of template polynucleotide hybridize to scaffold-bound first primers complementary thereto, and 3-prime ends of scaffold-bound complements to the template polynucleotide hybridize to scaffold-bound second primers complementary thereto. Nascent strands are polymerized by a polymerase, initiated at the scaffold-bound first and second primers to which the scaffold bound template polynucleotide and complements to and copies thereof are hybridized. Dehybridization of the strands is performed following polymerization, then successive polymerization reactions are performed followed by further dehybridization. In this manner, copies of and complements to the template polynucleotide are amplified, with the amplified copies and complements bound to the scaffold, forming a cluster. As disclosed herein, this clustering process may be performed on a scaffold, such as in solution, as opposed to conventional clustering which is performed on a surface of a substrate in a conventional SBS process. Because there are copies of and complements to only a single template polynucleotide clustered on the scaffold, a monoclonal cluster is present on the scaffold.

In an example, an end of a template polynucleotide includes or is attached to a nucleotide sequence that is complementary to a nucleotide sequence included in or attached to the single template site of the scaffold, referred to as the third template-site primer. In an example, a complement to the template polynucleotide may be synthesized on the scaffold initiated at the third template site primer.

In examples of on-scaffold clustering as disclosed herein, a template polynucleotide may be bound to a single template site of a scaffold according to any of various covalent or non-covalent bonds disclosed herein. For example, either end of a template polynucleotide may include a moiety or structure from a bonding site pair such as identified in Table 1, and the complementary moiety or structure of the same pair may be present at the single template site of the scaffold. Successive rounds of polymerization may then follow much as described above. For example, a 3-prime end of a template polynucleotide bound to the scaffold's single template site at or towards the template polynucleotide's 5-prime end may hybridize to an oligonucleotide primer bound to an accessory site of scaffold and a complement thereto synthesized by a DNA polymerase. Successive rounds of polymerization may then follow as described above, resulting in polymerization of multiple copies of the template polynucleotide and complements thereto emanating from accessory sites of the scaffold. Because only a single template polynucleotide was bound to the scaffold, the scaffold having only a single template polynucleotide site, such copies would constitute a monoclonal cluster on the scaffold.

In another example, a scaffold may attach to a surface of a substrate, such as a surface of a substrate for use in an SBS procedure. For example, accessory sites of a scaffold may include or be or become attached to sites attached to a surface of a substrate, or compositions that bond to a surface of a substrate. In an example, a surface of a substrate may be bound to primers, such as for example copies of primers that are complementary to first primers or second primers as described above, or both, as non-limiting examples. Such complementary primers may be attached either directly to a surface of a substrate or may be attached to a modified surface, such as a surface to which polymer molecules have been attached (e.g., PAZAM or related polymers) with primers attached to such polymers. Aforementioned first primers and second primers may be attached to accessory sites of a scaffold (directly, or via a polymer such as PAZAM or other PAZAM-like polymers as disclosed above as non-limiting examples, or spacer or other composition). Such first and second primers of or attached to a scaffold may hybridize to primers complementary thereto as attached to a surface of a substrate, thereby bonding a scaffold to the surface of the substrate.

Examples of first and second primers as discussed above may include primers used in existing SBS processes. Specific examples of suitable primers include P5 and/or P7 primers, which are used on the surface of commercial flow cells sold by Illumina, Inc., for sequencing on HISEQ^{™}, HISEQX^{™}, MISEQ^{™}, MISEQDX^{™}, MINISEQ^{™}, NEXTSEQ^{™}, NEXTSEQDX^{™}, NOVASEQ^{™}, GENOME ANALYZER^{™}, ISEQ^{™}, and other instrument platforms. And portion of a template polynucleotide that includes a nucleotide sequence corresponding to, or complementary to, a first or second primer as disclosed above may have, for example, a sequence corresponding to or complementary to a P5 primer (including a nucleotide sequence of AATGATACGGCGACCACCGAGATCTACAC (SEQ ID NO:7)), a P7 primer (including a nucleotide sequence of CAAGCAGAAGACGGCATACGAGAT (SEQ ID NO:8)), or both, in accordance with such primer sequences as used in the above-mentioned SBS platforms, or others.

A substrate for an SBS process may include, as non-limiting examples, substrates used in any of the aforementioned SBS platforms or others. As a non-limiting example, such a substrate may be a flow cell. As used herein, the term "flow cell" is intended to mean a vessel having a chamber (i.e., flow channel) where a reaction can be carried out, an inlet for delivering reagent(s) to the chamber, and an outlet for removing reagent(s) from the chamber. In some examples, the chamber enables the detection of a reaction or signal that occurs in the chamber. For example, the chamber can include one or more transparent surfaces allowing for the optical detection of arrays, optically labeled molecules, or the like, in the chamber. As used herein, a "flow channel" or "flow channel region" may be an area defined between two bonded components, which can selectively receive a liquid sample. In some examples, the flow channel may be defined between a patterned support and a lid, and thus may be in fluid communication with one or more depressions defined in the patterned support. In other examples, the flow channel may be defined between a non-patterned support and a lid.

As used herein, the term "depression" refers to a discrete concave feature in a patterned support having a surface opening that is completely surrounded by interstitial region(s) of the patterned support surface. Depressions can have any of a variety of shapes at their opening in a surface including, as examples, round, elliptical, square, polygonal, star shaped (with any number of vertices), etc. The cross-section of a depression taken orthogonally with the surface can be curved, square, polygonal, hyperbolic, conical, angular, etc. As an example, the depression can be a well. Also as used herein, a "functionalized depression" refers to the discrete concave feature where primers are attached, in some examples being attached to the surface of the depression by a polymer (such as a PAZAM or similar polymer).

The term flow cell "support" or "substrate" refers to a support or substrate upon which surface chemistry may be added. The term "patterned substrate" refers to a support in which or on which depressions are defined. The term "non-patterned substrate" refers to a substantially planar support. The substrate may also be referred to herein as a "support," "patterned support," or "non-patterned support." The support may be a wafer, a panel, a rectangular sheet, a die, or any other suitable configuration. The support is generally rigid and is insoluble in an aqueous liquid. The support may be inert to a chemistry that is used to modify the depressions. For example, a support can be inert to chemistry used to form a polymer coating layer, to attach primers such as to a polymer coating layer that has been deposited, etc. Examples of suitable supports include epoxy siloxane, glass and modified or functionalized glass, polyhedral oligomeric silsequioxanes (POSS) and derivatives thereof, plastics (including acrylics, polystyrene and copolymers of styrene and other materials, polypropylene, polyethylene, polybutylene, polyurethanes, polytetrafluoroethylene (such as TEFLON^{®} from Chemours), cyclic olefins/cyclo-olefin polymers (COP) (such as ZEONOR^{®} from Zeon), polyimides, etc.), nylon, ceramics/ceramic oxides, silica, fused silica, or silica-based materials, aluminum silicate, silicon and modified silicon (e.g., boron doped p+ silicon), silicon nitride (Si₃N₄), silicon oxide (SiO₂), tantalum pentoxide (TaO₅) or other tantalum oxide(s) (TaOₓ), hafnium oxide (HaO₂), carbon, metals, inorganic glasses, or the like. The support may also be glass or silicon or a silicon-based polymer such as a POSS material, optionally with a coating layer of tantalum oxide or another ceramic oxide at the surface. A POSS material may be that disclosed in Kejagoas et al., Microelectronic Engineering 86 (2009) 776-668, which is incorporated by reference herein in its entirety.

In an example, depressions may be wells such that the patterned substrate includes an array of wells in a surface thereof. The wells may be micro wells or nanowells. The size of each well may be characterized by its volume, well opening area, depth, and/or diameter.

Each well can have any volume that is capable of confining a liquid. The minimum or maximum volume can be selected, for example, to accommodate the throughput (e.g., multiplexity), resolution, analyte composition, or analyte reactivity expected for downstream uses of the flow cell. For example, the volume can be at least about 1×10⁻³ µm³, about 1×10⁻² µm³, about 0.1 µm³, about 1 µm³, about 10 µm³, about 100 µm³, or more. Alternatively or additionally, the volume can be at most about 1×10⁴ µm³, about 1×10³ µm³, about 100 µm³, about 10 µm³, about 1 µm³, about 0.1 µm³, or less.

The area occupied by each well opening on a surface can be selected based upon similar criteria as those set forth above for well volume. For example, the area for each well opening on a surface can be at least about 1×10⁻³ µm², about 1×10⁻² µm², about 0.1 µm², about 1 µm², about 10 µm², about 100 µm², or more. Alternatively or additionally, the area can be at most about 1×10³ µm², about 100 µm², about 10 µm², about 1 µm², about 0.1 µm², about 1×10⁻² µm², or less. The area occupied by each well opening can be greater than, less than or between the values specified above.

The depth of each well can be at least about 0.1 µm, about 1 µm, about 10 µm, about 100 µm, or more. Alternatively or additionally, the depth can be at most about 1×10³ µm, about 100 µm, about 10 µm, about 1 µm, about 0.1 µm, or less. The depth of each well 14' can be greater than, less than or between the values specified above.

In some instances, the diameter of each well can be at least about 50 nm, about 0.1 µm, about 0.5 µm, about 1 µm, about 10 µm, about 100 µm, or more. Alternatively or additionally, the diameter can be at most about 1×10³ µm, about 100 µm, about 10 µm, about 1 µm, about 0.5 µm, about 0.1 µm, or less (e.g., about 50 nm). The diameter can be about 150 nm, about 200 nm, about 250 nm, about 300 nm, about 350 nm, about 400 nm, about 450 nm, about 500 nm, about 550 nm, about 600 nm, about 650 nm, about 700 nm, about 750 nm, about 800 nm, about 900 nm, about 950 nm, about 1 µm, about 1.25 µm, about 1.5 µm, about 1.74 µm, about 2 µm, about 2.25 µm, about 2.5 µm, about 2.75 µm, about 3 µm, about 3.25 µm, about 3.5 µm, about 3.75 µm, about 4 µm, about 4.25 µm, about 4.5 µm, about 4.75 µm, about 5 µm, about5.25 µm, about 5.5 µm, about 5.75 µm, about 6 µm, about 6.25 µm, about 6.5 µm, about 6.75 µm, about 7 µm, about 7.25 µm, about 7.5 µm, about 7.75 µm, about 8 µm, about 8.25 µm, about 8.5 µm, about 8.75 µm, about 9 µm, about 9.25 µm, about 9.5 µm, or about 9.75 µm. The diameter of each well can be greater than, less than or between the values specified above. A nanowell as the term is used herein is intended to mean a well with a round opening whose largest diameter is about 1 µm or less.

It is to be understood that the ranges provided herein include the stated range and any value or sub-range within the stated range. For example, a range from about 100 nm to about 1 µm (1000 nm), should be interpreted to include not only the explicitly recited limits of from about 100 nm to about 1 µm, but also to include individual values, such as about 708 nm, about 945.5 nm, etc., and sub-ranges, such as from about 425 nm to about 825 nm, from about 550 nm to about 940 nm, etc. Furthermore, when "about" and/or "substantially" are/is utilized to describe a value, they are meant to encompass minor variations (up to +/- 10%) from the stated value.

In an example, a size of a nanoparticle may be such that presence of the nanoparticle in a well such as a nanowell occupies so much of the well's volume that another nanoparticle cannot occupy the well at the same time. Size of a nanoparticle may be designed or determined, in reference to a known size of wells in a surface of a substrate, such that it may enter a well in which no other nanoparticle is present but whose entry into a well would be prevented by presence of another nanoparticle that previously entered and still is present in the well. Nanoparticles sized so as not to be able to fit more than two to a well may promote monoclonality of a cluster within a well. For example, in a conventional SBS process, template polynucleotides may be introduced to a flow cell patterned with wells in a solution in a concentration calibrated to maximize the number of wells in which a template polynucleotide will seed (i.e., bind, such as to a primer attached to the well, directly or via a surface-attached polymer, that is complementary to an nucleotide sequence of part of a template nucleotide), but low enough as to minimize as much as possible the formation of polyclonal clusters.

In an example, a flow cell may include nano-scale regions that are not depressions or nanowells but otherwise spatially isolated regions within which a template polynucleotide or scaffold may bind, or seed, referred to herein as nanopads. In some examples, a flow cell surface includes nanopads, separated from each other by regions of surface where a template polynucleotide or scaffold may not bind. Nanopads may be spaced from one another so as to promote formation of monoclonal clusters. For example, nanopads may be separated from each other such that a cluster formed within one nanopad seeded by a single template polynucleotide would be separated sufficiently from another such nanopad that was seeded by only one template polynucleotide. However, it may be difficult to prevent the seeding of a nanopad by more than one template polynucleotide, resulting in one or more polyclonal clusters forming. In an example as disclosed herein, a nanoparticle may promote formation of monoclonal clusters in favor of polyclonal clusters by preventing more than one template polynucleotide from seeding or attaching within a given nanopad. For example, a size of a nanoparticle may be such that there is insufficient room on a nanopad for more than one nanoparticle to bind, where template polynucleotides bond to a single template polynucleotide sites of scaffolds.

In some instances, a polyclonal cluster may occur if two or more template polynucleotides with nucleotide sequences that differ from each other bind within, or seed, the same well as each other. Molecules may distribute among wells based on their concentration within an applied solution on the basis of a Poisson distribution, according to which there is a balance between minimizing the number of unoccupied wells (for increased efficiency of an SBS run) while minimizing a number of wells occupied by multiple, disparate template polynucleotides. Disparity between a minimum well size and a size of a template polynucleotide (e.g., a diameter of a B-DNA molecule may be on the order of 2 nm) may result in choosing between a concentration that does not utilize as much substrate surface, such as surface within wells, as available or preferred on the one hand and resulting in formation of an undesirable or undesirably high number of polyclonal clusters.

As disclosed herein, template polynucleotides may bond to a nanoparticle, with only one template polynucleotide bonding per nanoparticle. A nanoparticle may be sized so as to permit entry of a nanoparticle in a well of a flow cell in which another nanoparticle is non already present, but not to enter a well of a flow cell in which another nanoparticle is already present. Clustering, such as monoclonal clustering, may occur on a nanoparticle before a nanoparticle enters a well, resulting in monoclonal clusters being present in wells. Or, a template polynucleotide may bond to a template site of a nanoparticle and the nanoparticle may enter and bind within a well (for example, by binding of accessory sites to the surface or modification to the surface of a well), thereby seeding the well with only a single nanoparticle, and clustering may then proceed within the well, resulting in monoclonal clusters being present in wells. In some examples, some degree of clustering may occur on nanoparticles before they enter a well and further clustering may occur after the nanoparticle enters a well. All such examples include examples where monoclonal clusters form within wells. Furthermore, tuning a size of nanoparticles so as to reduce, minimize, or in an example eliminate the simultaneous presence of more than one nanoparticle in a well at one time may reduce, minimize, or in an example eliminate formation of polyclonal clusters.

Nanoparticle size may be tuned by modifying a size of a scaffold, modifying a size of accessories bonded to accessory sites such as polymers attached thereto, or both. Size of a nanoparticle may also be modified by an amount of clustering that has or has not occurred on the nanoparticle, such as by modifying a number of sites on a nanoparticle upon which copies of and complements to a template polynucleotide may bind during rounds of polymerization during clustering, with fewer such sites potentially resulting in a lower upper limit of nanoparticle size and more such sites potentially resulting in a larger upper limit of nanoparticle size. A number of rounds of polymerization during clustering may also modify nanoparticle size, with more rounds resulting in more copies of and complements to a template polynucleotide bound to the nanoparticle and therefore potentially increasing its upper size limit and fewer rounds resulting in fewer copies of and complements to a template polynucleotide bound to a nanoparticle and thus potentially reducing its upper size limit. A size of a nanoparticle may be determine according to its size before clustering on a scaffold has occurred or after clustering on a scaffold has occurred.

As used herein the term "nanoparticle" is intended to mean a particle with a largest dimension up to about 1,000 nm in size. Depending on the geometry, the dimension may refer to the length, width, height, diameter, etc. Although "diameter" is generally used to describe the dimension as one example herein, the nanoparticle described herein need not be spherical or circular. A nanoparticle as disclosed herein may have a diameter of about 2 nm, about 5 nm, about 7 nm, about 10 nm, about 12 nm, about 15 nm, about 17 nm, about 20 nm, about 22 nm, about 25 nm, about 27 nm, about 30 nm, about 32 nm, about 35 nm, about 40 nm, about 42 nm, about 45 nm, about 47 nm, about 50 nm, about 52 nm, about 55 nm, about 57 nm, about 60 nm, about 62 nm, about 65 nm, about 67 nm, about 70 nm, about 72 nm, about 75 nm, about 77 nm, about 80 nm, about 82 nm, about 85 nm, about 87 nm, about 90 nm, about 92 nm, about 95 nm, about 97 nm, about 100 nm, about 125 nm, about 150 nm, about 175 nm, about 200 nm, about 225 nm, about 250 nm, about 275 nm, about 300 nm, about 325 nm, about 350 nm, about 375 nm, about 400 nm, about 425 nm, about 450 nm, about 475 nm, about 500 nm, about 525 nm, about 550 nm, about 575 nm, about 600 nm, about 625 nm, about 650 nm, about 675 nm, about 700 nm, about 725 nm, about 750 nm, about 775 nm, about 800 nm, about 825 nm, about 850 nm, about 875 nm, about 900 nm, about 925 nm, about 950 nm, about 975 nm, or about 1,000 nm. Diameter of a nanoparticle is measured by dynamic light scattering (DLS), also known as quasi-elastic light scattering, expressed as twice the hydrodynamic radius (Rh), which may be determined on a DLS system or other system that includes DLS and other functionality (e.g., a ZETASIZER^{®}, Malvern Instruments Limited).

A nanoparticle as disclosed herein may have a diameter within a range of about 2 nm to about 10 nm, about 5 nm to about 15 nm, about 7 nm to about 20 nm, about 10 nm to about 25 nm, about 15 nm to about 30 nm, about 20 nm to about 50 nm, about 40 nm to about 60 nm, about 50 nm to about 75 nm, about 60 nm to about 100 nm, about 70 nm to about 100 nm, about 75 nm to about 100 nm, about 80 nm to about 110 nm, about 90 nm to about 130 nm, about 100 nm to about 150 nm, about 100 nm to about 200 nm, about 150 nm to about 225 nm, about 200 nm to about 250 nm, about 200 nm to about 300 nm, about 225 nm to about 275 nm, about 250 nm to about 300 nm, about 275 nm to about 325 nm, about 300 nm to about 400 nm, about 300 nm to about 350 nm, about 325 nm to about 375 nm, about 350 nm to about 400 nm, about 375 nm to about 425 nm, about 400 nm to about 500 nm, about 400 nm to about 450 nm, about 425 nm to about 475 nm, about 450 nm to about 500 nm, about 475 nm to about 525 nm, about 500 nm to about 600 nm, about 500 nm to about 550 nm, about 525 nm to about 575 nm, about 550 nm to about 600 nm, about 575 nm to about 625 nm, about 600 nm to about 700 nm, about 600 nm to about 625 nm, about 625 nm to about 675 nm, about 650 nm to about 700 nm, about 675 nm to about 725 nm, about 700 nm to about 800 nm, about 700 nm to about 725 nm, about 725 nm to about 775 nm, about 750 nm to about 800 nm, about 775 nm to about 825 nm, about 800 nm to about 900 nm, about 800 nm to about 850 nm, about 825 nm to about 875 nm, about 850 nm to about 900 nm, about 875 nm to about 925 nm, about 900 nm to about 1,000 nm, about 900 nm to about 950 nm, about 925 nm to about 975 nm, about 950 nm to about 1,000 nm, about 300 nm to about 450 nm, about 350 nm to about 500 nm, about 400 nm to about 550 nm, about 450 nm to about 600 nm, about 500 nm to about 650 nm, about 550 nm to about 700 nm, about 600 nm to about 750 nm, about 650 nm to about 800 nm, about 700 nm to about 850 nm, about 750 nm to about 900 nm, about 800 nm to about 950, or about 850 nm to about 1,000 nm.

For convenience and clarity, certain terms employed in the specification, examples, and claims are described herein.

An "acrylate group" includes the salts, esters, and conjugate bases of acrylic acid and its derivatives (e.g., methacrylic acid). The acrylate ion has the molecular formula CH₂=CHCOO⁻.

An "acrylamide monomer" is a monomer with the structure 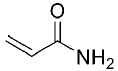 or a substituted analog thereof (e.g., methacrylamide or N-isopropylacrylamide). An example of a monomer including an acrylamide group and an azido group is azido acetamido pentyl acrylamide:

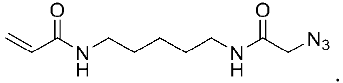

As used herein, "alkyl" refers to a straight or branched hydrocarbon chain that is fully saturated (i.e., contains no double or triple bonds). The alkyl group may have 1 to 20 carbon atoms. Example alkyl groups include methyl, ethyl, propyl, isopropyl, butyl, isobutyl, tertiary butyl, pentyl, hexyl, and the like. As an example, the designation "C1-4 alkyl" indicates that there are one to four carbon atoms in the alkyl chain, i.e., the alkyl chain is selected from the group consisting of methyl, ethyl, propyl, iso-propyl, n-butyl, isobutyl, sec-butyl, and t-butyl.

As used herein, "alkenyl" refers to a straight or branched hydrocarbon chain containing one or more double bonds. The alkenyl group may have 2 to 20 carbon atoms. Example alkenyl groups include ethenyl, propenyl, butenyl, pentenyl, hexenyl, and the like.

As used herein, "alkyne" or "alkynyl" refers to a straight or branched hydrocarbon chain containing one or more triple bonds. The alkynyl group may have 2 to 20 carbon atoms.

Alkoxy or alkoxyl refers to groups of from 1 to 20 carbon atoms - e.g., 1 to 10 carbon atoms, such as 1 to 6 carbon atoms, etc. of a straight or branched configuration attached to the parent structure through an oxygen. Examples include methoxy, ethoxy, propoxy, isopropoxy and the like.

Oxaalkyl refers to alkyl residues in which one or more carbons (and their associated hydrogens) have been replaced by oxygen. Examples include methoxypropoxy, 3,6,9-trioxadecyl and the like. The term oxaalkyl refers to compounds in which the oxygen is bonded via a single bond to its adjacent atoms (forming ether bonds); it does not refer to doubly bonded oxygen, as would be found in carbonyl groups. Similarly, thiaalkyl and azaalkyl refer to alkyl residues in which one or more carbons has been replaced by sulfur or nitrogen, respectively. Examples of azaalkyl include ethylaminoethyl and aminohexyl.

As used herein, "aryl" refers to an aromatic ring or ring system (i.e., two or more fused rings that share two adjacent carbon atoms) containing only carbon in the ring backbone. When the aryl is a ring system, every ring in the system is aromatic. The aryl group may have 6 to 18 carbon atoms. Examples of aryl groups include phenyl, naphthyl, azulenyl, and anthracenyl.

As used herein, the term "attached" refers to the state of two things being joined, fastened, adhered, connected, or bound to each other, either covalently or non-covalently (e.g., by hydrogen bonds, ionic bonds, van der Waals forces, hydrophilic interactions and hydrophobic interactions). For example, a nucleic acid can be attached to a functionalized polymer by a covalent or non-covalent bond.

An "azide" or "azido" functional group refers to -N₃.

As used herein, the "bonding region" refers to an area on a substrate that is to be bonded to another material, which may be, as examples, a spacer layer, a lid, another substrate, etc., or combinations thereof (e.g., a spacer layer and a lid). The bond that is formed at the bonding region may be a chemical bond (as described above), or a mechanical bond (e.g., using a fastener, etc.).

A "tert-butyloxycarbonyl group" (Boc) refers to a 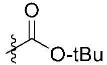 group. A "butyloxycarbonyloxy group" refers to a -OCO₂tBu group.

As used herein, "carbocyclyl" means a non-aromatic cyclic ring or ring system containing only carbon atoms in the ring system backbone. When the carbocyclyl is a ring system, two or more rings may be joined together in a fused, bridged or spiro-connected fashion. Carbocyclyls may have any degree of saturation, provided that at least one ring in a ring system is not aromatic. Thus, carbocyclyls include cycloalkyls, cycloalkenyls, and cycloalkynyls. The carbocyclyl group may have 3 to 20 carbon atoms. Examples of carbocyclyl rings include cyclopropyl, cyclobutyl, cyclopentyl, cyclohexyl, cyclohexenyl, 2,3-dihydro-indene, bicyclo[2.2.2]octanyl, adamantyl, and spiro[ 4.4]nonanyl.

As used herein, the term "carboxylic acid" or "carboxyl" refers to -COOH.

As used herein, "cycloalkylene" means a fully saturated carbocyclyl ring or ring system that is attached to the rest of the molecule via two points of attachment.

As used herein, "cycloalkenyl" or "cycloalkene" means a carbocyclyl ring or ring system having at least one double bond, wherein no ring in the ring system is aromatic. Examples include cyclohexenyl or cyclohexene and norbornenyl or norbornene. Also as used herein, "heterocycloalkenyl" or "heterocycloalkene" means a carbocyclyl ring or ring system with at least one heteroatom in ring backbone, having at least one double bond, wherein no ring in the ring system is aromatic.

As used herein, "hydroxy" or "hydroxyl" refers to an -OH group.

As used herein, the "primer" is defined as a single stranded nucleic acid sequence (e.g., single strand DNA or single strand RNA) that serves as a starting point for DNA or RNA synthesis. The 5' terminus of the primer may be modified to allow a coupling reaction with the functionalized polymer layer. The primer length can be any number of bases long and can include a variety of non-natural nucleotides. In an example, the primer is a short strand, ranging from 20 to 40 bases.

As used herein, the term "optionally substituted" may be used interchangeably with "unsubstituted or substituted". The term "substituted" refers to the replacement of one or more hydrogen atoms in a specified group with a specified radical. For example, substituted alkyl, aryl, cycloalkyl, heterocyclyl etc. refer to alkyl, aryl, cycloalkyl, or heterocyclyl wherein one or more H atoms in each residue are replaced with halogen, haloalkyl, alkyl, acyl, alkoxyalkyl, hydroxyloweralkyl, carbonyl, phenyl, heteroaryl, benzenesulfonyl, hydroxy, loweralkoxy, haloalkoxy, oxaalkyl, carboxy, alkoxycarbonyl [-C(=O)O-alkyl], alkoxycarbonylamino [HNC(=O)O-alkyl], carboxamido [-C(=O)NH₂], alkylaminocarbonyl [-C(=O)NH-alkyl], cyano, acetoxy, nitro, amino, alkylamino, dialkylamino, (alkyl)(aryl)aminoalkyl, alkylaminoalkyl (including cycloalkylaminoalkyl), dialkylaminoalkyl, dialkylaminoalkoxy, heterocyclylalkoxy, mercapto, alkylthio, sulfoxide, sulfone, sulfonylamino, alkylsulfinyl, alkylsulfonyl, alkylsulfonylamino, arylsulfonyl, arylsulfonylamino, acylaminoalkyl, acylaminoalkoxy, acylamino, amidino, aryl, benzyl, heterocyclyl, heterocyclylalkyl, phenoxy, benzyloxy, heteroaryloxy, hydroxyimino, alkoxyimino, oxaalkyl, aminosulfonyl, trityl, amidino, guanidino, ureido, benzyloxyphenyl, and benzyloxy. "Oxo" is also included among the substituents referred to in "optionally substituted"; it will be appreciated by persons of skill in the art that, because oxo is a divalent radical, there are circumstances in which it will not be appropriate as a substituent (e.g. on phenyl). In one example, 1, 2, or 3 hydrogen atoms may be replaced with a specified radical. In the case of alkyl and cycloalkyl, more than three hydrogen atoms can be replaced by fluorine; indeed, all available hydrogen atoms could be replaced by fluorine. Such compounds (e.g., perfluoroalkyl) fall within the class of "fluorohydrocarbons". To be clear, a generic term may encompass more than one substituent, that is, for example, "haloalkyl" or "halophenyl" refers to an alkyl or phenyl in which at least one, but perhaps more than one, hydrogen is replaced by halogen. In some examples, substituents are halogen, haloalkyl, alkyl, acyl, hydroxyalkyl, hydroxy, alkoxy, haloalkoxy, oxaalkyl, carboxy, cyano, acetoxy, nitro, amino, alkylamino, dialkylamino, alkylthio, alkylsulfinyl, alkylsulfonyl, alkylsulfonylamino arylsulfonyl, arylsulfonylamino and benzyloxy.

In describing compounds herein, the terminology "substituted with at least one oxygenated substituent" is used. An oxygenated substituent is a substituent that contains oxygen in addition to carbon and hydrogen; an oxygenated substituent may also include additional heteroatoms, such as nitrogen (for example, a carboxamide or methanesulfonyl). Typical examples of oxygenated substituents include alkoxy, hydroxy, fluoroalkoxy, formyl, acetyl and other C₁ to C₆ acyl chains.

### NON-LIMITING EXAMPLES

The following examples are intended to illustrate particular embodiments of the present disclosure, but are by no means intended to limit the scope thereof.

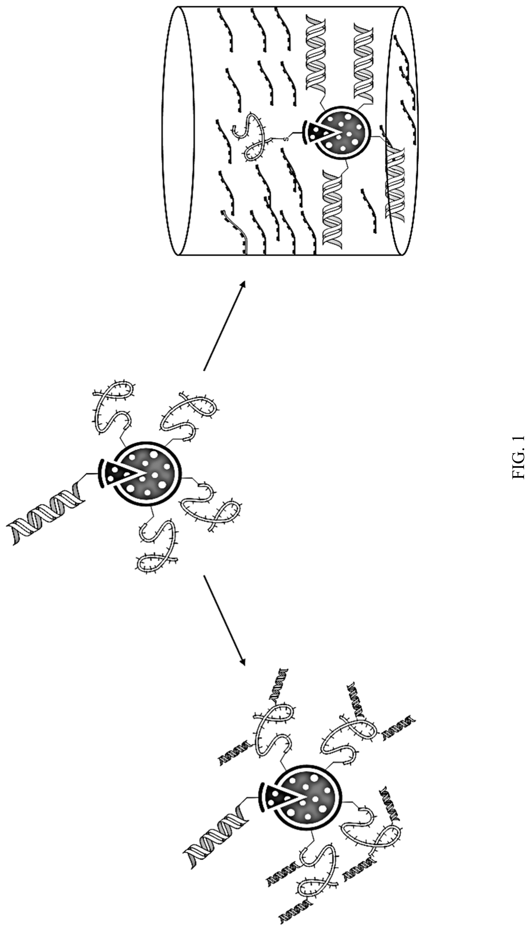

FIG. 1 shows an illustration of a non-limiting example of a nanoparticle as disclosed herein. In this non-limiting example, a single template polynucleotide site is shown as a wedge-shaped portion of a scaffold portion of a nanoparticle as disclosed. A single template polynucleotide is shown bound to the single template site. Also in this non-limiting example, a plurality of accessories are shown extending from accessory sites of the scaffold. In the center illustration, the accessories are shown as polymers. In the left panel, a number of copies of polynucleotides complementary to the template polynucleotide and copes of the template polynucleotide are shown, with such copies attached to and extending from the scaffold. In this example, they extend from the polymers, which in turn extend from the scaffold.

In the right panel, a nanoparticle with a template polynucleotide bound thereto at the single template site is shown in a well of a substrate. A plurality of accessory oligonucleotides are shown extending from the scaffold. Although not shown in the righthand panel, in this example the accessory oligonucleotides extend from the polymers that are attached to the scaffold. Nucleotide sequences of the accessory oligonucleotides are complementary to primers attached to the surface of the well. The accessory oligonucleotides thereby hybridize to the well-attached primers and attach to the surface of the well. Here, only one nanoparticle can be present in the well at a time because of the size of the nanoparticle relative to the size of the well. Thus, clustering initiated from the single template

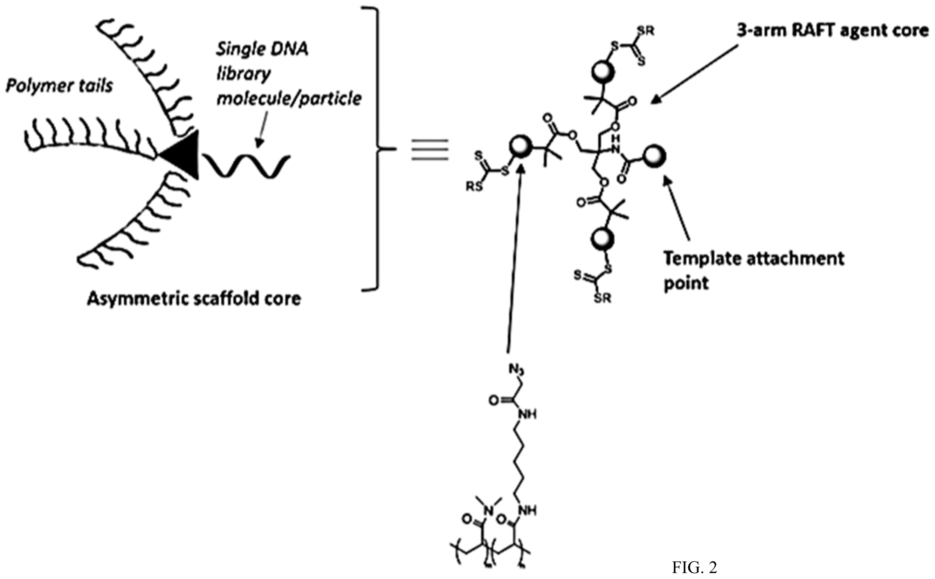

FIG. 2 is an illustration of a non-limiting example of a nanoparticle scaffold in accordance with the present disclosure. On the left, the scaffold is indicated as an asymmetric scaffold, bearing three polymer tails and a single template polynucleotide attached to the scaffold. On the right, an example of a molecular structure of a nanoparticle is depicted (including a 3-arm RAFT agent core), with a single template attachment point shown and polymers extending from a core of the scaffold. Also shown is a non-limiting example of acrylamide monomers that may form part of a scaffold, including accessory sites, depicted in this example as an azide group (e.g, a matrix monomer and primer attachment point).

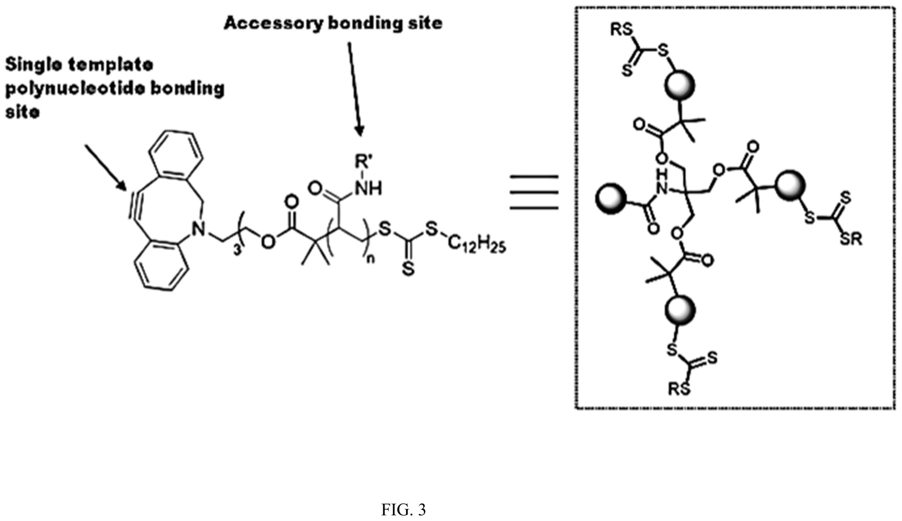

FIG. 3 shows another non-limiting example of a scaffold polymer as disclosed herein. On the right, a scaffold core with three polymer arms extending therefrom is shown, and a single template polynucleotide attachment site. On the left, the chemical structure of the core and one of the polymer arms is shown (the other polymer arms are omitted for purposes of clarity of illustration but would be included in the scaffold). A dibenzocyclooctene group is shown as the single template polynucleotide attachment site Accessory binding sites are shown on the illustrated polymer arm.

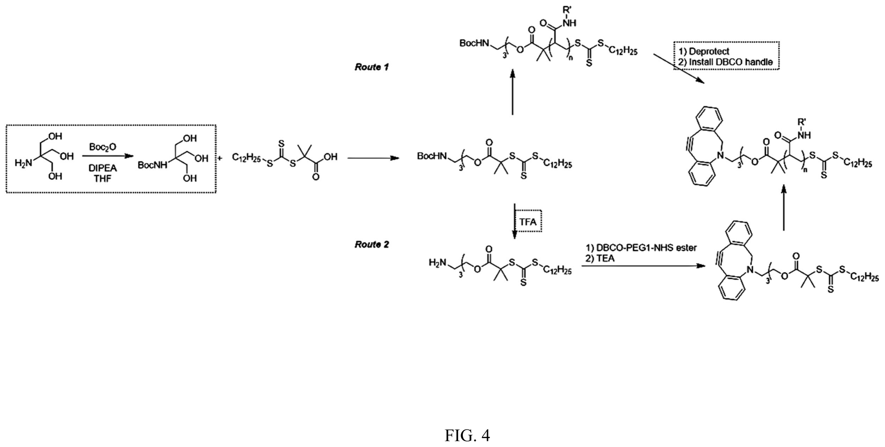

FIG. 4 depicts another non-limiting example of a synthesis scheme for an example of a scaffold in accordance with aspects of the present disclosure. In the example, the amine terminal of a tris molecule is blocked (t-butyl dicarbonate, or Boc2O, DIPEA, or N,N-Diisopropylethylamine, and THF, or Tetrahydrofuran), and a 2-(Dodecylthiocarbonothioylthio)-2-methylpropionic acid (DDMAT) group is added to the hydroxyl ends of the tris core. Two options for polymerization thereafter are shown. In Route 1, a standard aqueous RAFT polymerization is performed to add monomers to the chain, followed by deprotection of the amine group and attachment of a dibenzocyclooctene (DBCO) group thereto. In alternative Route 2, the block of the amine group is removed first with triflouroacetic acid (TFA), and the dibenzycyclooctene group is attached (DBCO-PEG1-NHS ester) first before RAFT polymerization is performed to add monomer groups.

An alternative scheme for synthesizing a scaffold in accordance with the present disclosure is as follows. 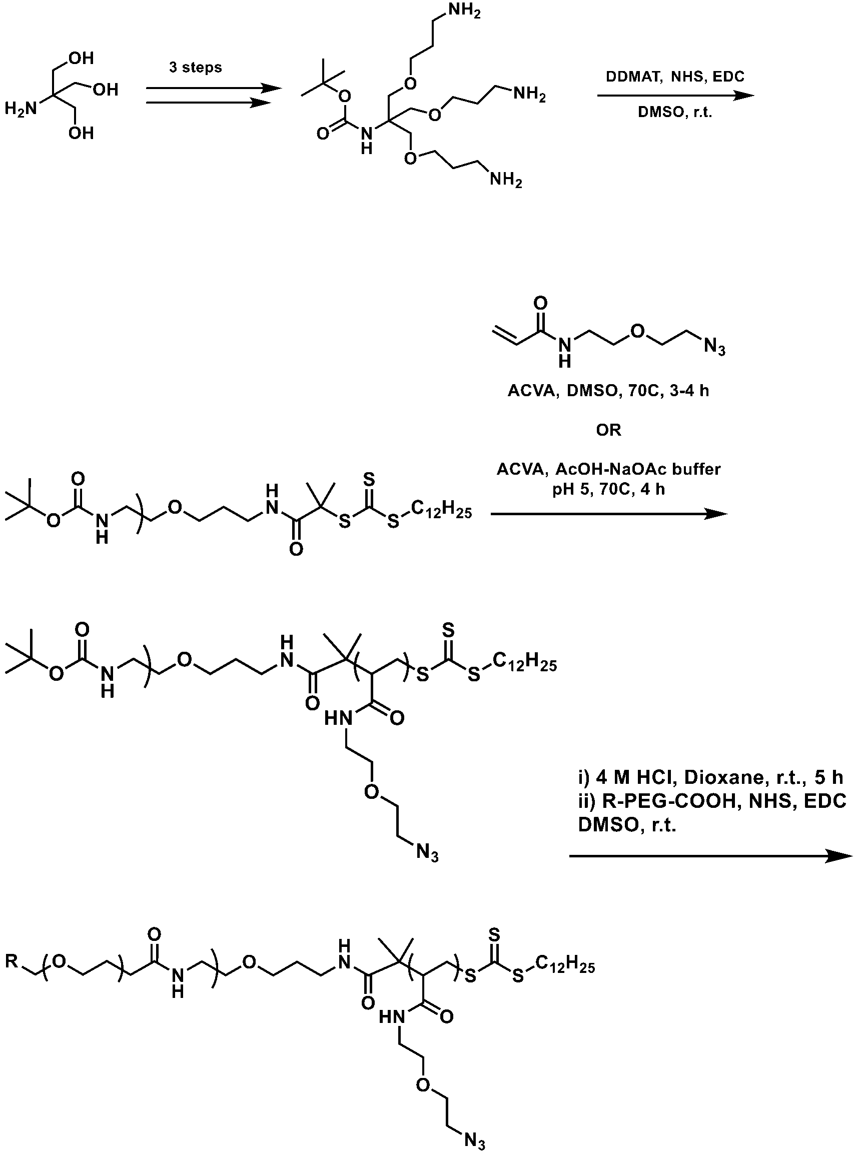 (in this example, for simplicity of illustration only one of the polymer arms is indicated after DDMAT addition, though all three arms would have the same structure). In this non-limiting working example, a PEG linker has been added between the template polynucleotide site (R) and the polymer arms, which include accessory sites, azide groups in this example.

Another scheme for synthesizing a scaffold that was done in accordance with aspects of the present disclosure is according to the following non-limiting working example: 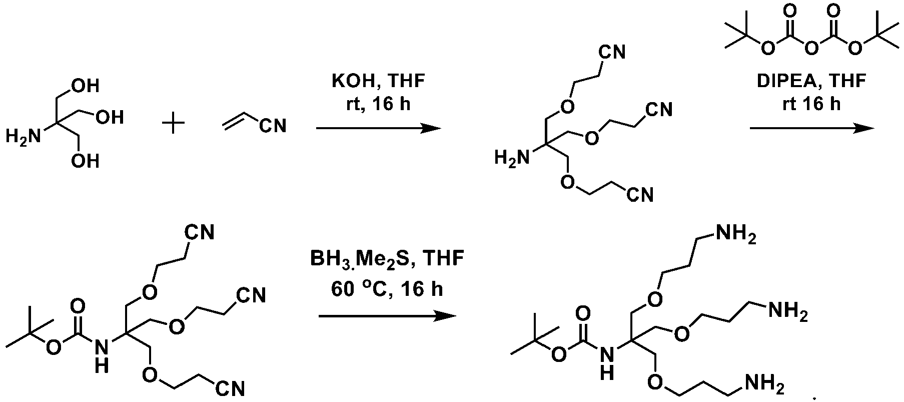 From there, the following non-limiting example of a synthesis scheme may be performed (again showing only one of three polymer arms for purposes of illustrative clarity though all polymer arms would be synthesized according to the same scheme):

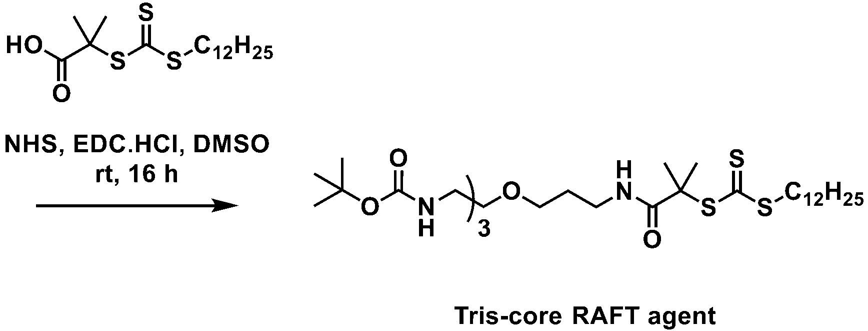

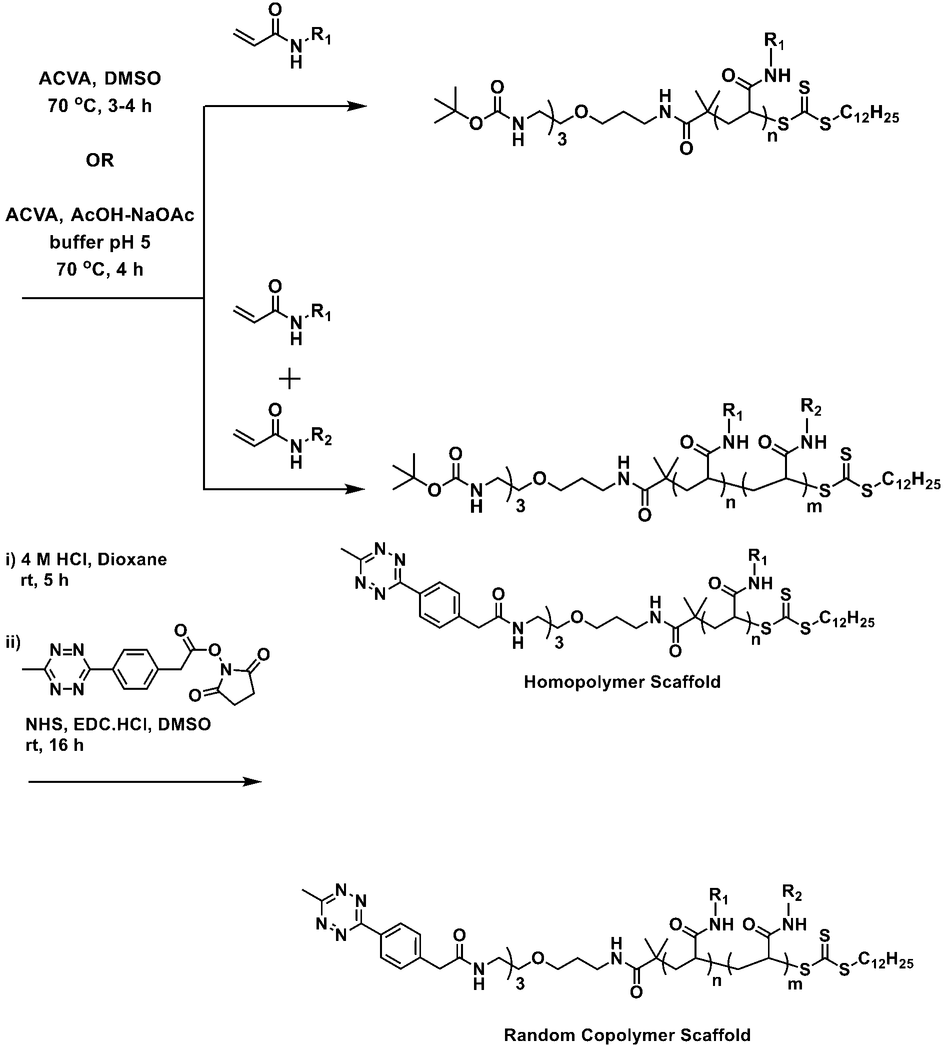

In this non-limiting example, the single template polynucleotide site is a tetrazine. The following R₁-bearing monomers may be added during polymerization for synthesis of different species of scaffold as disclosed herein (for adding an azide group for accessory bonding sites, as a non-limiting example):

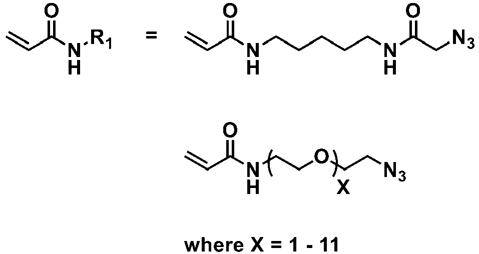

The following R₂-bearing monomers may optionally be added during polymerization for synthesis of different species of scaffold as disclosed herein::

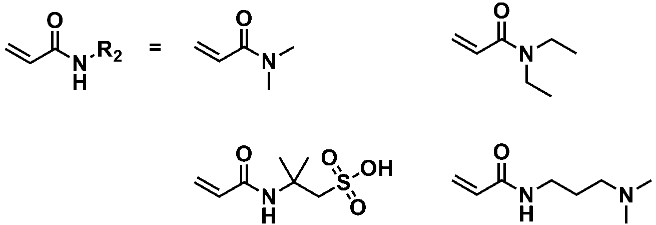

Acrylamide monomers may optionally be PEGylated according to the following scheme:

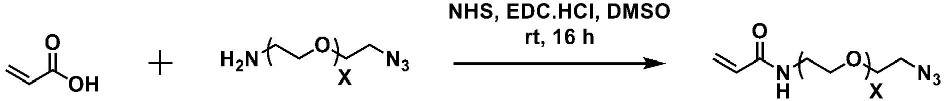

An alternative scheme for synthesizing a scaffold, from a triazine core, in accordance with the present disclosure is according to the following non-limiting example:

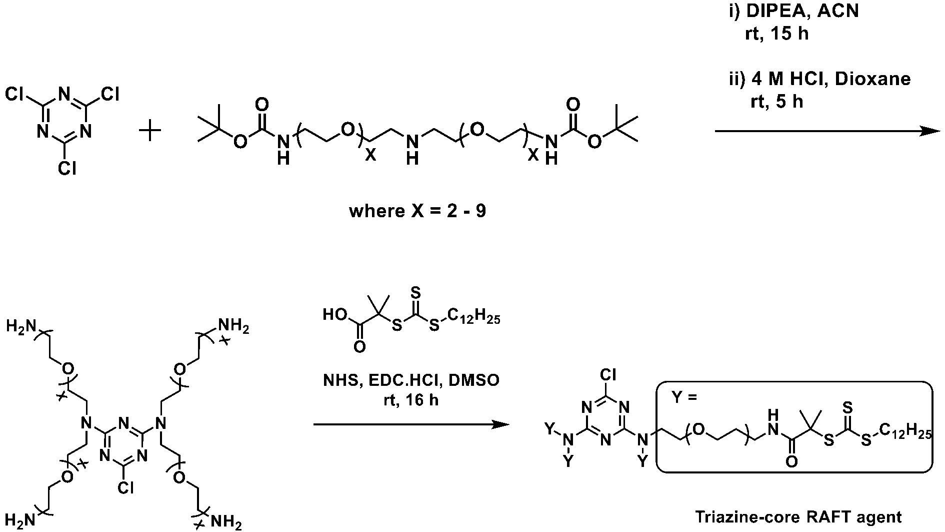

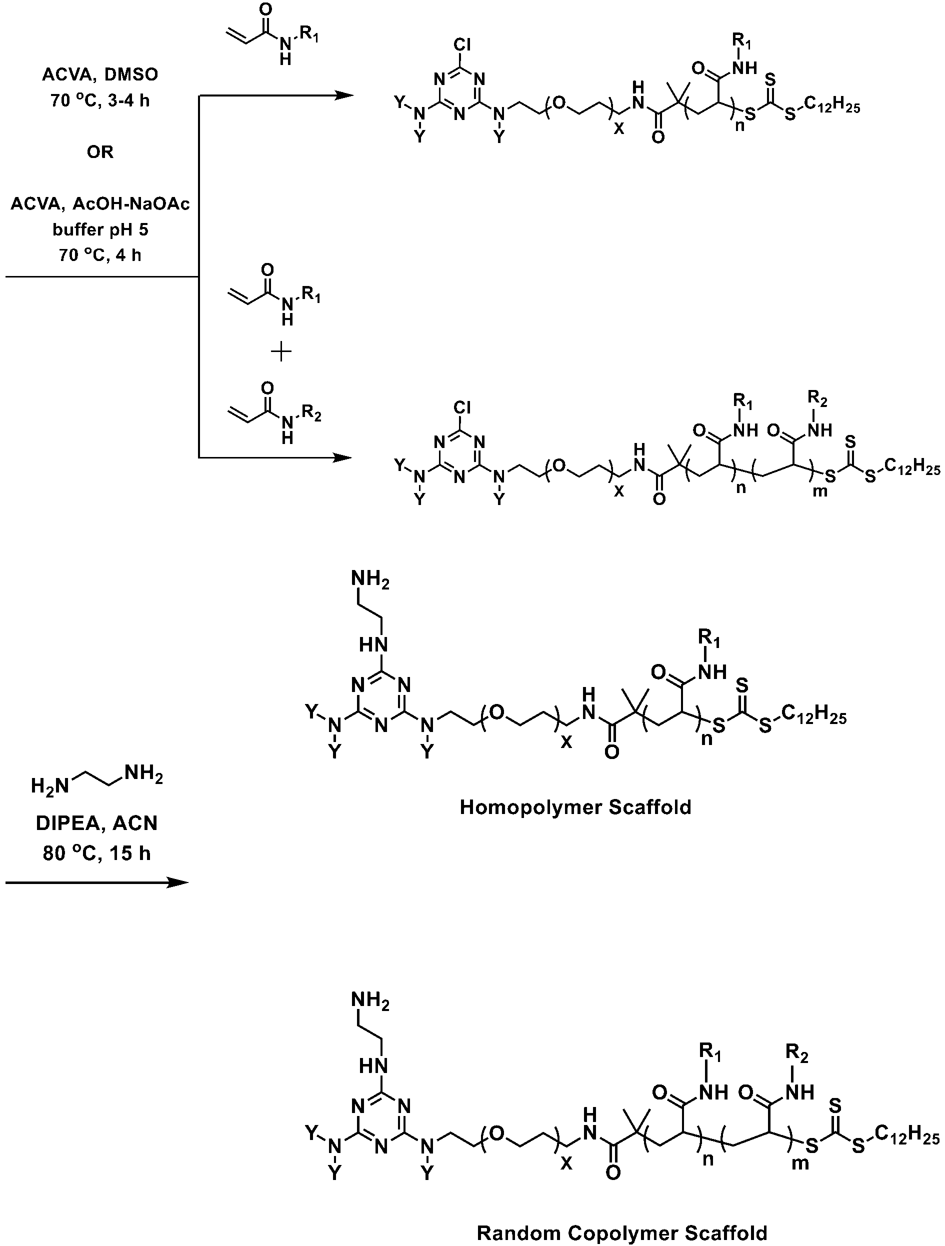

In this non-limiting example, the single template polynucleotide site is an amine, extending from the top of the tetrazine core as shown above. The following R₁-bearing monomers may be added during polymerization for synthesis of different species of scaffold as disclosed herein (for adding an azide group for accessory bonding sites, as a non-limiting example):

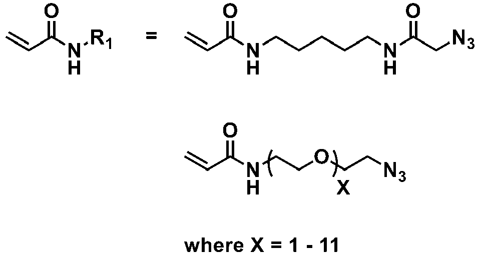

The following R₂-bearing monomers may optionally be added during polymerization for synthesis of different species of scaffold as disclosed herein::

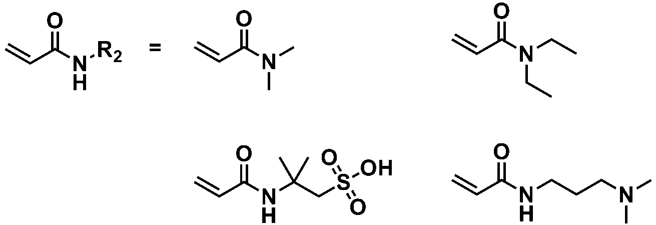

Acrylamide monomers may optionally be PEGylated according to the following scheme:

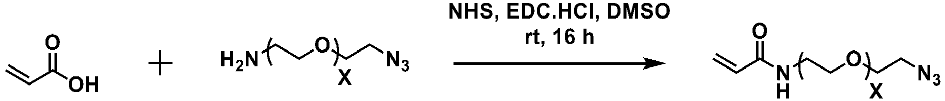

Bifunctional PEGylated secondary amines for addition to a triazine scaffold core may be synthesized according to the following scheme:

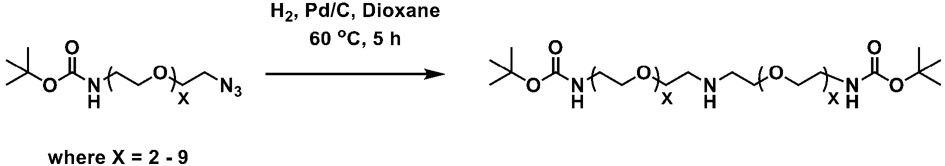

Stoichiometric control of components added during the initial addition of bifunctional PEGylated secondary amines to a triazine scaffold core and purification methods (based on, for example, differences in polarity) may be used to favor production of and isolate bi-substituted triazines (with two bifunctional secondary amines added) as opposed to mono- and tri-substituted products.

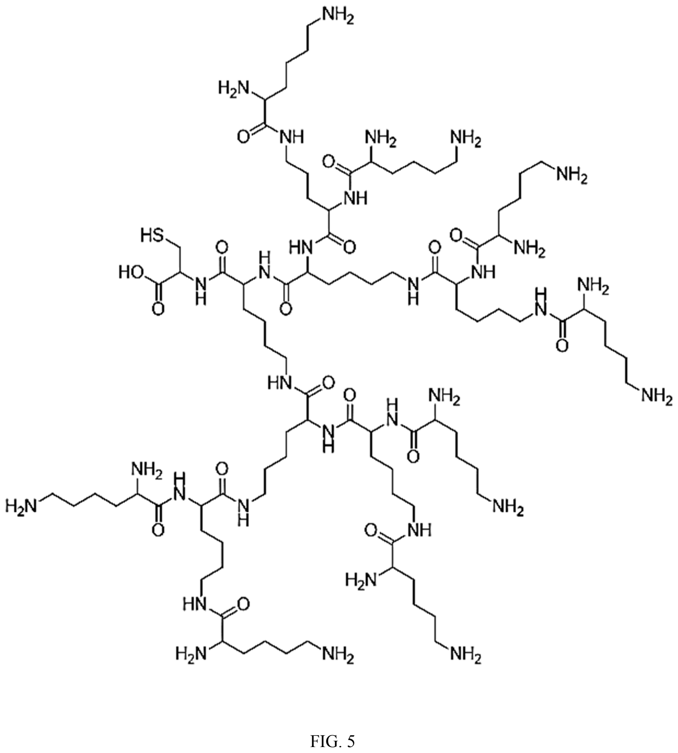

FIG. 5 shows an example of a dendrimer scaffold in accordance with aspects of the present disclosure. In this example, a polypeptide including four lysines and ending with a C-terminal cysteine is shown. The second, third, and fourth lysines include forks including an isopeptide bond via their epsilon amino groups to another lysine group. The second lysine has a single lysine attached by an isopeptide bond. The third lysine is attached to a dilysine, which has another lysine linked thereto by an isopeptide bond to an epsilon amino group of its C-terminal lysine. The fourth lysine attached to a trilysine residue, to which another lysine is attached to its second lysine by an isopeptide bond, and a dilysine reside with another lysine attached thereto by an isopeptide bond is attached to its C-terminal lysine.

Thus, in this example of a dendron scaffold, different numbers of generations emerge from branches extending from a cysteine core. In other example, more or fewer generations may be included, and different branches may have the same number of generations as each other different numbers of generations from each other. In other examples, additional species of amine acids may be included, such as via peptide bonds to lysine residues, between lysyl forks or at the end bearing the single template polynucleotide site.

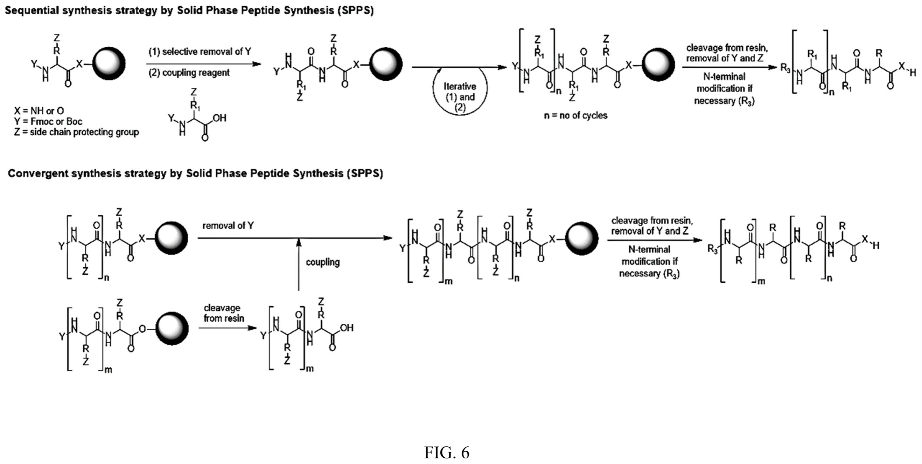

FIG. 6 illustrates synthesis schemes for nanoparticles with branched lysine substituents with alpha amino peptide and epsilon-amino isopeptide bonds, such as in a lysyl dendrimer structured scaffold or scaffold of lysine-containing polypeptides linked together by isopeptide bonds at epsilon-amino groups of lysine substituents to form a branched polypeptide scaffold. Solid phase peptide synthesis may be used to synthesize a sequence of amino acids together in, for example, a linear polypeptide chain according to the upper panel of FIG. 6 (sequential synthesis strategy by Solid Phase Peptide Synthesis (SPSS)). An amine group is blocked with fluorenylmethyloxycarbonyl (Fmoc) or tert-butyloxycarbonyl (Boc). Protective group is selectively removed in the presence of added composition/coupling reagent. By iterative protection of the free amino group (adding n number of monomers where n = number of cycles), followed by its removal and addition of an activated amino acid, a linear chain polypeptide may be synthesized (adding n number of monomers where n = number of cycles). Linear strands of charged amino acids including one or more lysine residues may thereby be generated as components of a scaffold. Composition may then be cleaved from a substrate resin and further modified as may be desirable.

As further depicted in the bottom panel of FIG. 6, a convergent solid phase protein synthesis method may be used wherein lengths of individually formed polypeptide chains including one or more lysine residues may be added as polymer sets during a synthesis step (lower panel, convergent synthesis strategy by solid phase peptide synthesis (SPPS)). In some examples, not depicted in FIG. 6, amino acids or polypeptides may be independently added as branches to a fork where the fork is a structure containing two amino groups (i.e., an N-terminal, such as an alpha-amino group of a lysine, or of another amino acid) and an epsilon amino acid of a lysine), rather than concatenated linearly from a single amino group as depicted in FIG. 22. For example, a lysine amino acid, having two amino groups may serve as a forked attachment point to which two branches of a linear polypeptide, or two individual lysine residues, may be attached, one to each amino group according to the solid phase protein synthesis scheme depicted in FIG. 6.

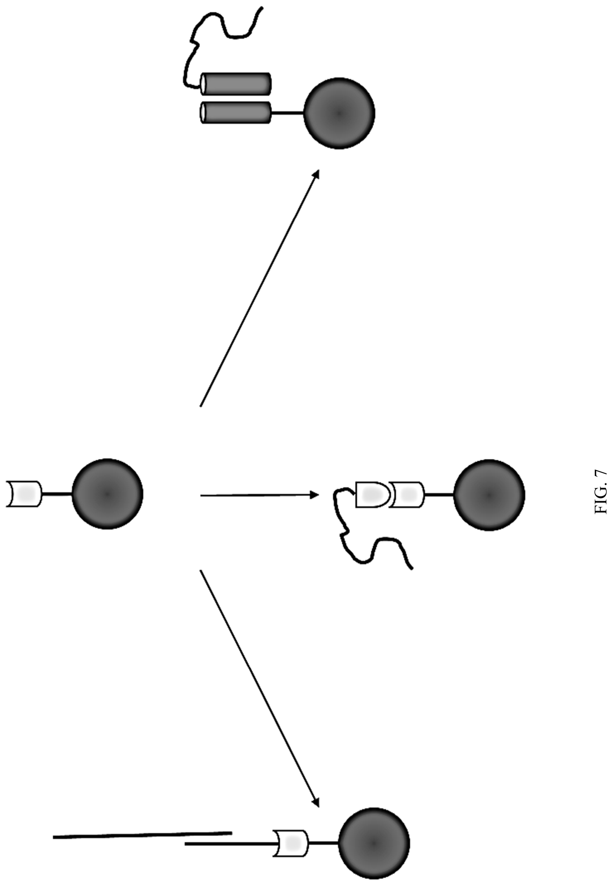

FIG. 7 is an illustration of different methods for bonding a template polynucleotide to a scaffold. At the top a scaffold is shown with a single template polynucleotide site. To the left, a template site primer is included in the single template polynucleotide site, and end of which is complementary to an end of a template polynucleotide, to permit non-covalent bonding of a template polynucleotide to the scaffold by Watson-Crick base pair hybridization. In the middle, a template polynucleotide and the single template polynucleotide site possess respectively complementary moieties or structures resulting in formation of a covalent bond forming between the template polynucleotide and the scaffold. On the right, a polypeptide is included in the single template site, and a polypeptide complementary thereto is attached to an end of the template polynucleotide. Noncovalent bonding between the polypeptide of the single template site and the template polynucleotide bonds a template polynucleotide to the scaffold.

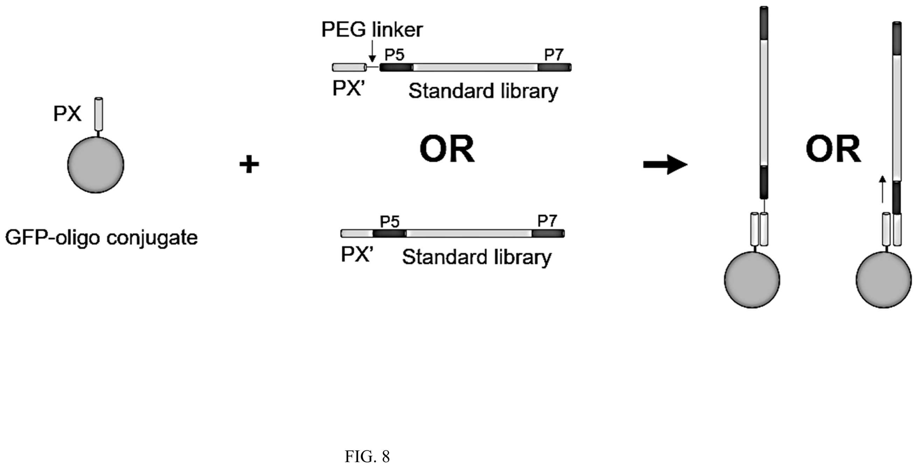

FIG. 8 shows examples of a template polynucleotide bonded to a single template site of a protein scaffold by hybridization to a template site primer represented by PX (GFP-oligo conjugate). Two examples of template polynucleotides of a library are shown, one a standard library molecule with P5 and P7 sequences, with a region complementary to the PX template site primer (denominated PX') at the 3-prime end, or a modified version where the PX' sequence is separated from the P5 sequence by a PEG linker. Ether strand bonds to a single template site of a scaffold. The standard library sequence can be used in a first strand extension polymerization reaction, with the PX primer serving as an initiation primer for polymerization of a nascent strand complementary to the template polynucleotide..

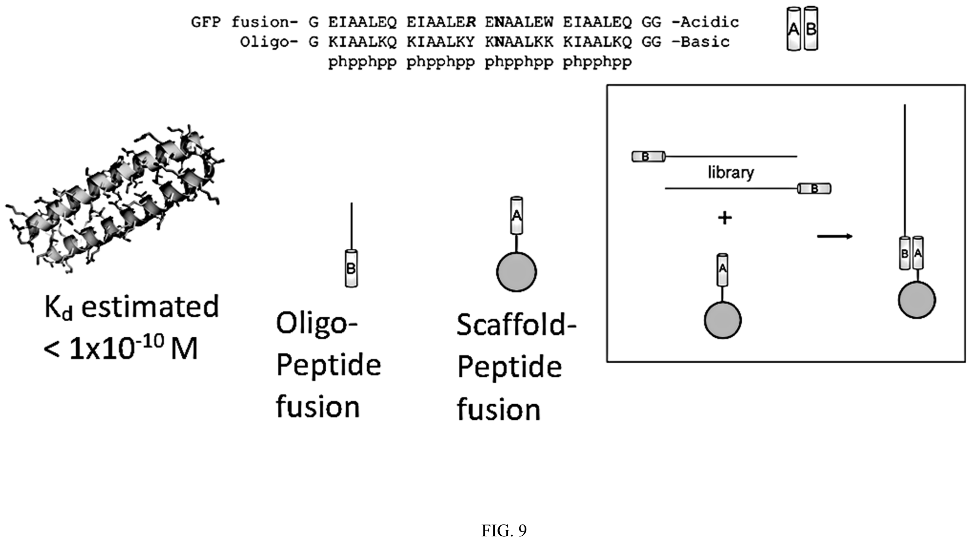

FIG. 9 shows an example of a non-covalent bond, specifically a coiled coil peptide non-covalent bond (in an example, with K_{D} in the picomolar range < 1 x 10⁻¹⁰ M). Two amino acid sequences for alpha helical polypeptide structures are shown that form two complementary bonding partners of a coiled coil attachment. By attaching one such sequence to the scaffold or to a library template polynucleotide, the template polynucleotide can be bound to the scaffold via non-covalent bonding between the alpha helices. In another example, one of the alpha helical sequences complementary to that attached to the scaffold can be attached to an accessory such as an accessory oligonucleotide for attachment of an accessory oligonucleotide to an accessory site.

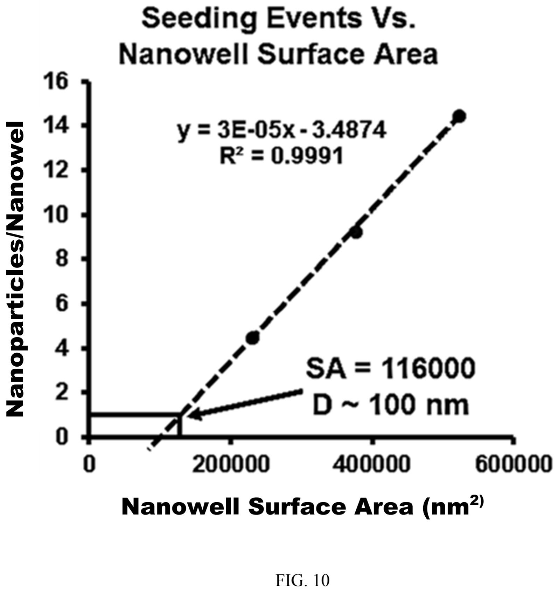

FIG. 10 shows a graph (Seeding Events vs. Nanowell Surface Area) of an example of tests of numbers of scaffolds of a given size that can be present in a nanowell (Dendrimers/Nanowell) of a given nanowell surface area (SA) or diameter (D). Nanoparticles (having a structure different from structures of nanoparticles of nanoparticles as disclosed herein) of about 100 nm in diameter were seeded into nanowells 185, 285, or 375 nm in diameter and the number of dendrimers per nanowell measured. Extrapolating from the best fit curve of the results (y = 3E-0.5x - 3.4874, R² = 0.9991) indicates that single-nanoparticle seeding of a nanowell would result, in this examples, of using a nanoparticle with a diameter of about 100 nm and a nanowell with a diameter of about 100 nm.

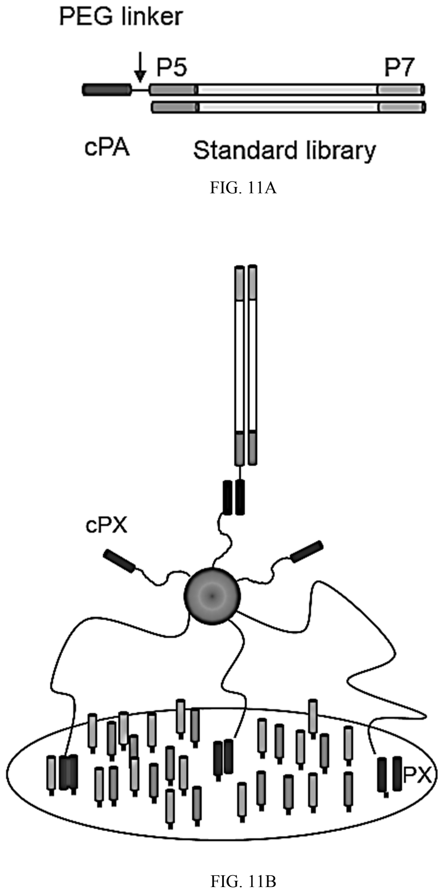

FIGs. 11A-11C show an example of seeding a substrate with template polynucleotides using a scaffold in accordance with aspects of the present disclosure. Scaffolds, structured as DNA dendrimer scaffolds in accordance with U.S. Provisional Application No. 62/952,799, had a diameter of from 50 nm - 150 nm. The scaffold includes a single template site (Pa) for bonding a template polynucleotide and a plurality of accessory sites (cPX). FIG. 11A shows a template polynucleotide and its complement, with a primer sequence added to each end (P5/cP5 and P7/cP7). The P5-primer end of the template polynucleotide is connected to a primer (cPa) by a PEG linker. The cPa primer is complementary to the single template site (Pa) of the scaffold. FIG. 11B is a depiction of the scaffold molecule hybridized, via its single template site (Pa), to the template polynucleotide and its complement depicted in FIG. 11A, via the cPa primer. The scaffold is attached to a substrate. The substrate is attached to primers (PX) that are complementary to accessory sites (cPX).of the scaffold. The substrate is also attached to primers to permit hybridization of template ends thereto to permit clustering on the substrate

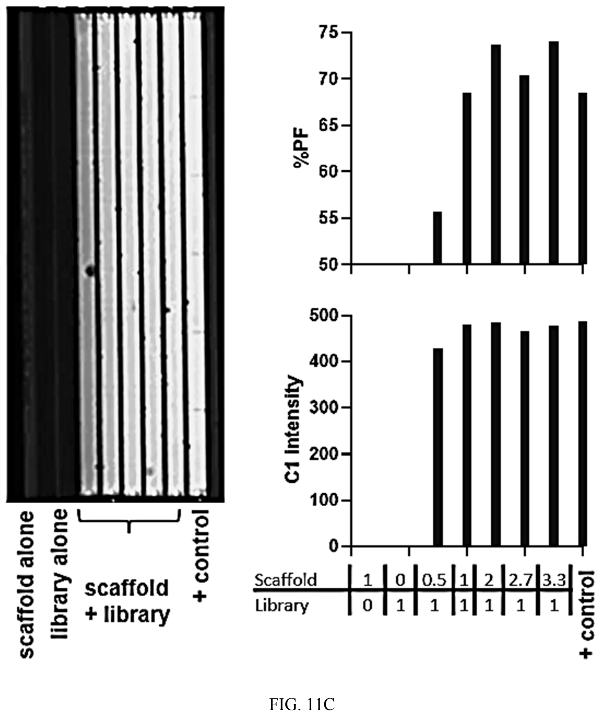

FIG. 11C depicts an example according to the foregoing demonstrating seeding a substrate with a template polynucleotide using a scaffold with a single template site followed by clustering. Scaffolds attached to template polynucleotides according to aspects of the present disclosure and FIGs. 11A and 11B. Scaffolds were combined with template polynucleotides (library) at the molar rations shown, a substrate (flow cell with nanowells for seeding) seeded therewith, then clustering performed according to a recombinase-driven cluster amplification process (ExAmp cluster amplification). Negative controls include scaffold without template and template without scaffold. As a positive control (+ control), clustering on substrate was performed without scaffold, using clustering on substrate following hybridization of template molecules to primers attached to the substrate not via a scaffold.

The left panel is an image of a flow cell following a clustering process according to the above conditions (2 negative controls, 5 conditions of various scaffold:template molar ratios, and 1 positive control). Fluorescence in all conditions except the negative controls indicate that a scaffold with a single template binding site can seed a substrate with a template polynucleotide and support a clustering process. Bar graphs are quantitative measurements of clustering results of the 8 conditions. Upper graph, C1 intensity is cycle 1 intensity as an indirect measure of the cluster size or yield (with intensity being directly proportional to cluster size or yield). Lower graph, %PF is % passing filter, which is the percent of nanowells passing a threshold filter indicating purity of cluster formed therein, i.e. directly proportional to number of nanowells with monoclonal clusters.

It should be appreciated that all combinations of the foregoing concepts and additional concepts discussed in greater detail herein (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein and may be used to achieve the benefits and advantages described herein.

The following numbered paragraphs set out certain aspects and features of the present invention.
1. A nanoparticle, comprising
   a scaffold, a single template site for bonding a template polynucleotide to the scaffold selected from a covalent template bonding site and a noncovalent template bonding site, and a plurality of accessory sites for bonding accessory oligonucleotides to the scaffold selected from covalent accessory oligonucleotide bonding sites and noncovalent accessory oligonucleotide bonding sites, wherein
   the scaffold is a compound of Formula I: 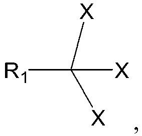
      each X is a compound of formula II: 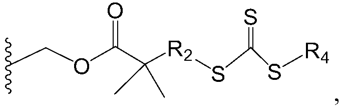
      wherein R₂ is selected from Formula IIIa: 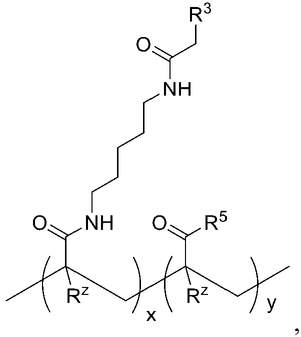 wherein R⁵ is selected from 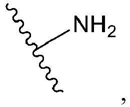 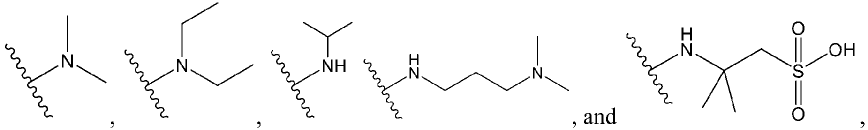 x is an integer in the range of from 1-20,000 and y is an integer in the range of from 1-100,000 and a ratio of x:y may be from approximately 10:90 to approximately 1:99, and wherein each R² is independently H or C₁₋₄ alkyl, and Formula IIIb: 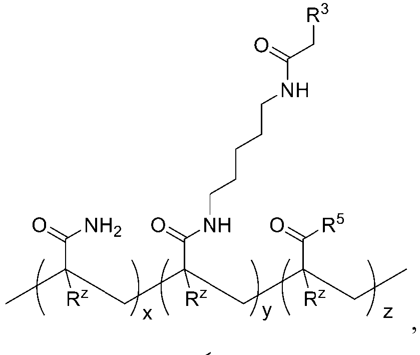 wherein R⁵ is selected from 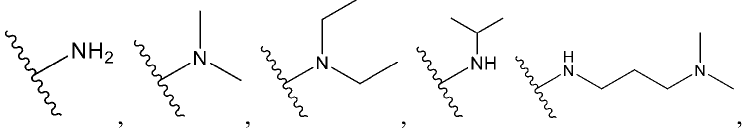 and 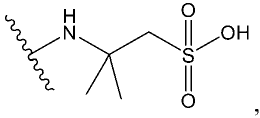 y is an integer in the range of from 1-2,000 and x and z are integers whose sum is in a range of from 1-10,000 and a ratio of (x:y):z may be from approximately (85):15 to approximately (95):5, and wherein each R^{z} is independently H or C₁₋₄ alkyl,
   R₁ includes the single template site for bonding a template polynucleotide to the scaffold, R⁴ is selected from an optionally substituted C₁-C₂₀ alkyl, an optionally substituted C₁-C₂₀ alkenyl, an optionally substituted C₁-C₂₀ alkynyl, an optionally substituted C₁-C₂₀ oxaalkyl, an optionally substituted C₁-C₂₀ thiaalkyl, and an optionally substituted C₁-C₂₀ azaalkyl, wherein substituted comprises substitution with one or more of a C₁-C₂₀ alkyl, a double-bonded oxygen, and a hydroxyl group, and R³ includes the accessory site for bonding accessory oligonucleotides.
2. The nanoparticle of paragraph 1, wherein the single template site comprises a covalent template bonding site.
3. The nanoparticle of paragraph 2, wherein the covalent template bonding site is selected from amine-NHS ester bonding site, an amine-imidoester bonding site, an amine-pentofluorophenyl ester bonding site, an amine-hydroxymethyl phosphine bonding site, a carboxyl-carbodiimide bonding site, a thiol-maleimide bonding site, a thiol-haloacetyl bonding site, a thiol-pyridyl disulfide bonding site, a thiol-thiosulfonate bonding site, a thiol-vinyl sulfone bonding site, an aldehyde-hydrazide bonding site, an aldehyde-alkoxyamine bonding site, an aldehyde-NHS ester bonding site, a hydroxy-isocyanate bonding site, an azide-alkyne bonding site, an azide-phosphine bonding site, a transcyclooctene-tetrazine bonding site, a norbornene-tetrazine bonding site, an azide-cyclooctyne bonding site, an azide-norbornene bonding site, an oxime bonding site, a SpyTag-SpyCatcher bonding site, a Snap-tag-O⁶-Benzylguanine bonding site, a CLIP-tag-O²-benzylcytosine bonding site, and a sortase-coupling bonding site.
4. The nanoparticle of paragraph 1, wherein the single template site comprises a noncovalent template bonding site.
5. The nanoparticle of paragraph 4, wherein the noncovalent template bonding site comprises a polynucleotide hybridization site.
6. The nanoparticle of paragraph 4, wherein the noncovalent template bonding site comprises a noncovalent peptide binding site and the noncovalent peptide binding site is selected from a coiled-coil bonding site and an avidin-biotin bonding site.
7. The nanoparticle of any one of paragraphs 1 through 6, wherein the plurality of accessory sites for bonding accessory oligonucleotides to the scaffold comprise covalent accessory oligonucleotide bonding sites.
8. The nanoparticle of paragraph 7, wherein the covalent accessory oligonucleotide bonding sites are selected from amine-NHS ester bonding sites, amine-imidoester bonding sites, amine-pentofluorophenyl ester bonding sites, amine-hydroxymethyl phosphine bonding sites, carboxyl-carbodiimide bonding sites, thiol-maleimide bonding sites, thiol-haloacetyl bonding sites, thiol-pyridyl disulfide bonding sites, thiol-thiosulfonate bonding sites, thiol-vinyl sulfone bonding sites, aldehyde-hydrazide bonding sites, aldehyde-alkoxyamine bonding sites, hydroxy-isocyanate bonding sites, azide-alkyne bonding sites, azide-phosphine bonding sites, transcyclooctene-tetrazine bonding sites, norbornene-tetrazine bonding sites, an azide-cyclooctyne bonding sites, azide-norbornene bonding sites, oxime bonding sites, SpyTag-SpyCatcher bonding sites, Snap-tag-O⁶-Benzylguanine bonding sites, CLIP-tag-O²-benzylcytosine bonding sites, sortase-coupling bonding sites, and any combination of two or more of the foregoing.
9. The nanoparticle of any one of paragraphs 1 through 6, wherein the accessory oligonucleotide bonding sites comprise noncovalent accessory oligonucleotide bonding sites.
10. The nanoparticle of paragraph 9, wherein the noncovalent accessory oligonucleotide bonding sites comprise polynucleotide hybridization sites.
11. The nanoparticle of paragraph 9, wherein the noncovalent accessory oligonucleotide bonding sites comprise noncovalent peptide binding sites and the noncovalent peptide binding sites are selected from one or both of coiled-coil bonding sites and avidin-biotin bonding sites.
12. The nanoparticle of any one of paragraphs 1 through 11, further comprising a single template polynucleotide bonded to the single template site.
13. The nanoparticle of any one of paragraphs 1 through 12, further comprising a plurality of accessory oligonucleotides bonded to the plurality of accessory sites.
14. The nanoparticle of any one of paragraphs 1 through 13, wherein the nanoparticle is at least about 10 nm in diameter.
15. A method, comprising bonding a single template polynucleotide to the single template site of the nanoparticle of any one of paragraphs 1 through 14.
16. A method, comprising bonding a plurality of accessory oligonucleotides to the plurality of accessory sites of the nanoparticle of any one of paragraphs 1 through 15.
17. The method of paragraph 15 or 16, further comprising synthesizing one or more scaffold-attached copies selected from copies of the template polynucleotide, copies of the polynucleotides complementary to the template polynucleotide, and copies of both, wherein the scaffold-attached copies extend from the accessory oligonucleotides.
18. The method of any one of paragraphs 15-17, further comprising attaching the scaffold to a substrate, wherein attaching comprises hybridizing accessory oligonucleotides with oligonucleotides attached to the substrate.
19. The method of paragraph 18, wherein the substrate comprises a plurality of nanowells and the oligonucleotides attached to the substrate are attached within the plurality of nanowells.
20. The method of paragraph 19, wherein no more than one scaffold binds within any one of the nanowells.
21. The method of any one of paragraphs 18 through 20 further comprising synthesizing one or more substrate-attached copies selected from copies of the template polynucleotide, copies of the polynucleotides complementary to the template polynucleotide, and copies of both, wherein the substrate-attached copies extend from oligonucleotides attached to a substrate.
22. The method of paragraph 17 or 21, further comprising sequencing at least one of scaffold-attached copies and substrate-attached copies, wherein sequencing comprises sequencing by synthesis.
23. A nanoparticle, comprising
   a scaffold, a single template site for bonding a template polynucleotide to the scaffold selected from a covalent template bonding site and a noncovalent template bonding site, and a plurality of accessory sites for bonding accessory oligonucleotides to the scaffold selected from covalent accessory oligonucleotide bonding sites and noncovalent accessory oligonucleotide bonding sites, wherein
   the scaffold comprises a dendrimer wherein the dendrimer comprises from 2 to 10 generations of constitutional repeating units, the constitutional repeating units comprise lysine wherein a lysine of an upstream generation forms a peptide bond with a first lysine of an immediately downstream generation and an isopeptide bond with a second lysine of the immediately downstream generation,
   the single template site extends from the C-terminal end of the lysine of the first generation of the dendrimer and the plurality of accessory sites extend from NH₂ groups of lysines of the last generation of the dendrimer.
24. The nanoparticle of paragraph 23, wherein the single template site comprises a covalent template bonding site.
25. The nanoparticle of paragraph 24, wherein the covalent template bonding site is selected from an amine-NHS ester bonding site, an amine-imidoester bonding site, an amine-pentofluorophenyl ester bonding site, an amine-hydroxymethyl phosphine bonding site, a carboxyl-carbodiimide bonding site, a thiol-maleimide bonding site, a thiol-haloacetyl bonding site, a thiol-pyridyl disulfide bonding site, a thiol-thiosulfonate bonding site, a thiol-vinyl sulfone bonding site, an aldehyde-hydrazide bonding site, an aldehyde-alkoxyamine bonding site, a hydroxy-isocyanate bonding site, an azide-alkyne bonding site, an azide-phosphine bonding site, a transcyclooctene-tetrazine bonding site, a norbornene-tetrazine bonding site, an azide-cyclooctyne bonding site, an azide-norbornene bonding site, an oxime bonding site, a SpyTag-SpyCatcher bonding site, a Snap-tag-O⁶-Benzylguanine bonding site, a CLIP-tag-O²-benzylcytosine bonding site, and a sortase-coupling bonding site.
26. The nanoparticle of any one of paragraph 23, wherein the single template site comprises a noncovalent template bonding site.
27. The nanoparticle of paragraph 26, wherein the noncovalent template bonding site comprises a polynucleotide hybridization site.
28. The nanoparticle of paragraph 28, wherein the noncovalent template bonding site comprises a noncovalent peptide binding site and the noncovalent peptide binding site is selected from a coiled-coil bonding site and an avidin-biotin bonding site.
29. The nanoparticle of any one of paragraphs 23 through 28, wherein the plurality of accessory sites for bonding accessory oligonucleotides to the scaffold comprise covalent accessory oligonucleotide bonding sites.
30. The nanoparticle of paragraph 29, wherein the covalent accessory oligonucleotide bonding sites are selected from amine-NHS ester bonding sites, amine-imidoester bonding sites, amine-pentofluorophenyl ester bonding sites, amine-hydroxymethyl phosphine bonding sites, carboxyl-carbodiimide bonding sites, thiol-maleimide bonding sites, thiol-haloacetyl bonding sites, thiol-pyridyl disulfide bonding sites, thiol-thiosulfonate bonding sites, thiol-vinyl sulfone bonding sites, aldehyde-hydrazide bonding sites, aldehyde-alkoxyamine bonding sites, hydroxy-isocyanate bonding sites, azide-alkyne bonding sites, azide-phosphine bonding sites, transcyclooctene-tetrazine bonding sites, norbornene-tetrazine bonding sites, an azide-cyclooctyne bonding sites, azide-norbornene bonding sites, oxime bonding sites, SpyTag-SpyCatcher bonding sites, Snap-tag-O⁶-Benzylguanine bonding sites, CLIP-tag-O²-benzylcytosine bonding sites, sortase-coupling bonding sites, and any combination of two or more of the foregoing.
31. The nanoparticle of any one of paragraphs 23 through 28, wherein the accessory oligonucleotide bonding sites comprise noncovalent accessory oligonucleotide bonding sites.
32. The nanoparticle of paragraph 31, wherein the noncovalent accessory oligonucleotide bonding sites comprise polynucleotide hybridization sites.
33. The nanoparticle of paragraph 31, wherein the noncovalent accessory oligonucleotide bonding sites comprise noncovalent peptide binding sites and the noncovalent peptide binding sites are selected from one or both of coiled-coil bonding sites and avidin-biotin bonding sites.
34. The nanoparticle of any one of paragraphs 23 through 33, further comprising a single template polynucleotide bonded to the single template site.
35. The nanoparticle of any one of paragraphs 23 through 34, further comprising a plurality of accessory oligonucleotides bonded to the plurality of accessory sites.
36. The nanoparticle of any one of paragraphs 23 through 35, wherein the nanoparticle is at least about 10 nm in diameter.
37. A method, comprising bonding a single template polynucleotide to the single template site of the nanoparticle of any one of paragraphs 23 through 36.
38. A method, comprising bonding a plurality of accessory oligonucleotides to the plurality of accessory sites of the nanoparticle of any one of paragraphs 23 through 37.
39. The method of paragraph 37 or 38, further comprising synthesizing one or more scaffold-attached copies selected from copies of the template polynucleotide, copies of the polynucleotides complementary to the template polynucleotide, and copies of both, wherein the scaffold-attached copies extend from the accessory oligonucleotides.
40. The method of any one of paragraphs 37-39, further comprising attaching the scaffold to a substrate, wherein attaching comprises hybridizing accessory oligonucleotides with oligonucleotides attached to the substrate.
41. The method of paragraph 40, wherein the substrate comprises a plurality of nanowells and the oligonucleotides attached to the substrate are attached within the plurality of nanowells.
42. The method of paragraph 41, wherein no more than one scaffold binds within any one of the nanowells.
43. The method of any one of paragraphs 40 through 42 further comprising synthesizing one or more substrate-attached copies selected from copies of the template polynucleotide, copies of the polynucleotides complementary to the template polynucleotide, and copies of both, wherein the substrate-attached copies extend from oligonucleotides attached to a substrate.
44. The method of paragraph 39 or 43, further comprising sequencing at least one of scaffold-attached copies and substrate-attached copies, wherein sequencing comprises sequencing by synthesis.
45. A nanoparticle, comprising
   a scaffold, a single template site for bonding a template polynucleotide to the scaffold selected from a covalent template bonding site and a noncovalent template bonding site, and a plurality of accessory sites for bonding accessory oligonucleotides to the scaffold selected from covalent accessory oligonucleotide bonding sites and noncovalent accessory oligonucleotide bonding sites, wherein
   the scaffold is a compound of Formula IV: each X is a compound of formula V: 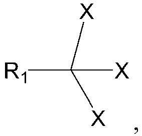 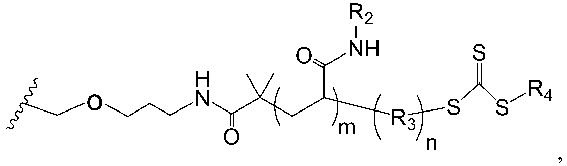 wherein R₂ is selected from Formula VIa: 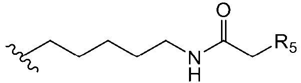 and Formula VIb: 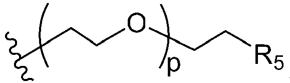 wherein p is an integer selected from 1 to 20, and R₅ comprises the accessory site for bonding accessory oligonucleotides, R³ is selected from a direct bond, 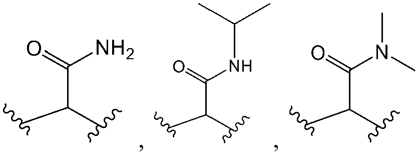 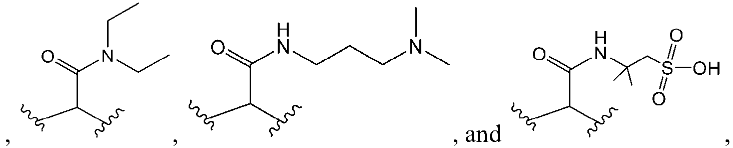 m is an integer from 1 to 20,000 and n is an integer from 1 to 100,000,
   R¹ comprises the single template site for bonding a template polynucleotide to the scaffold, R⁴ is selected from an optionally substituted C₁-C₂₀ alkyl, an optionally substituted C₁-C₂₀ alkenyl, an optionally substituted C₁-C₂₀ alkynyl, an optionally substituted C₁-C₂₀ oxaalkyl, an optionally substituted C₁-C₂₀ thiaalkyl, and an optionally substituted C₁-C₂₀ azaalkyl, wherein substituted comprises substitution with one or more of a C₁-C₂₀ alkyl, a double-bonded oxygen, and a hydroxyl group, and R³ comprises the accessory site for bonding accessory oligonucleotides.
46. The nanoparticle of paragraph 45, wherein the single template site comprises a covalent template bonding site.
47. The nanoparticle of paragraph 46, wherein the covalent template bonding site is selected from an amine-NHS ester bonding site, an amine-imidoester bonding site, an amine-pentofluorophenyl ester bonding site, an amine-hydroxymethyl phosphine bonding site, a carboxyl-carbodiimide bonding site, a thiol-maleimide bonding site, a thiol-haloacetyl bonding site, a thiol-pyridyl disulfide bonding site, a thiol-thiosulfonate bonding site, a thiol-vinyl sulfone bonding site, an aldehyde-hydrazide bonding site, an aldehyde-alkoxyamine bonding site, a hydroxy-isocyanate bonding site, an azide-alkyne bonding site, an azide-phosphine bonding site, a transcyclooctene-tetrazine bonding site, a norbornene-tetrazine bonding site, an azide-cyclooctyne bonding site, an azide-norbornene bonding site, an oxime bonding site, a SpyTag-SpyCatcher bonding site, a Snap-tag-O⁶-Benzylguanine bonding site, a CLIP-tag-O²-benzylcytosine bonding site, and a sortase-coupling bonding site.
48. The nanoparticle of any one of paragraph 45, wherein the single template site comprises a noncovalent template bonding site.
49. The nanoparticle of paragraph 48, wherein the noncovalent template bonding site comprise a polynucleotide hybridization site.
50. The nanoparticle of paragraph 48, wherein the noncovalent template bonding site comprises a noncovalent peptide binding site and the noncovalent peptide binding site is selected from one or both of a coiled-coil bonding site and an avidin-biotin bonding site.
51. The nanoparticle of any one of paragraphs 45 through 50, wherein the plurality of accessory sites for bonding accessory oligonucleotides to the scaffold comprise covalent accessory oligonucleotide bonding sites.
52. The nanoparticle of paragraph 51, wherein the covalent accessory oligonucleotide bonding sites are selected from amine-NHS ester bonding sites, amine-imidoester bonding sites, amine-pentofluorophenyl ester bonding sites, amine-hydroxymethyl phosphine bonding sites, carboxyl-carbodiimide bonding sites, thiol-maleimide bonding sites, thiol-haloacetyl bonding sites, thiol-pyridyl disulfide bonding sites, thiol-thiosulfonate bonding sites, thiol-vinyl sulfone bonding sites, aldehyde-hydrazide bonding sites, aldehyde-alkoxyamine bonding sites, hydroxy-isocyanate bonding sites, azide-alkyne bonding sites, azide-phosphine bonding sites, transcyclooctene-tetrazine bonding sites, norbornene-tetrazine bonding sites, an azide-cyclooctyne bonding sites, azide-norbornene bonding sites, oxime bonding sites, SpyTag-SpyCatcher bonding sites, Snap-tag-O⁶-Benzylguanine bonding sites, CLIP-tag-O²-benzylcytosine bonding sites, sortase-coupling bonding sites, and any combination of two or more of the foregoing.
53. The nanoparticle of any one of paragraphs 45 through 50, wherein the accessory oligonucleotide bonding sites comprises noncovalent accessory oligonucleotide bonding sites.
54. The nanoparticle of paragraph 53, wherein the noncovalent accessory oligonucleotide bonding sites comprise polynucleotide hybridization sites.
55. The nanoparticle of paragraph 53, wherein the noncovalent accessory oligonucleotide bonding sites comprise noncovalent peptide binding sites and the noncovalent peptide binding sites are selected from one or both of coiled-coil bonding sites and avidin-biotin bonding sites.
56. The nanoparticle of any one of paragraphs 45 through 55, further comprising a single template polynucleotide bonded to the single template site.
57. The nanoparticle of any one of paragraphs 45 through 56, further comprising a plurality of accessory oligonucleotides bonded to the plurality of accessory sites.
58. The nanoparticle of any one of paragraphs 45 through 57, wherein the nanoparticle at least about 10 nm in diameter.
59. A method, comprising bonding a single template polynucleotide to the single template site of the nanoparticle of any one of paragraphs 45 through 58.
60. A method, comprising bonding a plurality of accessory oligonucleotides to the plurality of accessory sites of the nanoparticle of any one of paragraphs 45 through 59.
61. The method of paragraph 59 or 60, further comprising synthesizing one or more scaffold-attached copies selected from copies of the template polynucleotide, copies of the polynucleotides complementary to the template polynucleotide, and copies of both, wherein the scaffold-attached copies extend from the accessory oligonucleotides.
62. The method of any one of paragraphs 59-61, further comprising attaching the scaffold to a substrate, wherein attaching comprises hybridizing accessory oligonucleotides with oligonucleotides attached to the substrate.
63. The method of paragraph 62, wherein the substrate comprises a plurality of nanowells and the oligonucleotides attached to the substrate are attached within the plurality of nanowells.
64. The method of paragraph 63, wherein no more than one scaffold binds within any one of the nanowells.
65. The method of any one of paragraphs 62 through 64 further comprising synthesizing one or more substrate-attached copies selected from copies of the template polynucleotide, copies of the polynucleotides complementary to the template polynucleotide, and copies of both, wherein the substrate-attached copies extend from oligonucleotides attached to a substrate.
66. The method of paragraph 61 or 65, further comprising sequencing at least one of scaffold-attached copies and substrate-attached copies, wherein sequencing comprises sequencing by synthesis.
67. A nanoparticle, comprising
   a scaffold, a single template site for bonding a template polynucleotide to the scaffold selected from a covalent template bonding site and a noncovalent template bonding site, and a plurality of accessory sites for bonding accessory oligonucleotides to the scaffold selected from covalent accessory oligonucleotide bonding sites and noncovalent accessory oligonucleotide bonding sites, wherein
   the scaffold is a compound of Formula VII: 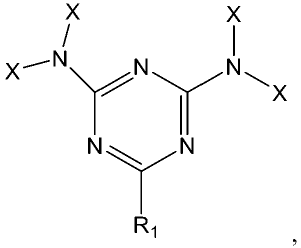 each X is a compound of formula VIII: 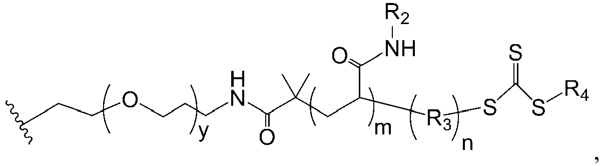 wherein y is an integer from 1 to 20, R₂ is selected from Formula IXa: 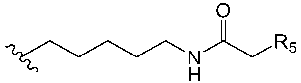 and Formula IXb: 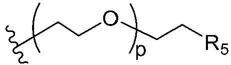 wherein p is an integer selected from 1 to 20, and R₅ comprises the accessory site for bonding accessory oligonucleotides, R₃ is selected from a direct bond, 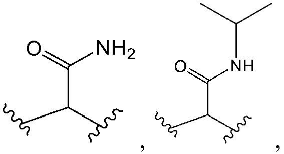 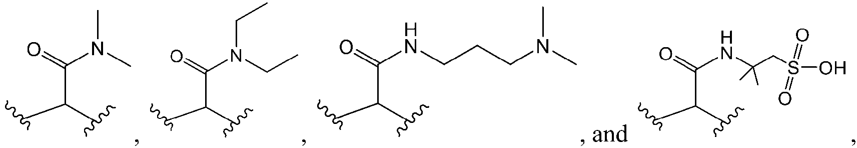 m is an integer from 1 to 2,000 and n is an integer from 1 to 10,000,
   R¹ comprises the single template site for bonding a template polynucleotide to the scaffold, R⁴ is selected from an optionally substituted C₁-C₂₀ alkyl, an optionally substituted C₁-C₂₀ alkenyl, an optionally substituted C₁-C₂₀ alkynyl, an optionally substituted C₁-C₂₀ oxaalkyl, an optionally substituted C₁-C₂₀ thiaalkyl, and an optionally substituted C₁-C₂₀ azaalkyl, wherein substituted comprises substitution with one or more of a C₁-C₂₀ alkyl, a double-bonded oxygen, and a hydroxyl group, and R³ comprises the accessory site for bonding accessory oligonucleotides.
68. The nanoparticle of paragraph 67, wherein the single template site comprises a covalent template bonding site.
69. The nanoparticle of paragraph 68, wherein the covalent template bonding site is selected from an amine-NHS ester bonding site, an amine-imidoester bonding site, an amine-pentofluorophenyl ester bonding site, an amine-hydroxymethyl phosphine bonding site, a carboxyl-carbodiimide bonding site, a thiol-maleimide bonding site, a thiol-haloacetyl bonding site, a thiol-pyridyl disulfide bonding site, a thiol-thiosulfonate bonding site, a thiol-vinyl sulfone bonding site, an aldehyde-hydrazide bonding site, an aldehyde-alkoxyamine bonding site, a hydroxy-isocyanate bonding site, an azide-alkyne bonding site, an azide-phosphine bonding site, a transcyclooctene-tetrazine bonding site, a norbornene-tetrazine bonding site, an azide-cyclooctyne bonding site, an azide-norbornene bonding site, an oxime bonding site, a SpyTag-SpyCatcher bonding site, a Snap-tag-O⁶-Benzylguanine bonding site, a CLIP-tag-O²-benzylcytosine bonding site, and a sortase-coupling bonding site.
70. The nanoparticle of any one of paragraph 67, wherein the single template site comprises a noncovalent template bonding site.
71. The nanoparticle of paragraph 70, wherein the noncovalent template bonding site comprises a polynucleotide hybridization site.
72. The nanoparticle of paragraph 70, wherein the noncovalent template bonding site comprises a noncovalent peptide binding site and the noncovalent peptide binding site is selected from a coiled-coil bonding site and an avidin-biotin bonding site.
73. The nanoparticle of any one of paragraphs 67 through 72, wherein the plurality of accessory sites for bonding accessory oligonucleotides to the scaffold comprise covalent accessory oligonucleotide bonding sites.
74. The nanoparticle of paragraph 73, wherein the covalent accessory oligonucleotide bonding sites are selected from amine-NHS ester bonding sites, amine-imidoester bonding sites, amine-pentofluorophenyl ester bonding sites, amine-hydroxymethyl phosphine bonding sites, carboxyl-carbodiimide bonding sites, thiol-maleimide bonding sites, thiol-haloacetyl bonding sites, thiol-pyridyl disulfide bonding sites, thiol-thiosulfonate bonding sites, thiol-vinyl sulfone bonding sites, aldehyde-hydrazide bonding sites, aldehyde-alkoxyamine bonding sites, hydroxy-isocyanate bonding sites, azide-alkyne bonding sites, azide-phosphine bonding sites, transcyclooctene-tetrazine bonding sites, norbornene-tetrazine bonding sites, an azide-cyclooctyne bonding sites, azide-norbornene bonding sites, oxime bonding sites, SpyTag-SpyCatcher bonding sites, Snap-tag-O⁶-Benzylguanine bonding sites, CLIP-tag-O²-benzylcytosine bonding sites, sortase-coupling bonding sites, and any combination of two or more of the foregoing.
75. The nanoparticle of any one of paragraphs 67 through 72, wherein the accessory oligonucleotide bonding sites comprises noncovalent accessory oligonucleotide bonding sites.
76. The nanoparticle of paragraph 75, wherein the noncovalent accessory oligonucleotide bonding sites comprise polynucleotide hybridization sites.
77. The nanoparticle of paragraph 75, wherein the noncovalent accessory oligonucleotide bonding sites comprise noncovalent peptide binding sites and the noncovalent peptide binding sites are selected from one or both of coiled-coil bonding sites and avidin-biotin bonding sites.
78. The nanoparticle of any one of paragraphs 67 through 77, further comprising a single template polynucleotide bonded to the single template site.
79. The nanoparticle of any one of paragraphs 67 through 78, further comprising a plurality of accessory oligonucleotides bonded to the plurality of accessory sites.
80. The nanoparticle of any one of paragraphs 67 through 79 wherein the nanoparticle is at least about 10 nm in diameter.
81. A method, comprising bonding a single template polynucleotide to the single template site of the nanoparticle of any one of paragraphs 67 through 80.
82. A method, comprising bonding a plurality of accessory oligonucleotides to the plurality of accessory sites of the nanoparticle of any one of paragraphs 67 through 81.
83. The method of paragraph 81 or 82, further comprising synthesizing one or more scaffold-attached copies selected from copies of the template polynucleotide, copies of the polynucleotides complementary to the template polynucleotide, and copies of both, wherein the scaffold-attached copies extend from the accessory oligonucleotides.
84. The method of any one of paragraphs 81-83, further comprising attaching the scaffold to a substrate, wherein attaching comprises hybridizing accessory oligonucleotides with oligonucleotides attached to the substrate.
85. The method of paragraph 84, wherein the substrate comprises a plurality of nanowells and the oligonucleotides attached to the substrate are attached within the plurality of nanowells.
86. The method of paragraph 85, wherein no more than one scaffold binds within any one of the nanowells.
87. The method of any one of paragraphs 84 through 86 further comprising synthesizing one or more substrate-attached copies selected from copies of the template polynucleotide, copies of the polynucleotides complementary to the template polynucleotide, and copies of both, wherein the substrate-attached copies extend from oligonucleotides attached to a substrate.
88. The method of paragraph 83 or 87, further comprising sequencing at least one of scaffold-attached copies and substrate-attached copies, wherein sequencing comprises sequencing by synthesis.

## Claims

1. A nanoparticle, comprising
a scaffold, a single template site for bonding a template polynucleotide to the scaffold selected from a covalent template bonding site and a noncovalent template bonding site, and a plurality of accessory sites for bonding accessory oligonucleotides to the scaffold selected from covalent accessory oligonucleotide bonding sites and noncovalent accessory oligonucleotide bonding sites, wherein
the scaffold comprises a dendrimer wherein the dendrimer comprises from 2 to 10 generations of constitutional repeating units, the constitutional repeating units comprise lysine wherein a lysine of an upstream generation forms a peptide bond with a first lysine of an immediately downstream generation and an isopeptide bond with a second lysine of the immediately downstream generation,
the single template site extends from the C-terminal end of the lysine of the first generation of the dendrimer and the plurality of accessory sites extend from NH₂ groups of lysines of the last generation of the dendrimer.

2. The nanoparticle of claim 1, wherein the single template site comprises a covalent template bonding site.

3. The nanoparticle of claim 2, wherein the covalent template bonding site is selected from an amine-NHS ester bonding site, an amine-imidoester bonding site, an amine-pentofluorophenyl ester bonding site, an amine-hydroxymethyl phosphine bonding site, a carboxyl-carbodiimide bonding site, a thiol-maleimide bonding site, a thiol-haloacetyl bonding site, a thiol-pyridyl disulfide bonding site, a thiol-thiosulfonate bonding site, a thiol-vinyl sulfone bonding site, an aldehyde-hydrazide bonding site, an aldehyde-alkoxyamine bonding site, a hydroxy-isocyanate bonding site, an azide-alkyne bonding site, an azide-phosphine bonding site, a transcyclooctene-tetrazine bonding site, a norbornene-tetrazine bonding site, an azide-cyclooctyne bonding site, an azide-norbornene bonding site, an oxime bonding site, a SpyTag-SpyCatcher bonding site, a Snap-tag-O⁶-Benzylguanine bonding site, a CLIP-tag-O²-benzylcytosine bonding site, and a sortase-coupling bonding site.

4. The nanoparticle of any one of claims 1 to 3, wherein the single template site comprises a noncovalent template bonding site; optionally wherein the noncovalent template bonding site comprises:
i) a polynucleotide hybridization site; or
ii) a noncovalent peptide binding site and the noncovalent peptide binding site is selected from a coiled-coil bonding site and an avidin-biotin bonding site.

5. The nanoparticle of any one of claims 1 through 4, wherein the plurality of accessory sites for bonding accessory oligonucleotides to the scaffold comprise covalent accessory oligonucleotide bonding sites.

6. The nanoparticle of claim 5, wherein the covalent accessory oligonucleotide bonding sites are selected from amine-NHS ester bonding sites, amine-imidoester bonding sites, amine-pentofluorophenyl ester bonding sites, amine-hydroxymethyl phosphine bonding sites, carboxyl-carbodiimide bonding sites, thiol-maleimide bonding sites, thiol-haloacetyl bonding sites, thiol-pyridyl disulfide bonding sites, thiol-thiosulfonate bonding sites, thiol-vinyl sulfone bonding sites, aldehyde-hydrazide bonding sites, aldehyde-alkoxyamine bonding sites, hydroxy-isocyanate bonding sites, azide-alkyne bonding sites, azide-phosphine bonding sites, transcyclooctene-tetrazine bonding sites, norbornene-tetrazine bonding sites, an azide-cyclooctyne bonding sites, azide-norbornene bonding sites, oxime bonding sites, SpyTag-SpyCatcher bonding sites, Snap-tag-O⁶-Benzylguanine bonding sites, CLIP-tag-O²-benzylcytosine bonding sites, sortase-coupling bonding sites, and any combination of two or more of the foregoing.

7. The nanoparticle of any one of claims 1 through 6, wherein the accessory oligonucleotide bonding sites comprise noncovalent accessory oligonucleotide bonding sites; optionally wherein the noncovalent accessory oligonucleotide bonding sites comprise:
i) polynucleotide hybridization sites; or
ii) noncovalent peptide binding sites and the noncovalent peptide binding sites are selected from one or both of coiled-coil bonding sites and avidin-biotin bonding sites.

8. The nanoparticle of any one of claims 1 through 7, further comprising a single template polynucleotide bonded to the single template site.

9. The nanoparticle of any one of claims 1 through 8, further comprising a plurality of accessory oligonucleotides bonded to the plurality of accessory sites.

10. The nanoparticle of any one of claims 1 through 9, wherein the nanoparticle is at least about 10 nm in diameter.

11. A method, comprising bonding a single template polynucleotide to the single template site of the nanoparticle of any one of claims 1 through 9.

12. A method, comprising bonding a plurality of accessory oligonucleotides to the plurality of accessory sites of the nanoparticle of any one of claims 1 through 10.

13. The method of claim 11 or 12, further comprising synthesizing one or more scaffold-attached copies selected from copies of the template polynucleotide, copies of the polynucleotides complementary to the template polynucleotide, and copies of both, wherein the scaffold-attached copies extend from the accessory oligonucleotides.

14. The method of any one of claims 11-13, further comprising attaching the scaffold to a substrate, wherein attaching comprises hybridizing accessory oligonucleotides with oligonucleotides attached to the substrate; optionally wherein the substrate comprises a plurality of nanowells and the oligonucleotides attached to the substrate are attached within the plurality of nanowells; further optionally wherein no more than one scaffold binds within any one of the nanowells.

15. The method of any one of claims 11 through 14 further comprising:
i) synthesizing one or more substrate-attached copies selected from copies of the template polynucleotide, copies of the polynucleotides complementary to the template polynucleotide, and copies of both, wherein the substrate-attached copies extend from oligonucleotides attached to a substrate; and/or
ii) sequencing at least one of scaffold-attached copies and substrate-attached copies, wherein sequencing comprises sequencing by synthesis.
